# EUROPEAN PATENT SPECIFICATION

(11) **EP 3 224 237 B1**
(45) Date of publication and mention of the grant of the patent: **11.09.2024**
(21) Application number: 15871953.4
(22) Date of filing: 23.12.2015
(51) Int. Cl.: C07C 233/05, C07C 235/06, C07C 275/06, C07C 311/03, C07D 213/06, C07D 215/12, C07D 233/64, C07D 277/20, C07D 333/06, A61K 31/16, A61P 31/00

(54) **NECROSIS INHIBITORS**
NEKROSEINHIBITOREN
INHIBITEURS DE NÉCROSE

(30) Priority: 24.12.2014 WO PCT/CN2014/094735
(43) Date of publication of application: 04.10.2017
(73) Proprietor: National Institute Of Biological Sciences, Beijing, Beijing 102206 (CN)
(72) Inventor: ZHANG, Zhiyuan, Beijing 102206 (CN); WANG, Xiaodong, Beijing 102206 (CN); LEI, Xiaoguang, Beijing 102206 (CN); SU, Yaning, Beijing 102206 (CN); HE, Sudan, Beijing 102206 (CN); RUAN, Hanying, Beijing 102206 (CN); SUN, Liming, Beijing 102206 (CN)
(74) Representative: Maiwald GmbH
(86) International application number: PCT/CN2015/098367
(87) International publication number: WO 2016/101885

(56) References cited:
- EP-A1- 2 540 720
- WO-A1-2008/116620
- WO-A1-2008/116620
- WO-A1-2009/023272
- WO-A1-2010/075561
- WO-A1-2012/087771
- WO-A2-2012/125544
- WO-A2-2012/125544
- WO-A2-2014/160185
- WO-A2-2014/160185
- CN-A- 102 503 860
- CN-A- 102 617 259
- US-A- 3 498 792
- US-A- 4 337 332
- US-A- 4 957 533
- US-A- 5 189 180
- US-A1- 2004 006 081
- US-A1- 2010 216 787
- US-A1- 2015 266 834
- HASEGAWA NAO ET AL: "Ruthenium-catalyzed cyclocarbonylation of aliphatic amides through the regioselective activation of unactivated C(sp3)-H bonds", TETRAHEDRON, ELSEVIER SCIENCE PUBLISHERS, AMSTERDAM, NL, vol. 69, no. 22, 8 February 2013 (2013-02-08), pages 4466 - 4472, XP028584931, ISSN: 0040-4020, DOI: 10.1016/J.TET.2013.02.006
- LIHONG ZHOU ET AL: "Palladium-Catalyzed [beta]-Acyloxylation of Simple Amide via sp 3 C-H Activation", ORGANIC LETTERS , 14(23), 6012-6015 CODEN: ORLEF7; ISSN: 1523-7052, vol. 16, no. 2, 17 January 2014 (2014-01-17), pages 508 - 511, XP055393823, ISSN: 1523-7060, DOI: 10.1021/ol403393w
- JENNY L MAKI ET AL: "Fluorescence polarization assay for inhibitors of the kinase domain of receptor interacting protein 1", ANALYTICAL BIOCHEMISTRY, ELSEVIER, AMSTERDAM, NL, vol. 427, no. 2, 21 May 2012 (2012-05-21), pages 164 - 174, XP028405139, ISSN: 0003-2697, [retrieved on 20120529], DOI: 10.1016/J.AB.2012.05.019
- TENG X ET AL: "Structure-activity relationship and liver microsome stability studies of pyrrole necroptosis inhibitors", BIOORGANIC & MEDICINAL CHEMISTRY LETTERS, PERGAMON, AMSTERDAM, NL, vol. 18, no. 11, 1 June 2008 (2008-06-01), pages 3219 - 3223, XP022711201, ISSN: 0960-894X, [retrieved on 20080425], DOI: 10.1016/J.BMCL.2008.04.048
- TENG ET AL: "Structure-activity relationship study of [1,2,3]thiadiazole necroptosis inhibitors", BIOORGANIC & MEDICINAL CHEMISTRY LETTERS, PERGAMON, AMSTERDAM, NL, vol. 17, no. 24, 17 October 2007 (2007-10-17), pages 6836 - 6840, XP022339583, ISSN: 0960-894X, DOI: 10.1016/J.BMCL.2007.10.024
- YAN REN ET AL: "Discovery of a Highly Potent, Selective, and Metabolically Stable Inhibitor of Receptor-Interacting Protein 1 (RIP1) for the Treatment of Systemic Inflammatory Response Syndrome", JOURNAL OF MEDICINAL CHEMISTRY, vol. 60, no. 3, 9 February 2017 (2017-02-09), pages 972 - 986, XP055435574, ISSN: 0022-2623, DOI: 10.1021/acs.jmedchem.6b01196
- ZHANG, MEIHUI ET AL.: "Synthesis and activity of tetrapeptidic HTLV-I protease inhibitors possessing different P3-cap moieties", BIOORGANIC & MEDICINAL CHEMISTRY, vol. 16, no. 10, 31 December 2008 (2008-12-31), XP022673161
- TSUNODA, TETSUTO ET AL.: "An efficient method for hydrolysis of N-monosubstituted amides. Utilization of intramolecular nitrogen-oxygen acyl migration in hydroxypivalimides", TETRAHEDRON, vol. 47, no. 24, 31 December 1991 (1991-12-31), pages 3928, XP008144044
- ZHOU, LIHONG ET AL.: "Palladium-Catalyzed beta-Acyloxylation of Simple Amide via sp3 C-H Activation", ORGANIC LETTERS, vol. 16, no. 2, 27 December 2013 (2013-12-27), XP055393823
- BIRCHALL, J. M. ET AL.: "Polyfluoroarenes. IV. 2, 3, 4, 5, 6-Pentafluorotoluene and related compounds", JOURNAL OF THE CHEMICAL SOCIETY, 31 December 1961 (1961-12-31), XP002187941
- KHALAFI-NEZHAD ALI ET AL.: "Silica boron-sulfuric acid nanoparticles (SBSANs): preparation, characterization and their catalytic application in the Ritter reaction for the synthesis of amide derivatives", JOURNAL OF MATERIALS CHEMISTRY, vol. 21, no. 34, 31 December 2011 (2011-12-31), XP055393828
- ZHU, MINGWEN ET AL.: "Aerobic Oxidative Amidation of Aromatic and Cinnamic Aldehydes with Secondary Amines by CuI/2-Pyridonate Catalytic System", JOURNAL OF ORGANIC CHEMISTRY, vol. 77, no. 20, 25 September 2012 (2012-09-25), XP055102501
- GEFFKEN, DETLEF ET AL.: "The reaction of 2, 2-disubstituted 3-hydroxypropionic acids with N- monosubstituted hydroxylammonium chlorides and dicyclohexylcarbodiimide", ARCHIV DER PHARMAZIE, vol. 309, no. 5, 31 December 1976 (1976-12-31), XP000951547
- KRETOV, A. E. ET AL.: "N-Arylsulfonamide derivatives of monoethanolamine", ZHURNAL OBSHCHEI KHIMII, vol. 29, 31 December 1959 (1959-12-31), XP008185261
- VELAVAN, A. ET AL.: "Unsymmetrical tetrasubstituted ureas from tertiary carbamoylimidazole: activation by AlMe3", ORGANIC & BIOMOLECULAR CHEMISTRY, vol. 10, no. 31, 31 December 2012 (2012-12-31), pages 6423, XP055269907, DOI: doi:10.1039/c2ob25412c
- VANDI, ANTONIO ET AL.: "Syntheses and properties of some N-substituted sulfamides", JOURNAL OF ORGANIC CHEMISTRY, vol. 26, 1 May 1961 (1961-05-01), XP002474071, DOI: doi:10.1021/jo01063a039
- TERAUCHI, HIROMI ET AL.: "Reaction of N-haloamide. XVI. Reaction of N, N- dichlorophenylacetamide with aliphatic secondary amines", CHEMICAL & PHARMACEUTICAL BULLETIN, vol. 20, no. 11, 31 December 1972 (1972-12-31), pages 2478, XP003032463
- S OMASEKHARA, S. ET AL.: "Chlorobenzenesulfonamides & some related benzothiadiazines", INDIAN JOURNAL OF CHEMISTRY, vol. 6, no. 1, 31 January 1968 (1968-01-31), XP001012823
- SAHU, BASANT ET AL.: "UV spectral study of some N-acyl derivatives of N-benzyl hydroxylamine", ACTA CIENCIA INDICA, CHEMISTRY, vol. 16 C, no. 2, 31 December 1990 (1990-12-31), pages 3628, XP008185262

## Description

### Introduction

Tumor necrosis factor alpha (TNF-α)-induced NF-κB activation plays a central role in the immune system and inflammatory responses. Receptor-interacting protein 1 (RIP1) is a multi-functional signal transducer involved in mediating nuclear factor κB (NF-κB) activation, apoptosis, and necroptosis. The kinase activity of RIP1 is critically involved in mediating necroptosis, a caspase-independent pathway of necrotic cell death. Holler et al. Nat Immunol 2000; 1: 489-495; Degterev et al. Nat Chem Biol 2008; 4: 313-321.

Necroptosis plays a role in various pathological forms of cell death, including ischemic brain injury, neurodegenerative diseases and viral infections. Dunai, et al., Dec 2011, Pathol. Oncol. Res.: POR 17 (4): 791-800. Necrostatin-1 (Nec-1), a small molecule inhibitor of RIP1 kinase activity, can block necroptosis. Degterev et al. Nat Chem Biol 2005; 1: 112-119.

Related patent publications include: US6756394, US8278344, US2012122889, US2009099242, US2010317701, US2011144169, US20030083386, US20120309795, WO2009023272, WO2010075290, WO2010075561, WO2012125544. Compounds useful in treating necrosis associated diseases are disclosed e.g. in WO2009023272 and WO2012125544. However, there is no pointer in these documents in the direction of the present invention.

### Summary of the Invention

The invention provides an inhibitor of human receptor interacting protein 1 kinase (RIP1) of formula: 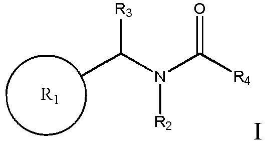 or a pharmaceutically acceptable salt, hydrate or stereoisomer the compound for use in the treatment of a necrosis-associated disease or condition, comprising administering an effective amount of a compound or a pharmaceutical composition to a patient in need thereof, wherein:

R1 is substituted or unsubstituted phenyl,

R2 is hydroxyl; R3 is H; R4 is 1,1-dimethylpropyl.

Also disclosed but not part of the invention are the corresponding sulfonamides of all the generally and specifically disclosed amides, e.g. 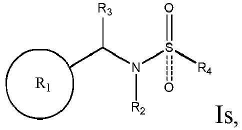 wherein S may be double bond to one or two O atoms, or a pharmaceutically acceptable salt, hydride or stereoisomer thereof, wherein the R moieties are as described herein, or a pharmaceutically acceptable salt, hydride or stereoisomer thereof.

The invention provides pharmaceutical compositions comprising the subject compounds. Methods of making and using the subject compounds are also disclosed. The compositions may comprise a pharmaceutically-acceptable excipient, be in effective, unit dosage form, and/or comprise another, different therapeutic agents for the targeted disease or condition. Also disclosed but not forming part of the invention are methods of treating a person in need thereof with an effective amount of the subject compound or pharmaceutical composition, and optionally, detecting a resultant improvement in the person's health or condition. The methods may also optionally include the antecedent step of determining that the person, particularly diagnosing and applicable disease or condition (herein).

The invention encompasses all combination of the particular embodiments recited herein.

### Description of Particular Embodiments of the Invention

The following descriptions of particular embodiments and examples are provided by way of illustration and not by way of limitation. Those skilled in the art will readily recognize a variety of noncritical parameters that could be changed or modified to yield essentially similar results. The invention provides myriad embodiments.
1. The invention provides amide inhibitors of human receptor interacting protein 1 kinase (RIP1).
2. In particular embodiments the present invention provides compounds of formula: 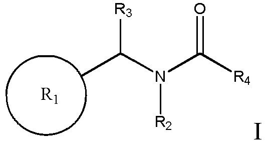 wherein:
R1 is substituted or unsubstituted phenyl,
R2 is hydroxyl, R3 is H, R4 is 1,1-dimethylpropyl, or a pharmaceutically acceptable salt, hydrate or stereoisomer the compound for use in the treatment of a necrosis-associated disease or condition, comprising administering an effective amount of a compound or a pharmaceutical composition to a patient in need thereof.

3. In another aspect, the compound is of formula: 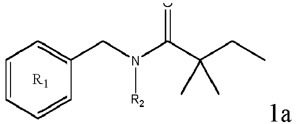 wherein:
R₁ is substituted or unsubstituted phenyl, and
R₂ is OH,
a pharmaceutically acceptable salt, hydrate or stereoisomer the compound.

4. In another aspect the compound is of formula: 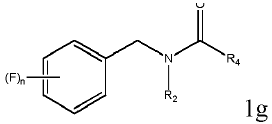 wherein
R₂ is OH, and
n is 0, 1, 2, 3, 4 or 5, or
a pharmaceutically acceptable salt, hydrate or stereoisomer the compound.
R4 is 1-dimethylpropyl,

5. The disclosure also describes compounds wherein (F)n, R2 and R4 are as follows:

| # | (F)n | R2 | R4 |
|---|---|---|---|
| 48 | 2F, 4F, 6F | Me | 1-dimethylpropyl |
| 49 | 2F, 3F, 4F | Me | 1-dimethylpropyl |
| 51 | 2F, 3F, 5F, 6F | Me | 1-dimethylpropyl |
| 75 | 2F, 3F, 5F | Me | 1-dimethylpropyl |
| 92 | - | OH | 1-dimethylpropyl |
| 93 | 2F, 3F, 5F | OH | 1-dimethylpropyl |
| 94 | 4F | OH | 1-dimethylpropyl |
| 95 | 2F, 4F | OH | 1-dimethylpropyl |
| 96 | 2F, 4F | OH | 1-dimethylpropyl |
| 97 | 2F, 3F, 4F | OH | 1-dimethylpropyl |
| 98 | 2F, 4F, 5F | OH | 1-dimethylpropyl |
| 99 | 3F, 4F, 5F | OH | 1-dimethylpropyl |
| S5 | 2-F, 3-F, 5-F | Me | 1-dimethyl, 2-difluoropropyl |
| S6 | - | OH | 1-dimethyl, 2-difluoropropyl |
| S9 | 4-F | OH | 1-dimethyl, 2-difluoropropyl |
| S10 | 2-F, 4-F | OH | 1-dimethyl, 2-difluoropropyl |
| S11 | 3-F, 4-F | OH | 1-dimethyl, 2-difluoropropyl |
| S12 | 2-F, 4-F, 5-F | OH | 1-dimethyl, 2-difluoropropyl |
| S13 | 3-F, 4-F, 5-F | OH | 1-dimethyl, 2-difluoropropyl |
| S14 | 2-F, 3-F, 5-F | OH | 1-dimethyl, 2-difluoropropyl |

Compounds 48,49,51,75,S5,S6,S9-S14 here-above are however not part of the invention.

6. In embodiments the subject compounds have a formula 92-99 of Table 1.

7. In embodiments the invention provides pharmaceutical compositions comprising a subject compound and a pharmaceutically-acceptable excipient, in unit dosage.

Also disclosed are pharmaceutical compositions comprising a subject compound and a pharmaceutically-acceptable excipient, in unit dosage, and a different therapeutic agent for a necrosis-associated disease or condition.

Also disclosed but not forming part of the invention are methods of treating a necrosis-associated disease or condition, comprising administering an effective amount of a subject compound or composition to a patient in need thereof.

The method of treatment may comprise the antecedent step of diagnosing the necrosis-associated disease or condition, or the subsequent step of detecting a resultant amelioration of the necrosis-associated disease or condition.

Applicable diseases or conditions are necrosis- (including necroptosis) associated and include neuro-degenerative disease of the central or peripheral nervous system, endotoxic/septic shock, terminal ileitis, myocarditis, arthritis, atherosclerosis, acute enteritis, ischemic necrosis, pathology resulting from renal failure or cell death, including retinal neuronal, cardiac muscle or immune cell death, such as chemo- or radiation-induced necrosis; liver disease, including drug-induced liver damage or toxicity, acute hepatitis, etc., pancreatic disease, including necrotizing pancreatitis, heart, mesenteric, retinal, hepatic or brain/cerebral ischemic injury, nephritis, ischemic injury during reperfusion or organ storage, head trauma, including traumatic brain injury, stroke, septic shock, coronary heart disease, cardiomyopathy, myocardial infarction, bone avascular necrosis, sickle cell disease, muscle wasting, gastrointestinal disease, tuberculosis, diabetes, pathogenic alteration of blood vessels, muscular dystrophy, graft-versus-host disease, viral, bacterial and fungal infection, Crohn's disease, ulcerative colitis, asthma, etc.

Exemplary applicable viruses are human immunodeficiency virus (HIV), Epstein-Barr virus (EBV), cytomegalovirus (CMV)5 human herpesviruses (HHV), herpes simplex viruses (HSV), human T-Cell leukemia viruses (HTLV)5 Varicella-Zoster virus (VZV), measles virus, papovaviruses (JC and BK), hepatitis viruses, adenovirus, parvoviruses, and human papillomaviruses. Exemplary diseases caused by viral infection include, but are not limited to, chicken pox, Cytomegalovirus infections, genital herpes, Hepatitis B and C, influenza, and shingles.

Exemplary applicable bacteria include, but are not limited to Campylobacter jejuni, Enterobacter species, Enterococcus faecium, Enterococcus faecalis, Escherichia coli (e.g., E. coli O157:H7), Group A streptococci, Haemophilus influenzae, Helicobacter pylori, listeria, Mycobacterium tuberculosis, Pseudomonas aeruginosa, S. pneumoniae, Salmonella, Shigella, Staphylococcus aureus, and Staphylococcus epidermidis. Exemplary diseases caused by bacterial infection include, but are not limited to, anthrax, cholera, diphtheria, foodborne illnesses, leprosy, meningitis, peptic ulcer disease, pneumonia, sepsis, tetanus, tuberculosis, typhoid fever, and urinary tract infection.

Exemplary applicable neurodegenerative diseases are Alzheimer's disease, Huntington's disease, Parkinson's disease, amyotrophic lateral sclerosis, HIV-associated dementia, cerebral ischemia, amyotropic lateral sclerosis, multiple sclerosis, Lewy body disease, Menke's disease, Wilson's disease, Creutzfeldt-Jakob disease, and Fahr disease.

Exemplary applicable muscular dystrophies or related diseases are Becker's muscular dystrophy, Duchenne muscular dystrophy, myotonic dystrophy, limb-girdle muscular dystrophy, Landouzy-Dejerine muscular dystrophy, facioscapulohumeral muscular dystrophy (Steinert's disease), myotonia congenita, Thomsen's disease, and Pompe's disease. Muscle wasting can be associated with cancer, AIDS, congestive heart failure, and chronic obstructive pulmonary disease, as well as include necrotizing myopathy of intensive care.

Unless contraindicated or noted otherwise, in these descriptions and throughout this specification, the terms "a" and "an" mean one or more, the term "or" means and/or and polynucleotide sequences are understood to encompass opposite strands as well as alternative backbones described herein. Furthermore, genuses are recited as shorthand for a recitation of all members of the genus; for example, the recitation of (C1-C3) alkyl is shorthand for a recitation of all C1-C3 alkyls: methyl, ethyl and propyl, including isomers thereof.

The term "heteroatom" as used herein generally means any atom other than carbon or hydrogen. Preferred heteroatoms include oxygen (O), phosphorus (P), sulfur (S), nitrogen (N), and halogens, and preferred heteroatom functional groups are haloformyl, hydroxyl, aldehyde, amine, azo, carboxyl, cyanyl, thocyanyl, carbonyl, halo, hydroperoxyl, imine, aldimine, isocyanide, iscyante, nitrate, nitrile, nitrite, nitro, nitroso, phosphate, phosphono, sulfide, sulfonyl, sulfo, and sulfhydryl.

The term "alkyl," by itself or as part of another substituent, means, unless otherwise stated, a straight or branched chain, or combination thereof, which is fully saturated, having the number of carbon atoms designated (i.e. C1-C8 means one to eight carbons). Examples of alkyl groups include methyl, ethyl, n-propyl, isopropyl, n-butyl, t-butyl, isobutyl, sec-butyl, and isomers of, for example, n-pentyl, n-hexyl, n-heptyl, n-octyl.

The term "alkenyl", by itself or as part of another substituent, means a straight or branched chain, or cyclic hydrocarbon radical, or combination thereof, which may be mono- or polyunsaturated, having the number of carbon atoms designated (i.e. C2-C8 means two to eight carbons) and one or more double bonds. Examples of alkenyl groups include vinyl, 2-propenyl, crotyl, 2-isopentenyl, 2-(butadienyl), 2,4-pentadienyl, 3-(1,4-pentadienyl) and higher homologs and isomers thereof.

The term "alkynyl", by itself or as part of another substituent, means a straight or branched chain hydrocarbon radical, or combination thereof, which may be mono- or polyunsaturated, having the number of carbon atoms designated (i.e. C2-C8 means two to eight carbons) and one or more triple bonds. Examples of alkynyl groups include ethynyl, 1- and 3-propynyl, 3-butynyl and higher homologs and isomers thereof.

The term "alkylene" by itself or as part of another substituent means a divalent radical derived from alkyl, as exemplified by -CH₂-CH₂-CH₂-CH₂-. Typically, an alkyl (or alkylene) group will have from 1 to 24 carbon atoms, with those groups having 10 or fewer carbon atoms being preferred. A "lower alkyl" or "lower alkylene" is a shorter chain alkyl or alkylene group, generally having eight or fewer carbon atoms.

The terms "alkoxy," "alkylamino" and "alkylthio" (or thioalkoxy) are used in their conventional sense, and refer to those alkyl groups attached to the remainder of the molecule via an oxygen atom, an amino group, or a sulfur atom, respectively.

The term "heteroalkyl," by itself or in combination with another term, means, unless otherwise stated, a stable straight or branched chain, or cyclic hydrocarbon radical, or combinations thereof, consisting of the stated number of carbon atoms and from one to three heteroatoms selected from the group consisting of O, N, P, Si and S, wherein the nitrogen, sulfur, and phosphorous atoms may optionally be oxidized and the nitrogen heteroatom may optionally be quaternized. The heteroatom(s) O, N, P and S may be placed at any interior position of the heteroalkyl group. The heteroatom Si may be placed at any position of the heteroalkyl group, including the position at which the alkyl group is attached to the remainder of the molecule. Examples include -CH₂-CH₂-O-CH₃, -CH₂-CH₂-NH-CH₃, -CH₂-CH₂-N(CH₃)-CH₃, -CH₂-S-CH₂-CH₃, -CH₂-CH₂,-S(O)-CH₃, -CH₂-CH₂-S(O)₂-CH₃, -CH=CH-O-CH₃, - Si(CH₃)₃, -CH₂-CH=N-OCH₃, and -CH=CH-N(CH3)-CH₃. Up to two heteroatoms may be consecutive, such as, for example, -CH₂-NH-OCH₃ and -CH₂-O-Si(CH₃)₃.

Similarly, the term "heteroalkylene," by itself or as part of another substituent means a divalent radical derived from heteroalkyl, as exemplified by -CH₂-CH₂-S-CH₂-CH₂- and -CH₂-S-CH₂-CH₂-NH-CH₂-. For heteroalkylene groups, heteroatoms can also occupy either or both of the chain termini (e.g., alkyleneoxy, alkylenedioxy, alkyleneamino, alkylenediamino, and the like). Still further, for alkylene and heteroalkylene linking groups, no orientation of the linking group is implied.

The terms "cycloalkyl" and "heterocycloalkyl", by themselves or in combination with other terms, represent, unless otherwise stated, cyclic versions of "alkyl" and "heteroalkyl", respectively. Accordingly, a cycloalkyl group has the number of carbon atoms designated (i.e., C3-C8 means three to eight carbons) and may also have one or two double bonds. A heterocycloalkyl group consists of the number of carbon atoms designated and from one to three heteroatoms selected from the group consisting of O, N, Si and S, and wherein the nitrogen and sulfur atoms may optionally be oxidized and the nitrogen heteroatom may optionally be quaternized. Additionally, for heterocycloalkyl, a heteroatom can occupy the position at which the heterocycle is attached to the remainder of the molecule. Examples of cycloalkyl include cyclopentyl, cyclohexyl, 1-cyclohexenyl, 3-cyclohexenyl, cycloheptyl, and the like. Examples of heterocycloalkyl include 1-(1,2,5,6-tetrahydropyrid- yl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like.

The terms "halo" and "halogen," by themselves or as part of another substituent, mean, unless otherwise stated, a fluorine, chlorine, bromine, or iodine atom. Additionally, terms such as "haloalkyl," are meant to include alkyl substituted with halogen atoms, which can be the same or different, in a number ranging from one to (2m'+1), where m' is the total number of carbon atoms in the alkyl group. For example, the term "halo(C1-C4)alkyl" is mean to include trifluoromethyl, 2,2,2-trifluoroethyl, 4-chlorobutyl, 3-bromopropyl, and the like. Thus, the term "haloalkyl" includes monohaloalkyl (alkyl substituted with one halogen atom) and polyhaloalkyl (alkyl substituted with halogen atoms in a number ranging from two to (2m'+1) halogen atoms, where m' is the total number of carbon atoms in the alkyl group). The term "perhaloalkyl" means, unless otherwise stated, alkyl substituted with (2m'+1) halogen atoms, where m' is the total number of carbon atoms in the alkyl group. For example the term "perhalo(C1-C4)alkyl" is meant to include trifluoromethyl, pentachloroethyl, 1,1,1-trifluoro-2-bromo-2-chloroethyl and the like.

The term "acyl" refers to those groups derived from an organic acid by removal of the hydroxy portion of the acid. Accordingly, acyl is meant to include, for example, acetyl, propionyl, butyryl, decanoyl, pivaloyl, benzoyl and the like.

The term "aryl" means, unless otherwise stated, a polyunsaturated, typically aromatic, hydrocarbon substituent which can be a single ring or multiple rings (up to three rings) which are fused together or linked covalently. Non-limiting examples of aryl groups include phenyl, 1-naphthyl, 2-naphthyl, 4-biphenyl and 1,2,3,4-tetrahydronaphthalene.

The term heteroaryl," refers to aryl groups (or rings) that contain from zero to four heteroatoms selected from N, O, and S, wherein the nitrogen and sulfur atoms are optionally oxidized and the nitrogen heteroatom are optionally quaternized. A heteroaryl group can be attached to the remainder of the molecule through a heteroatom. Non-limiting examples of heteroaryl groups include 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl and 6-quinolyl.

For brevity, the term "aryl" when used in combination with other terms (e.g., aryloxy, arylthioxy, arylalkyl) includes both aryl and heteroaryl rings as defined above. Thus, the term "arylalkyl" is meant to include those radicals in which an aryl group is attached to an alkyl group (e.g., benzyl, phenethyl, pyridylmethyl and the like) including those alkyl groups in which a carbon atom (e.g., a methylene group) has been replaced by, for example, an oxygen atom (e.g., phenoxymethyl, 2-pyridyloxymethyl, 3-(1-naphthyloxy)propyl, and the like).

Each of the above terms (e.g., "alkyl," "heteroalkyl," "aryl" and "heteroaryl") is meant to include both substituted and unsubstituted forms of the indicated radical. Preferred substituents for each type of radical are provided below.

Substituents for the alkyl and heteroalkyl radicals (as well as those groups referred to as alkylene, alkenyl, heteroalkylene, heteroalkenyl, alkynyl, cycloalkyl, heterocycloalkyl, cycloalkenyl and heterocycloalkenyl) can be a variety of groups selected from: -OR', =O, =NR', =N-OR', -NR'R", -SR', halogen, -SiR'R"R‴, -OC(O)R', -C(O)R', -CO₂R', -CONR'R", - OC(O)NR'R", -NR"C(O)R', -NR'-C(O)NR"R‴, -NR'-SO₂NR‴, -NR"CO₂R', -NH-C(NH₂)=NH, -NR'C(NH₂)=NH, -NH-C(NH₂)=NR', -S(O)R', -SO₂R', -SO₂NR'R", -NR"SOzR, -CN and -NO₂, in a number ranging from zero to three, with those groups having zero, one or two substituents being particularly preferred. R', R" and R‴ each independently refer to hydrogen, unsubstituted (C1-C8)alkyl and heteroalkyl, unsubstituted aryl, aryl substituted with one to three halogens, unsubstituted alkyl, alkoxy or thioalkoxy groups, or aryl-(C1-C4)alkyl groups. When R' and R" are attached to the same nitrogen atom, they can be combined with the nitrogen atom to form a 5-, 6- or 7-membered ring. For example, -NR'R" is meant to include 1-pyrrolidinyl and 4-morpholinyl. Typically, an alkyl or heteroalkyl group will have from zero to three substituents, with those groups having two or fewer substituents being preferred. More preferably, an alkyl or heteroalkyl radical will be unsubstituted or monosubstituted. Most preferably, an alkyl or heteroalkyl radical will be unsubstituted. From the above discussion of substituents, one of skill in the art will understand that the term "alkyl" is meant to include groups such as trihaloalkyl (e.g., -CF₃ and -CH₂CF₃).

Preferred substituents for the alkyl and heteroalkyl radicals are selected from: -OR', =O, -NR'R", -SR', halogen, -SiR'R"R‴, -OC(O)R', -C(O)R', -CO₂R', -CONR'R", -OC(O)NR'R", - NR"C(O)R', -NR"CO₂R', -NR'-SO₂NR"R‴, -S(O)R', -S02R', -SO₂NR'R", -NR"SO₂R, -CN and -NO₂, where R' and R" are as defined above. Further preferred substituents are selected from: - OR', =O, -NR'R", halogen, -OC(O)R', -CO₂R', -CONR'R", -OC(O)NR'R", -NR"C(O)R', - NR"CO₂R', -NR'-SO₂NR"R‴, -SO₂R', -SO₂NR'R", -NR"SO₂R, -CN and -NO₂.

Similarly, substituents for the aryl and heteroaryl groups are varied and selected from: halogen, -OR', -OC(O)R', -NR'R", -SR', -R', -CN, -NO₂, -CO₂R', -CONR'R", -C(O)R', - OC(O)NR'R", -NR"C(O)R', -NR"CO₂R', -NR'-C(O)NR"R‴, -NR'-SO₂NR"R‴, -NH-C(NH2)=NH, -NR'C(NH₂)=NH, -NH-C(NH₂)=NR', -S(O)R', -SO₂R', -SO₂NR'R", -NR"SO₂R, - N₃, -CH(Ph)₂, perfluoro(C1-C4)alko- xy and perfluoro(C1-C4)alkyl, in a number ranging from zero to the total number of open valences on the aromatic ring system; and where R', R" and R‴ are independently selected from hydrogen, (C1-C8)alkyl and heteroalkyl, unsubstituted aryl and heteroaryl, (unsubstituted aryl)-(C1-C4)alkyl and (unsubstituted aryl)oxy-(C1-C4)alkyl. When the aryl group is 1,2,3,4-tetrahydronaphthalene, it may be substituted with a substituted or unsubstituted (C3-C7)spirocycloalkyl group. The (C3-C7)spirocycloalkyl group may be substituted in the same manner as defined herein for "cycloalkyl". Typically, an aryl or heteroaryl group will have from zero to three substituents, with those groups having two or fewer substituents being preferred. An aryl or heteroaryl group can be unsubstituted or monosubstituted, or alternatively unsubstituted.

Preferred substituents for aryl and heteroaryl groups are selected from: halogen, -OR', - OC(O)R', -NR'R", -SR', -R', -CN, -NO₂, -CO₂R', -CONR'R", -C(O)R',-OC(O)NR'R", - NR"C(O)R', -S(O)R', -SO₂R', -SO₂NR'R", -NR"SO₂R, -N₃, -CH(Ph)₂, perfluoro(C1-C4)alkoxy and perfluoro(C1-C4)alkyl, where R' and R" are as defined above. Further preferred substituents are selected from: halogen, -OR', -OC(O)R', -NR'R", -R', -CN, -NO₂, -CO₂R', -CONR'R", - NR"C(O)R', -SO₂R', -SO₂NR'R", -NR"SO₂R, perfluoro(C1-C4)alkoxy and perfluoro(C1-C4)alkyl.

The substituent -CO₂H, as used herein, includes bioisosteric replacements therefor; see, e.g., The Practice of Medicinal Chemistry; Wermuth, C. G., Ed.; Academic Press: New York, 1996; p. 203.

Two of the substituents on adjacent atoms of the aryl or heteroaryl ring may optionally be replaced with a substituent of the formula -T-C(O)-(CH₂)q-U-, wherein T and U are independently -NH-, -O-, -CH₂- or a single bond, and q is an integer of from 0 to 2. Alternatively, two of the substituents on adjacent atoms of the aryl or heteroaryl ring may optionally be replaced with a substituent of the formula -A-(CH2)r-B-, wherein A and B are independently -CH₂-, -O-, -NH-, -S-, -S(O)-, -S(O)₂-, -S(O)₂NR'- or a single bond, and r is an integer of from 1 to 3. One of the single bonds of the new ring so formed may optionally be replaced with a double bond. Alternatively, two of the substituents on adjacent atoms of the aryl or heteroaryl ring may optionally be replaced with a substituent of the formula -(CH₂)s-X-(CH₂)t- -, where s and t are independently integers of from 0 to 3, and X is -O-, -NR'-, -S-, - S(O)-, -S(O)₂-, or -S(O)₂NR'-. The substituent R' in -NR'- and -S(O)₂NR'- is selected from hydrogen or unsubstituted (C1-C6)alkyl.

Preferred substituents are disclosed herein and exemplified in the tables, structures, examples, and claims, and may be applied across different compounds of the invention, i.e. substituents of any given compound may be combinatorially used with other compounds.

In particular cases, substituents are independently substituted or unsubstituted heteroatom, substituted or unsubstituted, 0-3 heteroatom C1-C6 alkyl, substituted or unsubstituted, 0-3 heteroatom C2-C6 alkenyl, substituted or unsubstituted, 0-3 heteroatom C2-C6 alkynyl, or substituted or unsubstituted, 0-3 heteroatom C6-C14 aryl, wherein each heteroatom is independently oxygen, phosphorus, sulfur or nitrogen.

Applicable substituents can be independently aldehyde, aldimine, alkanoyloxy, alkoxy, alkoxycarbonyl, alkyloxy, alkyl, amine, azo, halogens, carbamoyl, carbonyl, carboxamido, carboxyl, cyanyl, ester, halo, haloformyl, hydroperoxyl, hydroxyl, imine, isocyanide, iscyante, N-tert-butoxycarbonyl, nitrate, nitrile, nitrite, nitro, nitroso, phosphate, phosphono, sulfide, sulfonyl, sulfo, sulfhydryl, thiol, thiocyanyl, trifluoromethyl or trifluromethyl ether (OCF3).

The term "pharmaceutically acceptable salts" is meant to include salts of the active compounds which are prepared with relatively nontoxic acids or bases, depending on the particular substituents found on the compounds described herein. When compounds of the invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, oxalic, maleic, malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like. Certain specific compounds of the invention contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the invention.

In addition to salt forms, also disclosed are compounds in a prodrug form. Prodrugs of the compounds described herein are those compounds that undergo chemical changes under physiological conditions to provide the compounds of the invention. Additionally, prodrugs can be converted to the compounds of the invention by chemical or biochemical methods in an ex vivo environment. For example, prodrugs can be slowly converted to the compounds of the invention when placed in a transdermal patch reservoir with a suitable enzyme or chemical reagent. Prodrugs are often useful because, in some situations, they may be easier to administer than the parent drug. They may, for instance, be more bioavailable by oral administration than the parent drug. The prodrug may also have improved solubility in pharmacological compositions over the parent drug. A wide variety of prodrug derivatives are known in the art, such as those that rely on hydrolytic cleavage or oxidative activation of the prodrug. An example, without limitation, of a prodrug would be a compound of the invention which is administered as an ester (the "prodrug"), but then is metabolically hydrolyzed to the carboxylic acid, the active entity. Additional examples include peptidyl derivatives of a compound of the invention.

Certain compounds of the invention can exist in unsolvated forms as well as solvated forms, including hydrated forms. In general, the solvated forms are equivalent to unsolvated forms. Certain compounds of the invention may exist in multiple crystalline or amorphous forms. In general, all physical forms are equivalent for the uses contemplated by the invention.

Certain compounds of the invention possess asymmetric carbon atoms (optical centers) or double bonds; the racemates, diastereomers, geometric isomers and individual isomers are thus also disclosed.

The compounds of the invention may also contain unnatural proportions of atomic isotopes at one or more of the atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds of the invention are thus also disclosed.

The term "therapeutically effective amount" refers to the amount of the subject compound that will elicit, to some significant extent, the biological or medical response of a tissue, system, animal or human that is being sought by the researcher, veterinarian, medical doctor or other clinician, such as when administered, is sufficient to prevent development of, or alleviate to some extent, one or more of the symptoms of the condition or disorder being treated. The therapeutically effective amount will vary depending on the compound, the disease and its severity and the age, weight, etc., of the mammal to be treated.

The invention also provides pharmaceutical compositions comprising the subject compounds and a pharmaceutically acceptable excipient, particularly such compositions comprising a unit dosage of the subject compounds, particularly such compositions copackaged with instructions describing use of the composition to treat an applicable disease or condition (herein).

The compositions for administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules, losenges or the like in the case of solid compositions. In such compositions, the compound is usually a minor component (from about 0.1 to about 50% by weight or preferably from about 1 to about 40% by weight) with the remainder being various vehicles or carriers and processing aids helpful for forming the desired dosing form.

Suitable excipients or carriers and methods for preparing administrable compositions are known or apparent to those skilled in the art and are described in more detail in such publications as Remington's Pharmaceutical Science, Mack Publishing Co, NJ (1991). In addition, the compounds may be advantageously used in conjunction with other therapeutic agents as described herein or otherwise known in the art, particularly other anti-necrosis agents. Hence the compositions may be administered separately, jointly, or combined in a single dosage unit.

The amount administered depends on the compound formulation, route of administration, etc. and is generally empirically determined in routine trials, and variations will necessarily occur depending on the target, the host, and the route of administration, etc. Generally, the quantity of active compound in a unit dose of preparation may be varied or adjusted from about 1, 3, 10 or 30 to about 30, 100, 300 or 1000 mg, according to the particular application. Unit dosage forms can be packaged in a multipack adapted for sequential use, such as blisterpack, comprising sheets of at least 6, 9 or 12 unit dosage forms. The actual dosage employed may be varied depending upon the requirements of the patient and the severity of the condition being treated. Determination of the proper dosage for a particular situation is within the skill of the art. Generally, treatment is initiated with smaller dosages which are less than the optimum dose of the compound. Thereafter, the dosage is increased by small amounts until the optimum effect under the circumstances is reached. For convenience, the total daily dosage may be divided and administered in portions during the day if desired.

The compounds can be administered by a variety of methods including, but not limited to, parenteral, topical, oral, or local administration, such as by aerosol or transdermally, for prophylactic and/or therapeutic treatment. Also, in accordance with the knowledge of the skilled clinician, the therapeutic protocols (e.g., dosage amounts and times of administration) can be varied in view of the observed effects of the administered therapeutic agents on the patient, and in view of the observed responses of the disease to the administered therapeutic agents.

The therapeutics of the invention can be administered in a therapeutically effective dosage and amount, in the process of a therapeutically effective protocol for treatment of the patient. For more potent compounds, microgram (ug) amounts per kilogram of patient may be sufficient, for example, in the range of about 1, 10 or 100 ug/kg to about 0.01, 0.1, 1, 10, or 100 mg/kg of patient weight though optimal dosages are compound specific, and generally empirically determined for each compound.

In general, routine experimentation in clinical trials will determine specific ranges for optimal therapeutic effect, for each therapeutic, each administrative protocol, and administration to specific patients will also be adjusted to within effective and safe ranges depending on the patient condition and responsiveness to initial administrations. However, the ultimate administration protocol will be regulated according to the judgment of the attending clinician considering such factors as age, condition and size of the patient as well as compounds potency, severity of the disease being treated. For example, a dosage regimen of the compoundss can be oral administration of from 10 mg to 2000 mg/day, preferably 10 to 1000 mg/day, more preferably 50 to 600 mg/day, in two to four (preferably two) divided doses. Intermittent therapy (e.g., one week out of three weeks or three out of four weeks) may also be used.

It is understood that the examples and embodiments described herein are for illustrative purposes only.

### Examples

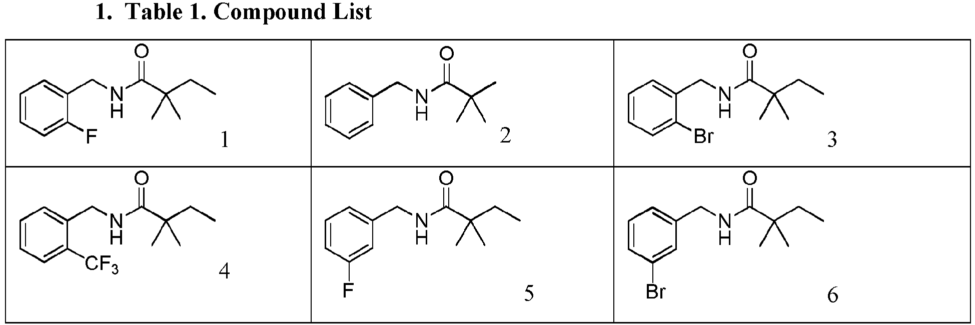

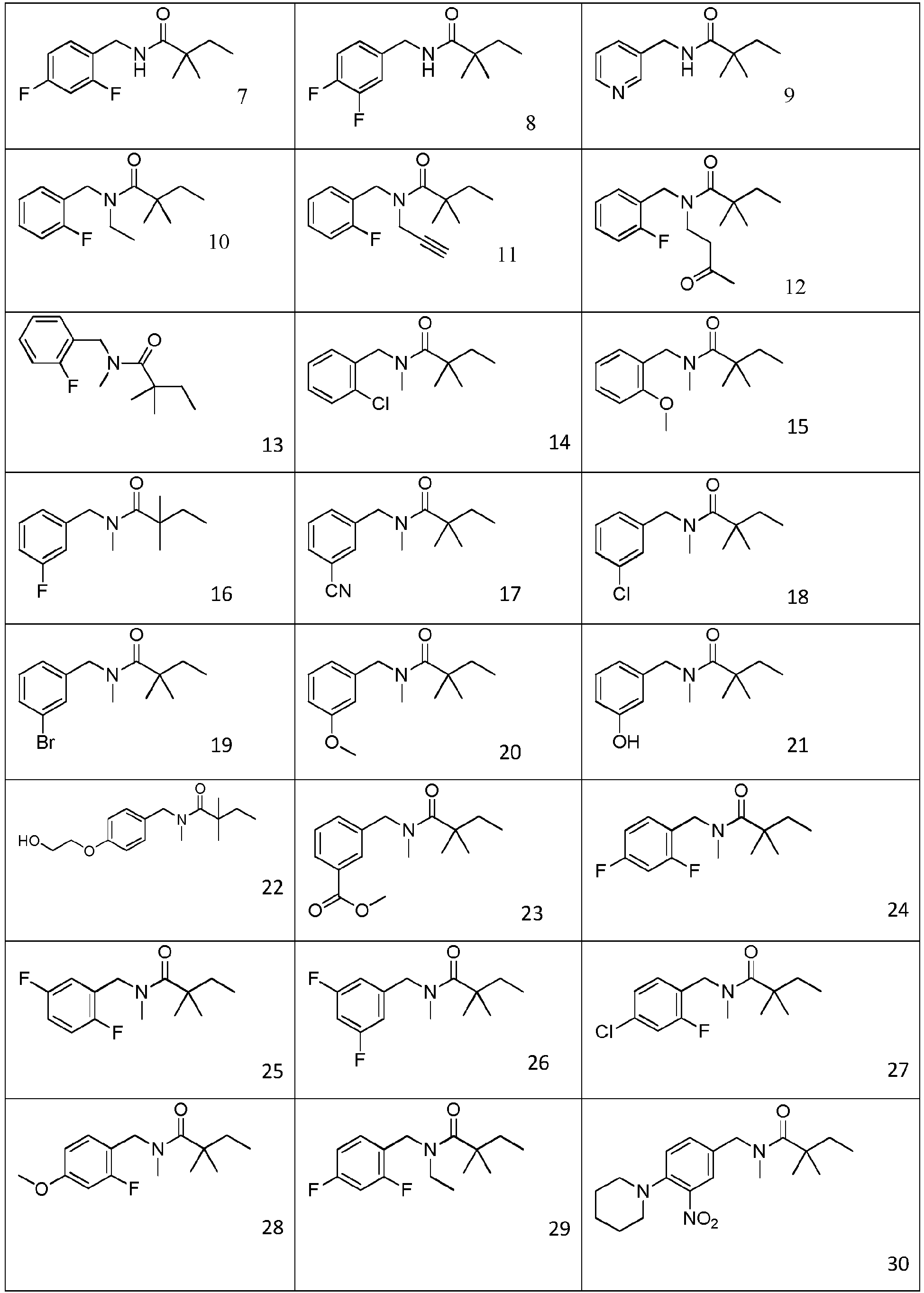

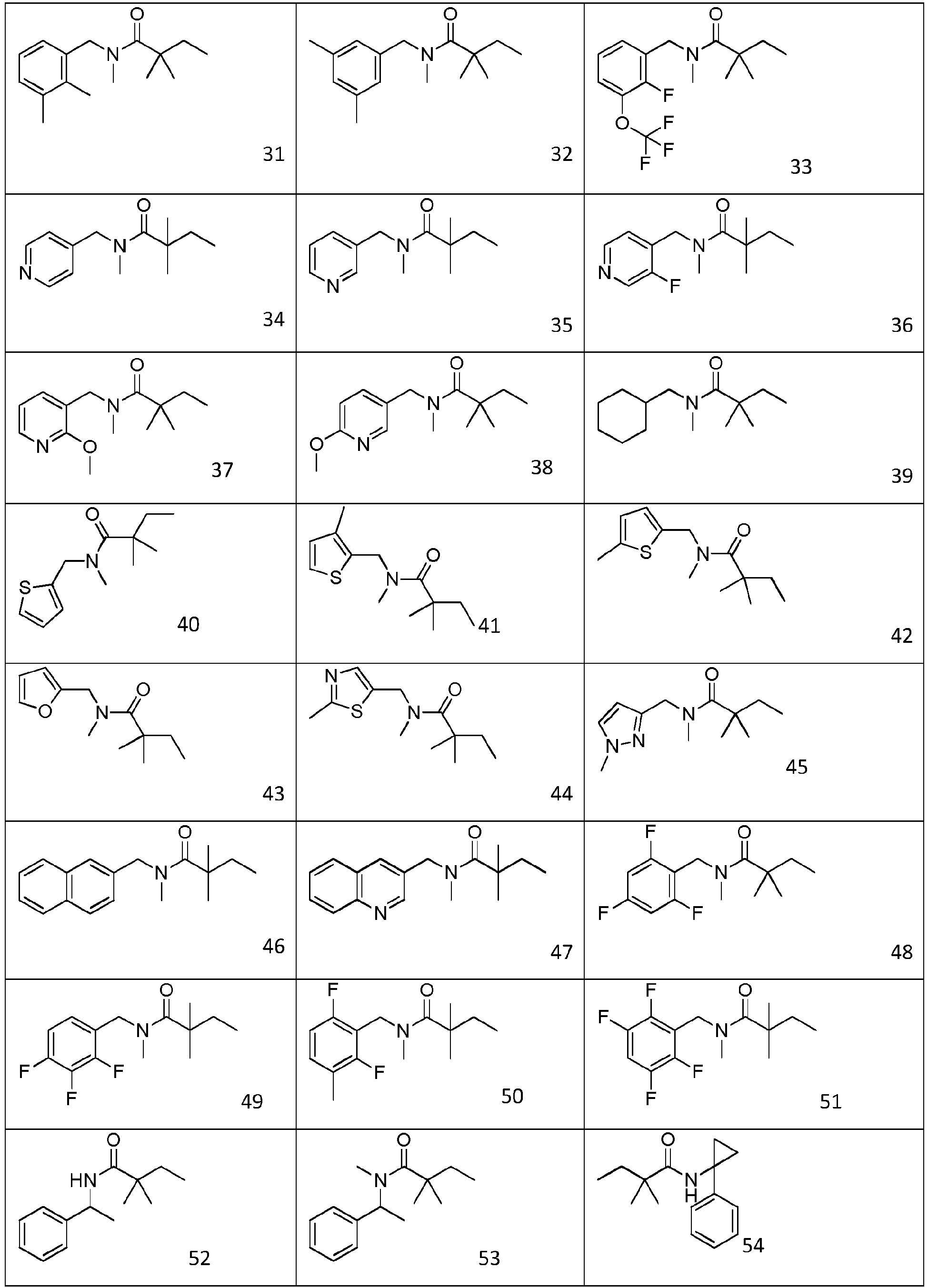

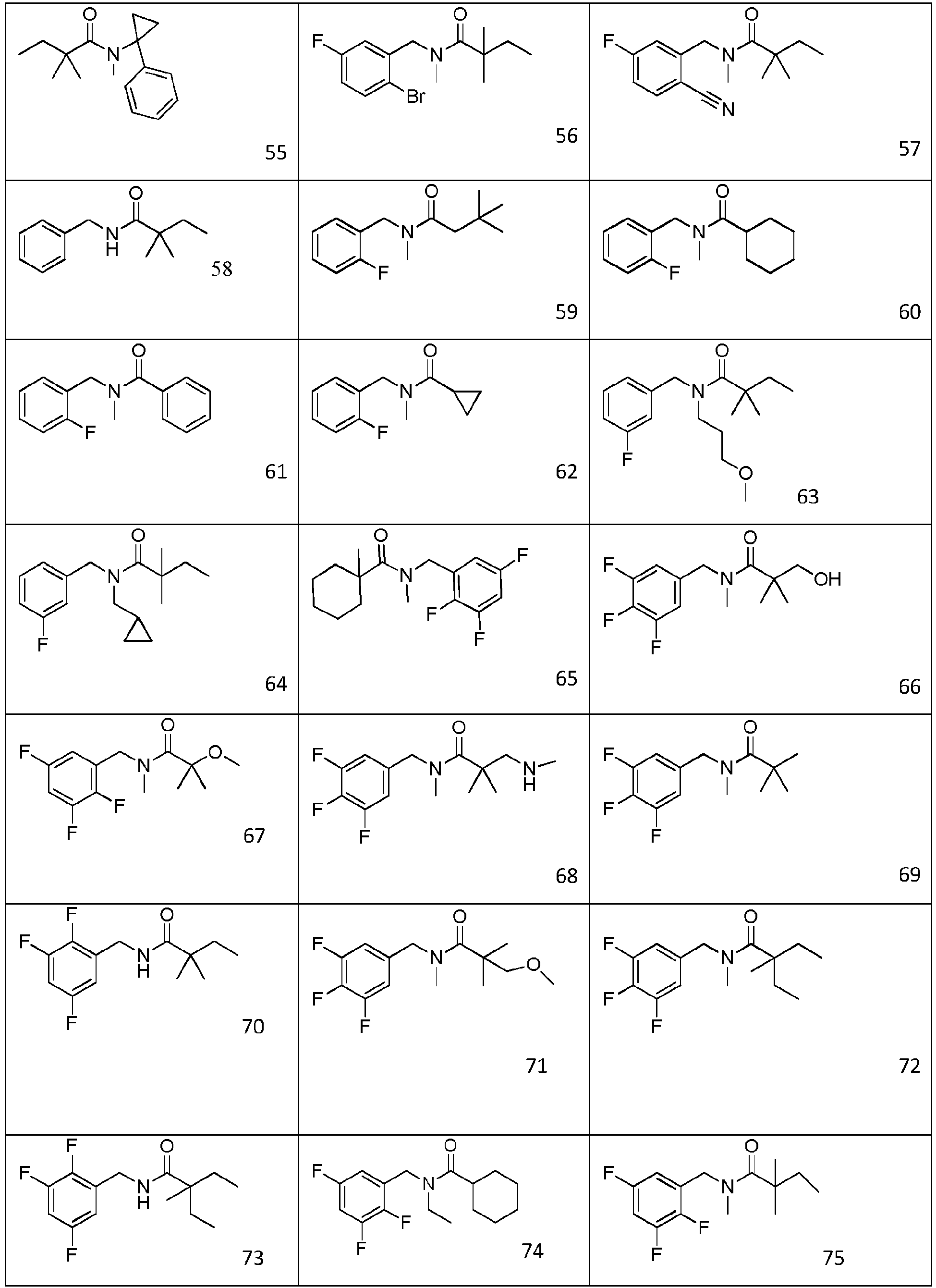

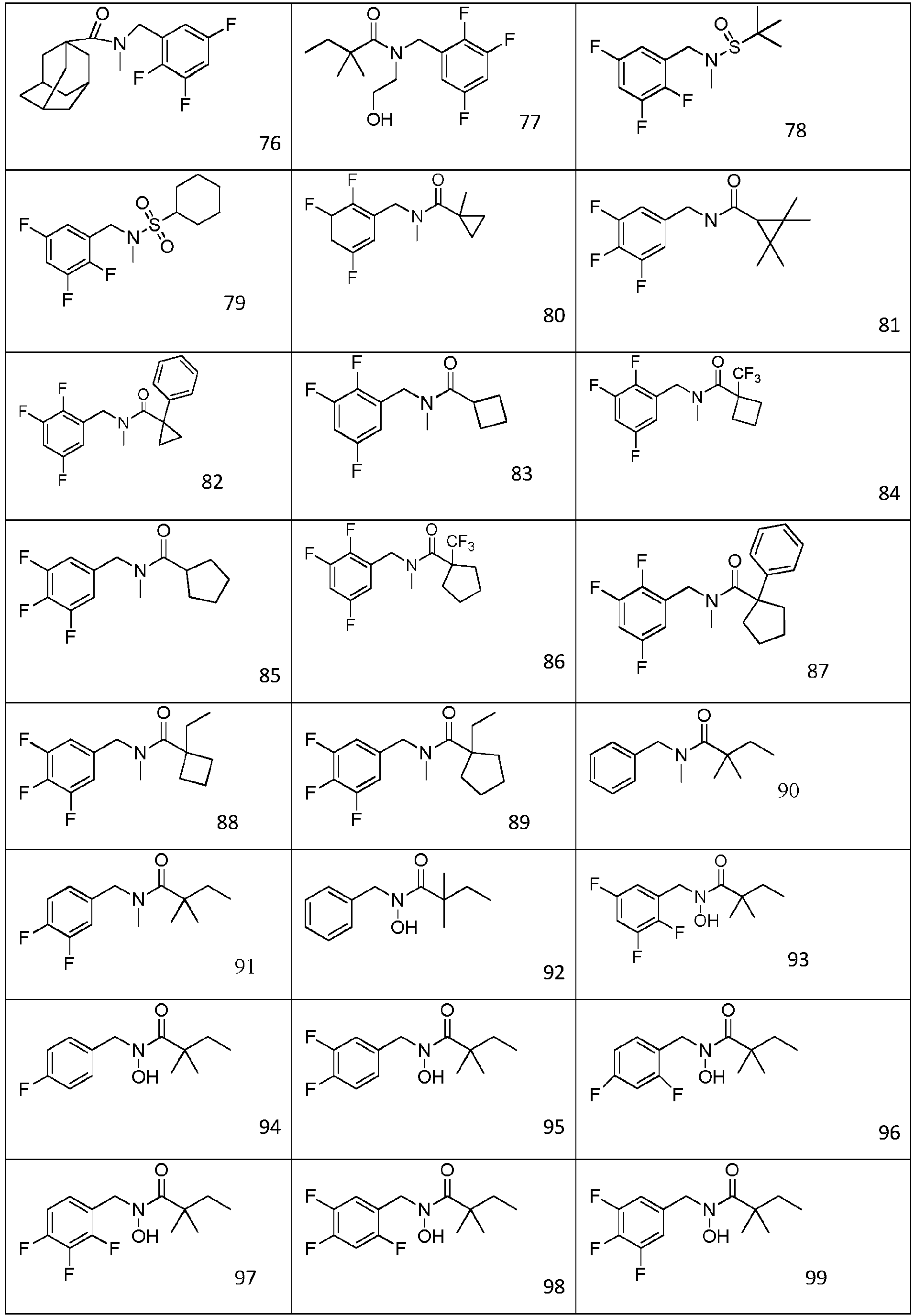

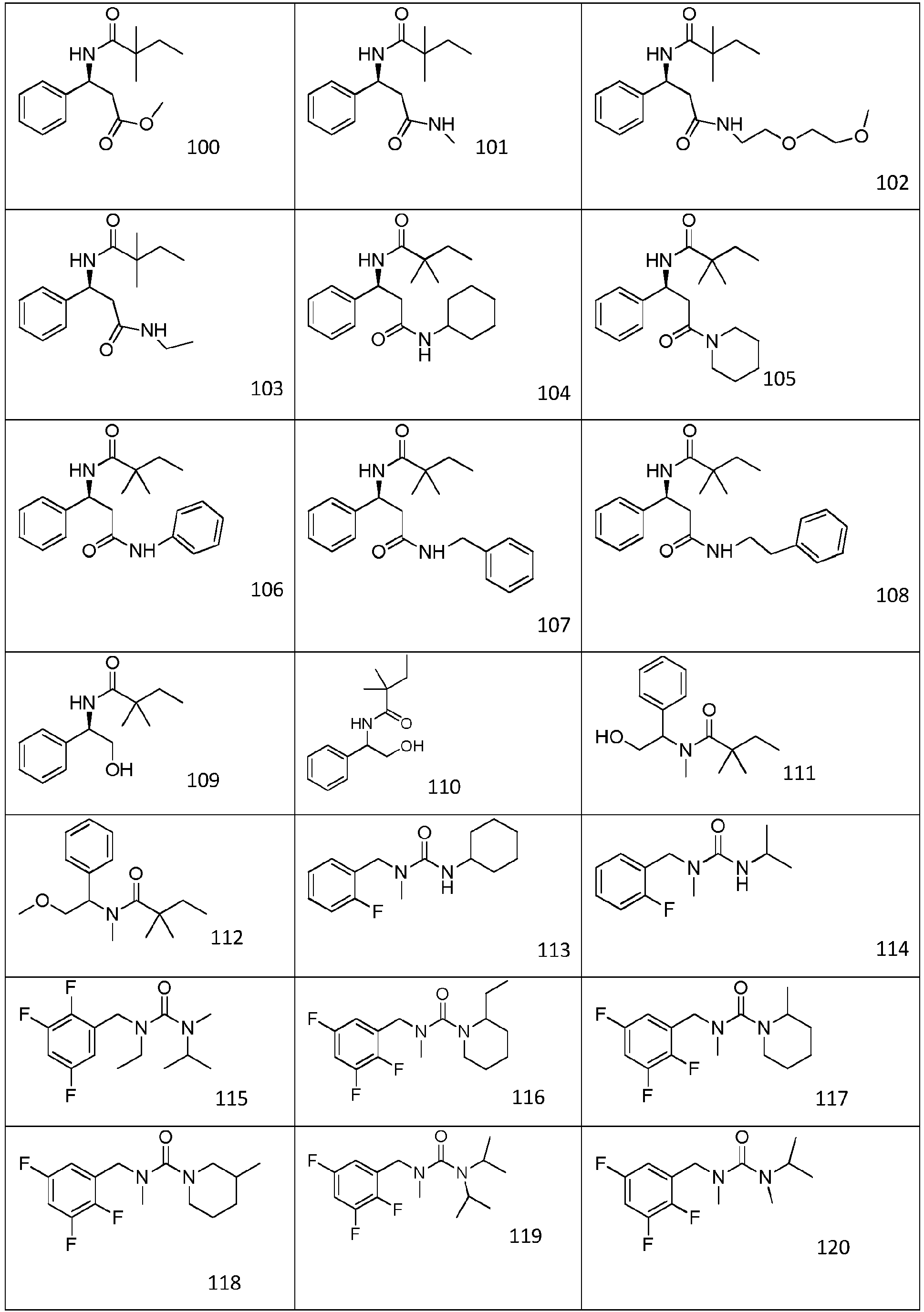

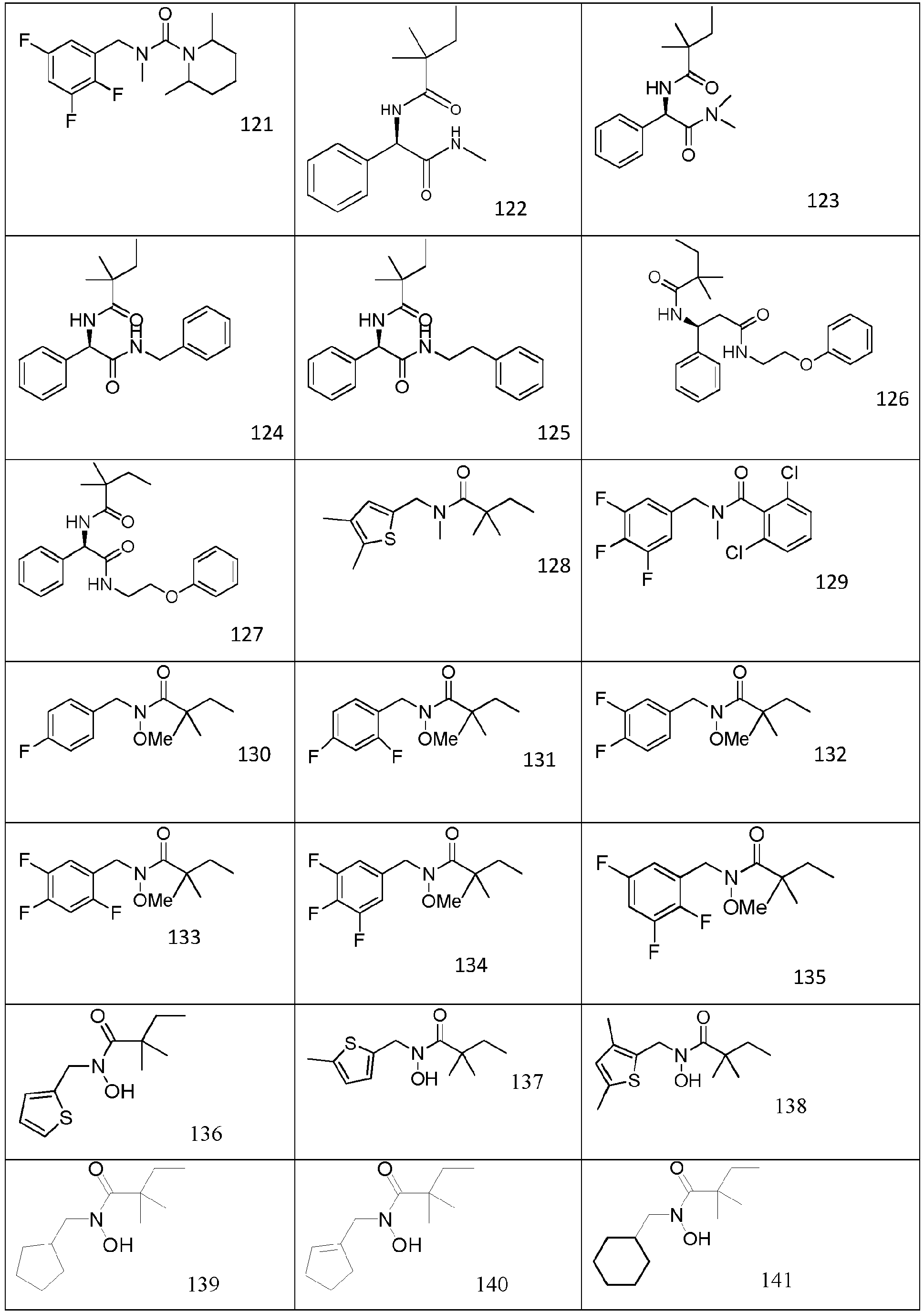

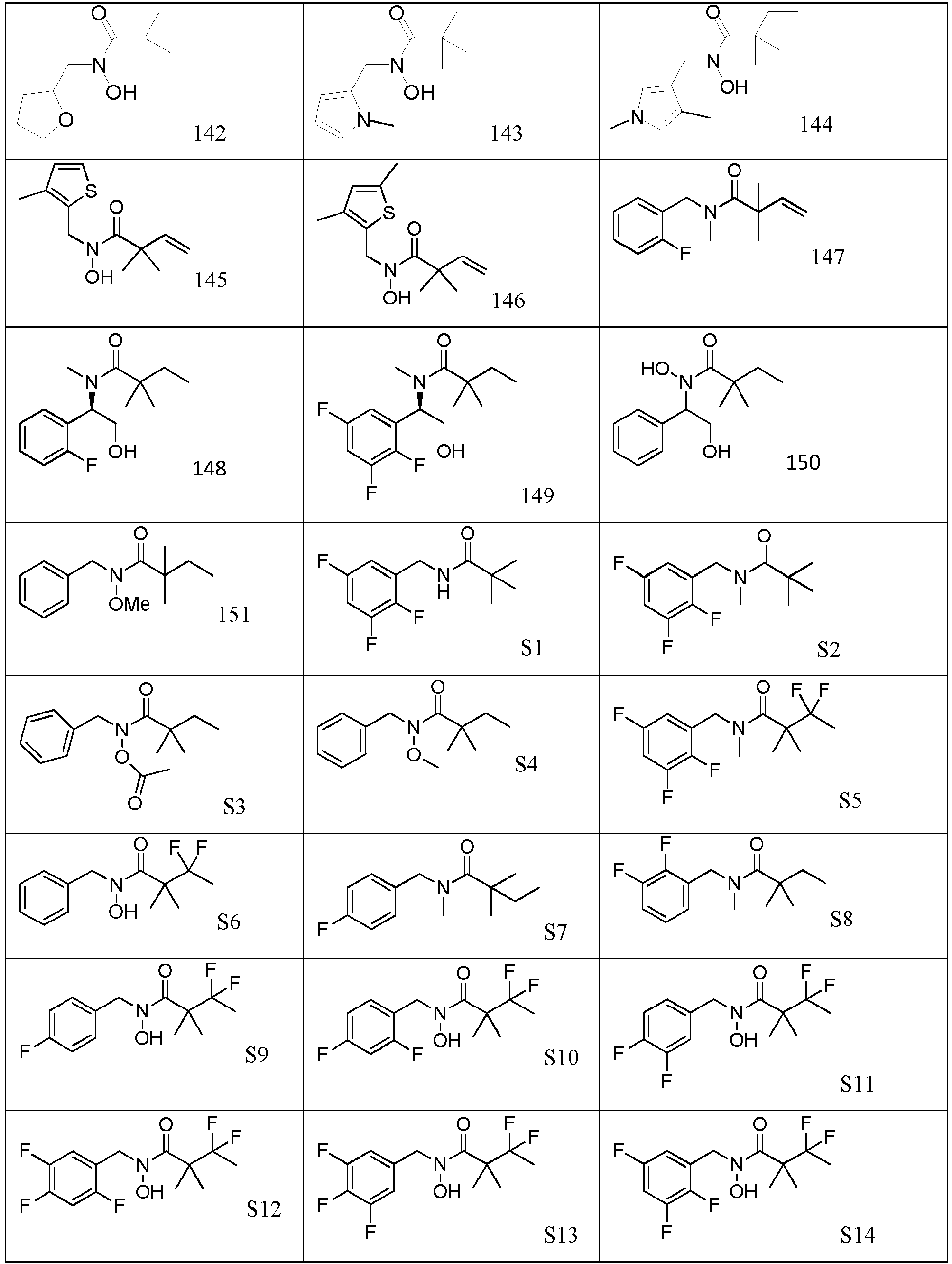

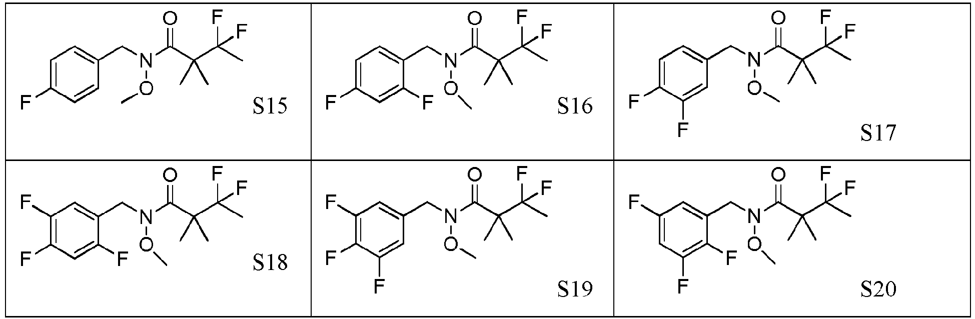

It is hereby made clear that among all the compounds of Table 1 disclosed here-above, as well as their process for the preparation described below, **only** compounds 92-99 are part of the invention. The remaining compounds and preparation thereof are only disclosed for illustrative/exemplary purposes.

### 2. Compound Preparation.

### Compound 1:

### Preparation of N-(2-fluorobenzyl)-2,2-dimethylbutanamide

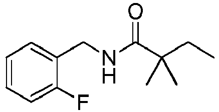

SOCl₂ (30ml) was added to the 2, 2-dimethylbutanoic acid (5.22g) in toluene with stirring. Then the reaction mixture was warmed to 80 °C for 5 h. After removal of the solvent, 4.268g of 2,2-dimethylbutanoyl chloride was obtained, which was dissolved in DCM and added dropwise to the (2-fluorophenyl)methanamine (1.698g) dissolved in DCM contains TEA (4.8g) at 0°C. Stirring was continued at room temperature for 10 h. After all amines had been consumed as judged by TLC, the mixture was quenched with ice-water. Extracted with DCM, dried over Na₂SO₄, concentrated and purified by silca gel chromatography to give the desired product (2.57g, 72.6%). ¹H NMR (CDCl₃): δ 7.30-7.34 (m, 1H), 7.23-7.25 (m, 1H), 7.00-7.11 (m, 2H), 6.06 (br, 1H), 4.48 (d, 2H, *J* = 6.0 Hz), 1.55 (q, 2H, *J* = 7.6 Hz), 1.16 (s, 6H), 0.81 (t, 3H, *J*=7.6 Hz).

### Compound 2:

### Preparation of N-benzylpivalamide

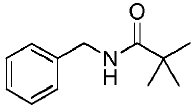

Phenylmethanamine( 80.3 mg ) and triethylamine ( 0.625ml ) were dissolved in DCM (2 ml), pivaloyl chloride (120 mg ) was added at 0°C and the mixture was stirred at room temperature for 3h.The mixture was quenched with ice-water. Extracted with DCM, dried over Na₂SO₄, concentrated and purified by silca gel chromatography to give the desired product ( 58.6 mg, 40.9% ). ¹H NMR (CDCl₃): δ 7.25-7.37 (m, 5H), 5.89 (br, 1H), 4.45 (d, 2H, *J* = 5.6 Hz), 1.23 (s, 9H).

### Compound 3:

### Preparation of N-(2-bromobenzyl)-2,2-dimethylbutanamide

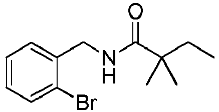

The titled compound 3 was prepared in 58.3% from (2-bromophenyl)methanamine ( 93mg ) and 2,2-dimethylbutanoyl chloride (80.76mg ) according to the procedure outlined for compound 1. ¹H NMR (CDCl₃): δ 7.55 (d, 1H, *J*= 8.0 Hz), 7.37-7.40 (m, 1H), 7.26-7.30 (m, 1H), 7.12-7.17 (m, 1H) 6.14 (br, 1H), 4.50 (d, 2H, *J* = 6.0 Hz), 1.55 (q, 2H, *J* = 7.6 Hz), 1.16 (s, 6H), 0.80 (t, 3H, *J*=7.6 Hz).

### Compound 4 Preparation of 2,2-dimethyl-N-(2-(trifluoromethyl)benzyl)butanamide

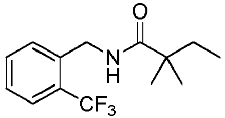

The titled compound 4 was prepared in 71% from 2-(trifluoromethyl)phenyl)-[0141] methanamine( 87.6mg ) and 2,2-dimethylbutanoyl chloride (105 mg ) according to the procedure outlined for compound 1. ¹H NMR (CDCl₃): δ 7.64-7.66 (d, 1H, *J* = 8.0 Hz), 7.50-7.56 (m, 2H), 7.36-7.40 (m, 1H), 5.97 (br, 1H), 4.62 (d, 2H, *J* = 4.8 Hz), 1.55 (q, 2H, *J* = 7.6 Hz), 1.15 (s, 6H), 0.80 (t, 3H, J=7.6 Hz).

### Compound 5 Preparation of N-(3-fluorobenzyl)-2,2-dimethylbutanamide

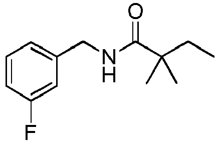

### The titled compound 5 was prepared in 70% yield from

(3-fluorophenyl)methanamine ( 93mg ) and 2,2-dimethylbutanoyl chloride (80.74 mg ) according to the procedure outlined for compound 1.¹H NMR (400 MHz, CDCl₃) δ :7.33 - 7.27 (m, 1H), 7.04 (d, *J* = 8.0 Hz, 1H), 6.93-6.98 (m, 2H), 4.45 (d, *J* = 5.8 Hz, 2H), 1.58 (q, *J* = 7.5 Hz, 3H), 1.19 (s, 6H), 0.86 (t, *J* = 7.5 Hz, 3H).

### Compound 6 Preparation of N-(3-bromobenzyl)-2,2-dimethylbutanamide

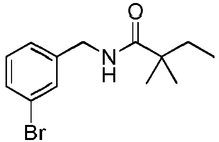

The titled compound 6 was prepared in 87% from (3-bromophenyl)methanamine ( 93mg ) and 2,2-dimethylbutanoyl chloride (105 mg ) according to the procedure outlined for compound 1. ¹H NMR (CDCl₃): δ 7.38-7.41 (m, 2H), 7.17-7.21 (m, 2H), 5.96 (br, 1H), 4.42 (d, 2H, *J* = 6.0 Hz), 1.55 (q, 2H, *J* = 7.6 Hz), 1.15 (s, 6H), 0.80 (t, 3H, *J*=7.6 Hz).

### Compound 7 Preparation of N-(2,4-difluorobenzyl)-2,2-dimethylbutanamide

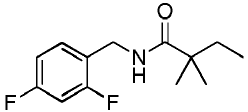

The titled compound 7 was prepared in 40.9% yield form (2,4-difluorophenyl)methanamine ( 228.8mg ) and 2,2-dimethylbutanoyl chloride (430.3 mg) according to the procedure outlined for compound 1. ¹H NMR (CDCl₃): δ 7.30-7.36 (m, 1H), 6.77-6.86 (m, 2H), 5.97 (br, 1H), 4.44 (d, 2H, *J* = 6.0 Hz), 1.55 (q, 2H, *J* = 7.6 Hz), 1.17 (s, 6H), 0.80 (t, 3H, *J* = 7.6 Hz).

### Compound 8 Preparation of N-(3,4-difluorobenzyl)-2,2-dimethylbutanamide

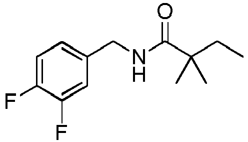

The titled compound 8 was prepared in 71.3% yield form (3,4-difluorophenyl)methanamine ( 114.4mg ) and 2,2-dimethylbutanoyl chloride (215mg )according to the procedure outlined for compound 1. ¹H NMR (CDCl₃): δ 7.06-7.14 (m, 2H), 6.97-7.00 (m, 1H), 5.95 (br, 1H), 4.40 (d, 2H, *J* = 6.0 Hz), 1.56 (q, 2H, *J* = 7.6 Hz), 1.17 (s, 6H), 0.84 (t, 3H, *J* = 7.6 Hz).

### Compound 9 Preparation of 2,2-dimethyl-N-(pyridin-3-ylmethyl)butanamide

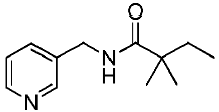

The titled compound 9 was prepared in 51.2% yield form pyridin-3-ylmethanamine ( 54.07 mg ) and 2,2-dimethylbutanoyl chloride ( 134.6 mg )according to the procedure outlined for compound 1. ¹H NMR (CDCl₃): δ 8.52 (s, 2H) 7.61-7.64 (m, 1H), 7.25-7.28 (m, 1H), 6.05 (br, 1H), 4.47 (d, 2H, *J* = 6.0 Hz), 1.58 (q, 2H, *J* = 7.6 Hz), 1.18 (s, 6H), 0.84 (t, 3H, *J* = 7.6 Hz).

### Compound 10 Preparation of N-ethyl-N-(2-fluorobenzyl)-2,2-dimethylbutanamide

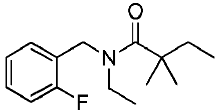

N-(2-fluorobenzyl)-2,2-dimethylbutanamide ( 40mg) was dissolved in 4 ml of dry DMF, 8.61mg of NaH was added at 0°C under N2 and stirred for 2h. Iodoethane (56.2 mg ) was added and the mixture was allowed to warm to room temperature and stirred for 11h. The mixture was quenched with cold water and extracted with DCM, the combined organic layers was washed with water, brine, dried over Na₂SO₄, concentrated and the residue was purified by silica gel column chromatography to give the product ( 9.3mg, 20.6%). ¹H NMR (CDCl₃): δ 7.22-7.26 (m, 2H), 7.03-7.12 (m, 2H), 4.69 (s, 2H), 3.43 (d, 2H, *J* = 5.2 Hz), 1.67 (q, 2H, *J=* 7.6 Hz), 1.26 (s, 6H), 1.17 (t, 3H, *J*=6.8 Hz), 0.89 (t, 3H, *J*=7.6 Hz).

### Compound 11 Preparation of N-(2-fluorobenzyl)-2,2-dimethyl-N-(prop-2-yn-1-yl)butanamide

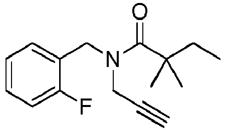

### N-(2-fluorobenzyl)prop-2-yn-1-amine was prepared in 42% yield according to the procedure outlined for compound 10, 68.2 mg of the amide was used as starting

Material and reacted with 2,2-dimethylbutanoyl chloride ( 170mg ) and the desired compound 11 was prepared in 30% yield. ¹H NMR (CDCl₃): δ 7.23-7.26 (m, 2H), 7.03-7.13 (m, 2H), 4.83 (s, 2H), 4.15 (d, 2H, *J* = 2.4 Hz), 2.23 (t, 1H, *J* = 2.4 Hz), 1.70 (q, 2H, *J* = 7.6 Hz), 1.29 (s, 6H), 0.89 (t, 3H, *J* =7.6 Hz).

### Compound 12 Preparation of N-(2-fluorobenzyl)-2,2-dimethyl-N-(3-oxobutyl)butanamide

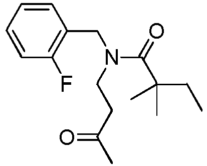

A mixture of (2-fluorophenyl)methanamine (125mg), paraformaldehyde(36mg), acetone(116mg) and concentrated hydrochloric acid(0.1ml) in EtOH(1ml) was heated in a sealed flask at 110°C for 16h After the mixture was cooled to room temperature, the solvent was removed and EtOAc was added, the resulting suspension was vigorously stirred for 1h and then filtered and washed with EtOAc to afford 200mg of 4-((2-fluorobenzyl)amino)butan-2-one, which was used directly in the next step without further purification.

The resulting amide (200mg) was dissolve in dry THF (10ml), and TEA (0.3ml) was added. The mixture was cooled to 0°C, 2,2-dimethylbutanoylchloride (274mg) was added and stirred for 4 h at room temperature. The mixture was quenched with water and extracted with EtOAc. The combined organic layer were washed with brine and dried over Na₂SO₄. After removal of the solvent, the residue was purified by silica gel chromatography to afford the compound 12 (180mg, 60%). ¹H NMR (CDCl₃): δ 7.23-7.28 (m, 1H), 7.10-7.19 (m, 2H), 7.02-7.07 (m, 1H), 4.75 (s, 2H), 3.54 (br, 2H), 2.77 (t, 2H, *J* = 7.2 Hz), 2.13 (s, 3H), 1.66 (q, 2H, *J=* 7.6 Hz), 1.23 (s, 6H), 0.89 (t, 3H, *J* =7.6 Hz).

### Compound 13: Preparation of N-(2-fluorobenzyl)-N,2,2-trimethylbutanamide

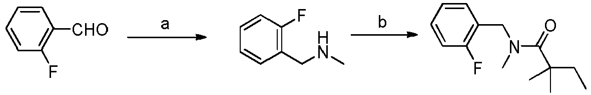

Reagent and conditions: (a): (1) CH₃NH₂.HCl, K₂CO₃, MeOH, rt, 1.5h; (b): NaBH₄ (c) 2, 2-dimethylbutanoyl chloride, DIEA, THF, rt, 2h.

A mixture of K₂CO₃ (207 mg, 1.5 mmoL) and methanamine hydrochloride (202mg, 3.0 mmoL) in 5 mL of MeOH was stirred at room temperature for 30 min. Then 2-fluorobenzaldehyde (248 mg, 2.0 mmoL) was added to the mixture and stirred at room temperature for 1 h. The mixture was cooled to 0°C, and NaBH₄ (113.5 mg, 3.0 mmoL) was added in portions. The mixture was stirred at 0°C for 1h. The solid was filtered and washed with EtOAc. The filtrate was evaporated to dryness and the
residue was dissolved in EtOAc and was washed with water, brine, dried over Na₂SO₄. The residue was dissolved in 10mL of dry THF. DIEA (264 mg, 2.05 mmoL) was added, 2,2-dimethylbutanoyl chloride (275 mg, 2.05 mmoL) was added slowly to the solution at 0°C under nitrogen, then stirred at room temperature for 2 h. 15 mL of water was added to the solution and extracted with EtOAc (10 mL x 3). The combined organic was washed with 1M HCl, brine, dried with Na₂SO₄ and concentrated in vacuo. The residue was purified by silica gel column chromatography (PE/EA= 1/2) to give the 230 mg of 1 as a brown solid (total yield = 45.1%). ¹H NMR(CDCl₃, 400 M Hz):*δ* (ppm)7.22-7.28 (m, 2H), 7.01-7.12 (m, 2 H), 4.68 (s, 2 H), 3.05(s, 3H), 1.65 (q, 2H, J = 7.6 Hz), 1.27(s, 6H), 0.89(t, 3H, J = 7.6 Hz)LC-MS (ESI) [M+H] ⁺calad for C₁₄H₂₀FNO, 238.2; found, 238.4.

### Compound 14: Preparation of N-(2-chlorobenzyl)-N,2,2-trimethylbutanamide

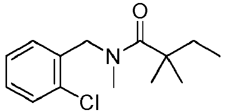

The titled compound 14 was prepared in 48% yield from 2-chlorobenzaldehyde(281mg), methanamine hydrochloride (202mg) and 2,2-dimethylbutanoyl chloride(275mg) according to the procedure outlined for compound 13.¹H NMR(CDCl₃, 400 M Hz):δ7.35-7.37 (m, 1 H), 7.16-7.25 (m, 3 H), 4.74 (s, 2 H), 3.05 (s, 3 H), 1.70 (q, 2H, J = 7.6 Hz), 1.28 (s, 6 H), 0.91 (t, 3 H, J = 7.6 Hz).LC-MS (ESI) [M+H] ⁺calad forC₁₄H₂₀ClNO, 254.2 ;found, 254.4.

### Compound 15: Preparation of N-(2-methoxybenzyl)-N,2,2-trimethylbutanamide

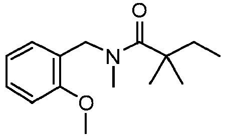

The titled compound 15 was prepared in 65% yield from 2-methoxybenzaldehyde (136mg), methanamine hydrochloride(101mg) and2,2-dimethylbutanoyl chloride(140 mg) according to the procedure outlined for compound 13.¹H NMR(CDCl₃, 400 M Hz):*δ*7.21-7.26 (m, 1 H), 7.09-7.13 (m, 1 H), 6.86-6.95 (m, 2 H), 4.66 (s, 2 H), 3.83 (s, 3 H), 2.99 (s, 3 H), 1.68 (q, 2H, J = 7.6 Hz), 1.26 (s, 6 H), 0.90 (t, 3 H, J = 7.6 Hz)LC-MS (ESI) [M+H] ⁺calad for C₁₅H₂₃NO₂, 250.1; found 250.3.

### Compound 16: Preparation of N-(3-fluorobenzyl)-N,2,2-trimethylbutanamide

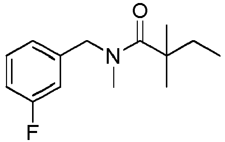

The titled compound 16 was prepared in 65% yield from 3-fluorobenzaldehyde (124mg), methanamine hydrochloride (101mg) and 2,2-dimethylbutanoyl chloride(140mg) according to the procedure outlined for compound 13. ¹H NMR(CDCl₃, 400 M Hz):*δ*7.25-7.31 (m, 1 H), 6.91-7.01 (m, 3 H), 4.61 (s, 2 H), 3.01 (s, 3 H), 1.69 (q, 2 H, J = 7.6 Hz), 1.29 (s, 6 H), 0.90 (t, 3 H, J = 7.6 Hz).LC-MS (ESI) [M+H] ⁺calad for C₁₄H₂₀FNO, 238.1; found 238.4.

### Compound 17: Preparation of N-(3-cyanobenzyl)-N,2,2-trimethylbutanamide

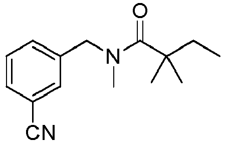

The titled compound 17 was prepared in 62% yield from 3-formylbenzonitrile (131mg), methanamine hydrochloride (101mg) and 2,2-dimethylbutanoyl chloride(140mg) according to the procedure outlined for compound 13. ¹H NMR (CDCl₃, 400 M Hz):*δ*7.41-7.56 (m, 4 H), 4.61(s, 2 H), 3.05 (s, 3 H), 1.68 (q, 2 H, J = 7.6 Hz), 1.28 (s, 6 H), 0.88 (t, 3 H, J = 7.6 Hz). LC-MS (ESI)[M+H] ⁺calad forC₁₅H₂₀N₂O, 245.1; found 245.3.

### Compound 18: Preparation of N-(3-chlorobenzyl)-N,2,2-trimethylbutanamide

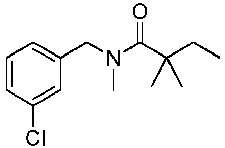

The titled compound 18 was prepared in 48% yield from 3-chlorobenzaldehyde (140mg), methanamine hydrochloride (101mg) and 2,2-dimethylbutanoyl chloride(140mg) according to the procedure outlined for compound 13. ¹H NMR(CDCl₃, 400 M Hz):δ7.20-7.26 (m, 3 H), 7.10-7.12 (m, 1 H), 4.59 (s, 2 H), 3.01 (s, 3 H), 1.68 (q, 2 H, J = 7.6 Hz), 1.28 (s, 6 H), 0.90 (t, 3 H, J = 7.6 Hz).LC-MS (ESI) [M+H] ⁺calad forC₁₄H₂₀ClNO, 254.1; found 254.3.

### Compound 19: Preparation of N-(3-bromobenzyl)-N,2,2-trimethylbutanamide

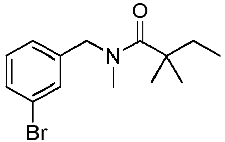

The titled compound 19 was prepared in 48% yield from 3-bromobenzaldehyde (185 mg), methanamine hydrochloride (101mg) and 2,2-dimethylbutanoyl chloride(140mg) according to the procedure outlined for compound 13. ¹H NMR (CDCl₃, 400 M Hz): δ 7 7.36-7.40 (m, 2 H), 7.16-7.22 (m, 2 H), 4.59 (s, 2 H), 3.01 (s, 3 H), 1.69 (q, 2 H, J = 7.6 Hz), 1.29 (s, 6 H), 0.90 (t, 3 H, J = 7.6 Hz).LC-MS (ESI) [M+H] ⁺calad for C₁₄H₂₀BrNO, 298.1; found, 298.3, 300.4.

### Compound 20: Preparation of N-(3-methoxybenzyl)-N,2,2-trimethylbutanamide

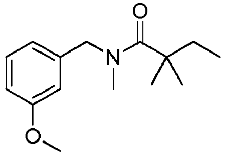

The titled compound 8 was prepared in 57% yield from 3-methoxybenzaldehyde(136mg), methanamine hydrochloride(101mg) and 2,2-dimethylbutanoyl chloride(140mg) according to the procedure outlined for compound 13.¹H NMR(CDCl₃, 400 M Hz):δ7.22-7.25 (m, 1 H), 6.76-6.81 (m, 3 H), 4.61 (s, 2 H), 3.78 (s, 3 H), 2.98 (s,3 H), 1.69 (q, 2 H, J = 7.6 Hz), 1.29 (s, 6 H), 0.91 (t, 3 H, J = 7.6 Hz).LC-MS (ESI) [M+H] ⁺calad forC₁₅H₂₃NO₂, 250.2; found 250.4.

### Compound 21: Preparation of N-(3-hydroxybenzyl)-N,2,2-trimethylbutanamide

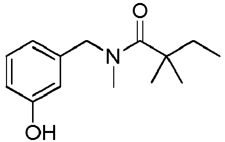

The titled compound 21 was prepared in 33% yield from 3-hydroxybenzaldehyde(122mg), methanamine hydrochloride(101mg) and 2,2-dimethylbutanoyl chloride(140mg) according to the procedure outlined for compound 1.¹H NMR(CDCl₃, 400 M Hz):δ7.17 (t, 1 H, J = 7.6 Hz), 6.71-6.79 (m, 3 H), 4.58 (s, 2 H), 2.98 (s, 3 H), 1.68 (q, 2 H, J = 7.6 Hz), 1.29 (s, 6 H), 0.90 (t, 3 H, J = 7.6 Hz).LC-MS (ESI) [M+H] ⁺calad forC₁₄H₂₁NO₂, 236.2; found, 236.4.

### Compound 22: Preparation of N-(3-(2-hydroxyethoxy)benzyl)-N,2,2-trimethylbutanamide

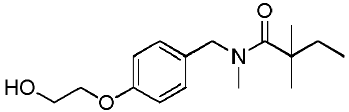

The titled compound 22 was prepared in48% yield from 4-(2-hydroxyethoxy)benzaldehyde (166mg), methanamine hydrochloride(101mg) and 2,2-dimethylbutanoyl chloride(140mg) according to the procedure outlined for compound 13. ¹H NMR(CDCl₃,400 M Hz):δ7.14-7.17 (m, 2 H), 6.85-6.89 (m, 2 H), 4.56 (s, 2 H), 4.06-4.08 (m, 2 H), 3.94-3.96 (m, 2 H), 2.96 (s, 3 H), 1.67 (q, 2 H, J = 7.6 Hz), 1.27 (s, 6 H), 0.88 (t, 3 H, J = 7.6 Hz). LC-MS (ESI) [M+H] ⁺calad forC₁₆H₂₅NO₃, 280.2; found, 280.4.

### Compound 23:

### Preparation of methyl 3-((N,2,2-trimethylbutanamido)methyl)benzoate

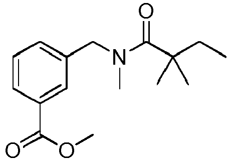

The titled compound 23 was prepared in 46% yield from methyl 3-formylbenzoate (164mg), methanamine hydrochloride(101mg) and 2,2-dimethylbutanoyl chloride(140mg) according to the procedure outlined for compound 13.¹H NMR(CDCl₃, 400 M Hz):δ7.87-7.94(m, 2 H), 7.38-7.45 (m, 2 H), 4.66 (s, 2 H), 3.90 (s, 3 H), 3.01 (s, 3 H), 1.69 (q, 2 H, J = 7.6 Hz), 1.29 (s, 6 H), 0.90 (t, 3 H, J = 7.6 Hz).LC-MS (ESI) [M+H] ⁺calad for: C₁₆H₂₃NO₃, 278.2; found, 278.4.

### Compound 24: Preparation of N-(2,4-difluorobenzyl)-N,2,2-trimethylbutanamide

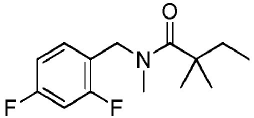

The titled compound 24 was prepared in 56% yield from 2,4-difluorobenzaldehyde (284mg), methanamine hydrochloride(202mg) and 2,2-dimethylbutanoyl chloride(275mg) according to the procedure outlined for compound 13.¹H NMR(CDCl₃, 400 M Hz):δ7.25-7.31 (m, 1 H), 6.76-6.86 (m, 2 H), 4.60 (s, 2 H), 3.06 (s, 3 H), 1.65 (q, 2 H, J= 7.6 Hz), 1.26 (s, 6 H), 0.85 (t, 3 H, J= 7.6 Hz ).LC-MS (ESI) [M+H] ⁺calad for: C₁₄H₁₉F₂NO, 256.1; found , 256.3.

### Compound 25: Preparation of N-(2,5-difluorobenzyl)-N,2,2-trimethylbutanamide

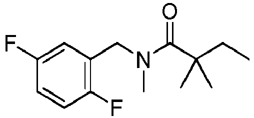

The titled compound 25 was prepared in 59% yield from 2,5-difluorobenzaldehyde (284mg), methanamine hydrochloride(202mg) and 2,2-dimethylbutanoyl chloride(275mg) according to the procedure outlined for compound 13. ¹H NMR (CDCl₃, 400 M Hz): δ6.89-7.02 (m, 3 H), 4.63 (s, 2 H), 3.08 (s, 3 H), 1.68 (q, 2 H, J = 7.6 Hz), 1.28 (s, 6 H), 0.88 (t, 3 H, J = 7.6 Hz).LC-MS (ESI) [M+H] ⁺calad for: C₁₄H₁₉F₂NO, 256.1; found, 256.4.

### Compound 26: Preparation of N-(3,5-difluorobenzyl)-N,2,2-trimethylbutanamide

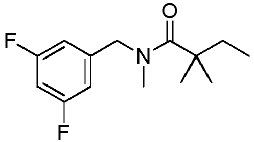

The titled compound 14 was prepared in 59% yield from 3,5-difluorobenzaldehyde (284mg), methanamine hydrochloride(202mg) and 2,2-dimethylbutanoyl chloride(275mg) according to the procedure outlined for compound 1.¹H NMR(CDCl₃, 400 M Hz):δ6.89-6.75 (m, 3 H), 4.57 (s, 2 H), 3.04 (s, 3 H), 1.69 (q, 2 H, J = 7.6 Hz), 1.29 (s, 6 H), 0.90 (t, 3 H, J = 7.6 Hz). LC-MS (ESI) [M+H] ⁺calad for: C₁₄H₁₉F₂NO, 256.1; found, 256.3.

### Compound 27: Preparation of N-(4-chloro-2-fluorobenzyl)-N,2,2-trimethylbutanamide

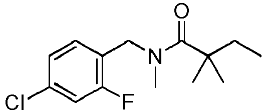

The titled compound 27 was prepared in 59% yield from4-chloro-2-fluorobenzaldeyde (316mg), methanamine hydrochloride (202mg) and 2,2-dimethylbutanoylchloride(275mg) according to the procedure outlined for compound 13. ¹H NMR (CDCl₃, 400 M Hz): δ7.21-7.26 (m, 1 H), 7.05-7.11 (m, 2 H), 4.60 (s, 2 H), 3.06 (s, 3 H), 1.65 (q, 2 H, J = 7.6 Hz), 1.26 (s, 6 H), 0.85 (t, 3 H, J = 7.6 Hz). LC-MS (ESI) [M+H] ⁺calad for: C₁₄H₁₉ClFNO, 272.1; found, 272.4.

### Compound 28: Preparation of N-(2-fluoro-4-methoxybenzyl)-N,2,2-trimethylbutanamide

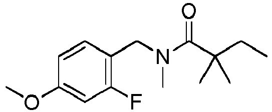

The titled compound 16 was prepared in 57% yield from2-fluoro-4-methoxybenzaldehyde (208mg), methanamine hydrochloride (202mg) and 2,2-dimethylbutanoyl chloride(275mg) according to the procedure outlined for compound 13. ¹H NMR(CDCl₃, 400 M Hz):δ7.20 (t, 1 H, J = 8.8 Hz), 6.57-6.67 (m, 2 H), 4.59 (s, 2 H), 3.78 (s, 3 H), 3.01 (s, 3 H), 1.65 (q, 2 H, J = 7.6 Hz), 1.26 (s, 6 H), 0.86 (t, 3 H, J = 7.6 Hz).LC-MS (ESI) [M+H] ⁺calad for:C₁₅H₂₂FNO₂, 268.2; found ,268.4.

### Compound 29 (for exemplary purposes only): Preparation of N-(2,4-difluorobenzyl)-N-ethyl-2,2-dimethylbutanamide

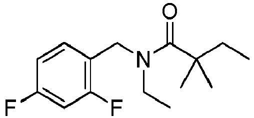

The titled compound 29 was prepared in 57% yield from2,4-difluorobenzaldehyde(284mg), ethylamine hydrochloride(248mg) and 2,2-dimethylbutanoyl chloride(275mg) according to the procedure outlined for compound 13. ¹H NMR(CDCl₃, 400 M Hz):δ7.22-7.26 (m, 1 H), 6.74-6.83 (m, 2 H), 4.59 (s, 2 H), 3.41-3.42 (m, 2 H), 1.63 (q, 2 H, J = 7.6 Hz), 1.23 (s, 6 H), 1.15 (t, 3 H, J = 7.6 Hz), 0.85 (t, 3 H, J = 7.6 Hz). LC-MS (ESI) [M+H] ⁺calad for: C₁₅H₂₁F₂NO, 270.2; found, 270.4.

### Compound 30:

### Preparation of N,2,2-trimethyl-N-(3-nitro-4-(piperidin-1-yl)benzyl)butanamide

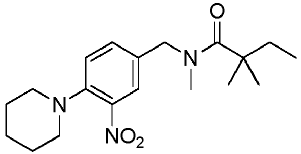

The titled compound 30 was prepared in 66% yield from 3-nitro-4-(piperidin-1-yl)benzaldehyde (234mg), methanamine hydrochloride(101mg) and 2,2-dimethylbutanoyl chloride(193mg) according to the procedure outlined for compound 13. ¹H NMR(CDCl₃, 400 M Hz): δ7.61 (d, 1 H, J = 2.4 Hz), 7.35 (dd, 1H, J = 8.4, 2.4 Hz), 7.16 (d, 1 H, J = 8.4 Hz), 4.54 (s, 2 H), 3.03-3.06 (m, 7 H), 1.73-1.77 (m, 4 H), 1.68 (q, 2 H, J = 7.6 Hz), 1.57-1.62 (m, 2 H), 1.28 (s, 6 H), 0.89 (t, 3 H, J = 7.6 Hz). LC-MS (ESI) [M+H] ⁺calad for: C₁₉H₂₉N₃O₃, 348.2; found 348.4.

### Compound 31: Preparation of N-(2,3-dimethylbenzyl)-N,2,2-trimethylbutanamide

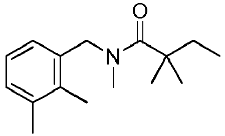

The titled compound 31 was prepared in 76% yield from 2,3-dimethylbenzaldehyde (134mg), methanamine hydrochloride(101mg) and 2,2-dimethylbutanoyl chloride(134 mg) according to the procedure outlined for compound 13. ¹H NMR(CDCl₃, 400 M Hz):δ7.05-7.09 (m, 2 H), 6.93-6.95 (m, 1 H), 4.65 (s, 2 H), 2.97 (s,3 H), 2.29 (s, 3 H), 2.16 (s, 3 H), 1.69 (q, 2 H, J = 7.6 Hz), 1.28 (s, 6 H), 0.92 (t, 3 H, J = 7.6 Hz). LC-MS (ESI) [M+H] ⁺calad for: C₁₆H₂₅NO, 248.2; found 248.4.

### Compound 32: Preparation of N-(3,5-dimethylbenzyl)-N,2,2-trimethylbutanamide

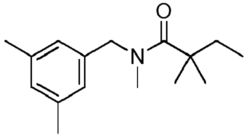

The titled compound 32 was prepared in 76% yield from 3,5-dimethylbenzaldehyde (134mg), methanamine hydrochloride(101mg) and2,2-dimethylbutanoyl chloride(134mg) according to the procedure outlined for compound 13. ¹H NMR (CDCl₃, 400 M Hz):δ6.89 (s, 1 H), 6.82 (s, 2 H), 4.57 (s, 2 H), 2.96 (s, 3 H), 2.29 (s, 6 H), 1.69 (q, 2 H, J = 7.6 Hz), 1.29 (s, 6 H), 0.91 (t, 3 H, J = 7.6 Hz). LC-MS (ESI) [M+H] ⁺calad for: C₁₆H₂₅NO, 248.2; found 248.4.

### Compound 33: Preparation of N-(2-fluoro-3-(trifluoromethoxy)benzyl)-N,2,2-trimethylbutanamide

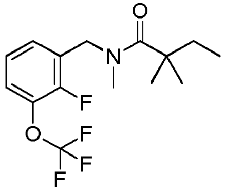

The titled compound 33 was prepared in 76% yield from 2-fluoro-3-(trifluoromethoxy)benzaldehyde (192mg), methanamine hydrochloride(101mg) and2,2-dimethylbutanoyl chloride(134mg) according to the procedure outlined for compound 13. ¹HNMR (CDCl₃, 400 M Hz):δ7.51 (t, 2 H, J = 7.2 Hz), 7.20 (t, 1 H, J = 7.6 Hz), 4.68 (s, 2 H), 3.11 (s, 3 H), 1.68 (q, 2 H, J = 7.6 Hz), 1.27 (s, 6 H), 0.86 (t, 3 H, J = 7.6 Hz). LC-MS (ESI) [M+H] ⁺calad forC₁₅H₁₉F₄NO₂, 322.1; found 322.3.

### Compound 34: Preparation of N,2,2-trimethyl-N-(pyridin-4-ylmethyl)butanamide

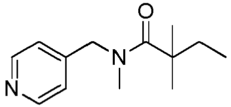

The titled compound 34 was prepared in 86% yield from isonicotinaldehyde(214mg), methanamine hydrochloride(202mg) and2,2-dimethylbutanoyl chloride(289mg) according to the procedure outlined for compound 13. ¹HNMR(CDCl₃, 400 M Hz):δ 8.55 (brs, 2 H), 7.13 (d, 2 H, J = 5.6 Hz), 4.59 (s, 2 H), 3.05 (s, 3 H), 1.69 (q, 2 H, J = 7.6 Hz), 1.28 (s, 6 H), 0.89 (t, 3 H, J = 7.6 Hz). LC-MS (ESI) [M+H] ⁺calad forC₁₃H₂₀N₂O, 221.2; found, 221.4.

### Compound 35: Preparation of N,2,2-trimethyl-N-(pyridin-3-ylmethyl)butanamide

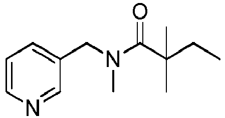

The titled compound 35 was prepared in 86% yield from nicotinaldehyde(214mg), methanamine hydrochloride(202mg) and 2,2-dimethylbutanoyl chloride(289mg) according to the procedure outlined for compound 13. ¹HNMR (CDCl₃, 400 M Hz):δ8.48-8.51 (m, 2 H), 7.58-7.61 (m, 1 H), 7.24-7.27 (m, 1 H), 4.59 (s, 2 H), 3.03 (s, 3 H), 1.66 (q, 2 H, J = 7.6 Hz), 1.27 (s, 6 H), 0.86 (t, 3 H, J = 7.6 Hz). LC-MS (ESI) [M+H] ⁺calad forC₁₃H₂₀N₂O, 221.2;found, 221.4.

### Compound 36: Preparation of N-((3-fluoropyridin-4-yl)methyl)-N,2,2-trimethylbutanamide

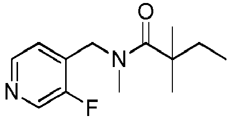

The titled compound 36 was prepared in 78% yield from 3-fluoroisonicotinaldehyde (125mg), methanamine hydrochloride (101mg) and2,2-dimethylbutanoyl chloride(134mg) according to the procedure outlined for compound 13. ¹HNMR(CDCl₃, 400 M Hz):δ8.41 (d, 1 H, J = 1.6 Hz), 8.35 (d, 1 H, J = 4.8 Hz), 7.18 (dd, 1 H, J = 6.0, 5.2 Hz), 4.65 (s, 2 H), 3.12 (s, 3 H), 1.68 (q, 2 H, J = 7.6 Hz), 1.27 (s, 6 H), 0.87 (t, 3 H, J = 7.6 Hz). LC-MS (ESI) [M+H] ⁺calad forC₁₃H₁₉FN₂O, 239.1; found 239.3.

### Compound 37: Preparation of N-((2-methoxypyridin-3-yl)methyl)-N,2,2-trimethylbutanamide

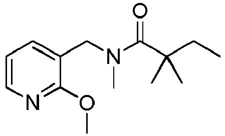

The titled compound 37 was prepared in 72% yield from 2-methoxynicotinaldehyde (137mg), methanamine hydrochloride(101mg) and 2,2-dimethylbutanoyl chloride(160mg) according to the procedure outlined for compound 13. ¹HNMR(CDCl₃, 400 M Hz):δ8.06 (dd, 1 H, J = 5.2, 2.0 Hz), 7.40 (d, 1 H, J = 7.2 Hz), 6.86 (dd, 1 H, J = 7.2, 5.2 Hz), 4.56 (s, 2 H), 3.97 (S, 3 H), 3.05 (s, 3 H), 1.67 (q, 2 H, J = 7.6 Hz), 1.25 (s, 6 H), 0.87 (t, 3 H, J = 7.6 Hz). LC-MS (ESI) [M+H] ⁺calad forC₁₄H₂₂N₂O₂, 251.2; found 251.4.

### Compound 38: Preparation of N-((6-methoxypyridin-3-yl)methyl)-N,2,2-trimethylbutanamide

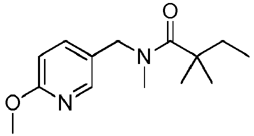

The titled compound 38 was prepared in 72% yield from 6-methoxynicotinaldehyde (137mg), methanamine hydrochloride (101mg) and2,2-dimethylbutanoyl chloride(160mg) according to the procedure outlined for compound 13. ¹HNMR(CDCl₃, 400 M Hz):δ 8.01-8.02 (m, 1 H), 7.53 (d, 1 H, J = 8.4 ), 6.71 (d, 1 H, J = 8.4), 4.51 (s, 2 H), 3.92 (s, 3 H), 3.00 (s, 3 H), 1.66 (q, 2 H, J = 7.6 Hz), 1.26 (s, 6 H), 0.85 (t, 3 H, J = 7.6 Hz). LC-MS (ESI) [M+H] ⁺calad forC₁₄H₂₂N₂O₂, 251.2; found 251.4.

### Compound 39: Preparation of N-(cyclohexylmethyl)-N,2,2-trimethylbutanamide

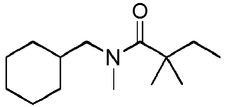

The titled compound 39 was prepared in 62% yield from cyclohexanecarbaldehyde (112mg), methanamine hydrochloride (101mg) and2,2-dimethylbutanoyl chloride(160mg) according to the procedure outlined for compound 13. ¹HNMR(CDCl₃, 400 MHz):δδ 3.16 (d, 2 H, J = 6.8 Hz), 3.07 (s,3 H),1.92 (m, 1 H), 1.63-1.73 (m, 8 H), 1.24 (s, 6 H), 1.08-1.19 (m,4 H), 0.87 (t, 3 H, J = 7.6 Hz). LC-MS (ESI) [M+H] ⁺calad forC₁₄H₂₇NO, 226.2; found 226.4.

### Compound 40: Preparation of N,2,2-trimethyl-N-(thiophen-2-ylmethyl)butanamide

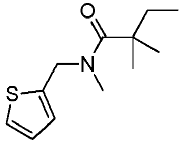

The titled compound 40 was prepared in 62% yield from thiophene-2-carbaldehyde (224mg), methanamine hydrochloride (202mg) and2,2-dimethylbutanoyl chloride(119mg) according to the procedure outlined for compound 13. ¹HNMR (CDCl₃, 400 MHz):δ7.21 (dd, 1 H, J = 4.8, 1.6 Hz), 6.92-6.95 (m, 2 H), 4.71 (s, 2 H), 3.05 (s, 3 H), 1.66 (q, 2H, J = 7.6 Hz), 1.27 (s, 6 H), 0.86 (t, 3 H, J = 7.6 Hz). LC-MS (ESI) [M+H] ⁺calad forC₁₂H₁₉NOS, 226.1; found 226.4.

### Compound 41: Preparation of N,2,2-trimethyl-N-((3-methylthiophen-2-yl)methyl)butanamide

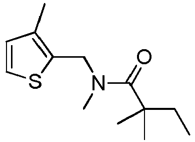

The titled compound 41 was prepared in 42% yield from 3-methylthiophene-2-carbaldehyde (200mg), methanamine hydrochloride (161mg) and2,2-dimethylbutanoylchloride(218mg) according to the procedure outlined for compound 13. ¹HNMR(CDCl₃,400MHz):δ7.11 (d, 1 H, J = 5.2 Hz), 6.78 (d, 1 H, J = 5.2 Hz), 4.68 (s, 2 H), 3.03 (s, 3 H), 2.23 (s, 3 H), 1.67 (q, 2 H, J = 7.6 Hz), 1.27 (s, 6 H), 0.89 (t, 3 H, J = 7.6 Hz). LC-MS (ESI) [M+H] ⁺calad forC₁₃H₂₁NOS, 234.1; found 234.4.

### Compound 42: Preparation of N,2,2-trimethyl-N-((5-methylthiophen-2-yl)methyl)butanamide

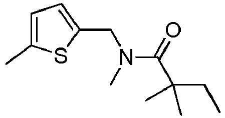

The titled compound42 was prepared in 42% yield from 5-methylthiophene-2-carbaldehyde (200mg), methanamine hydrochloride (161mg) and2,2-dimethylbutanoylchloride (218mg) according to the procedure outlined for compound 13. ¹HNMR (CDCl₃,400MHz):δ6.72 (d, 1 H, J = 3.2 Hz), 6.56-6.57 (m, 1 H), 4.62 (s, 2 H), 3.03 (s, 3 H), 2.43 (d, 3 H, J = 1.2 Hz), 1.661 (q, 2 H, J = 7.6 Hz), 1.269 (s, 6 H), 0.877 (t, 3 H, J = 7.6 Hz). LC-MS (ESI) [M+H] ⁺calad for C₁₃H₂₁NOS, 234.1; found 234.4.

### Compound 43: Preparation of N-(furan-2-ylmethyl)-N,2,2-trimethylbutanamide

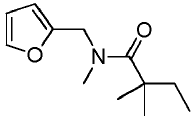

The titled compound 43 was prepared in 22% yield from furan-2-carbaldehyde (500mg), methanamine hydrochloride (527mg) and2,2-dimethylbutanoylchloride (714mg) according to the procedure outlined for compound 13. ¹HNMR(CDCl₃,400MHz):7.34-7.35 (m, 1 H), 6.31-6.33 (m, 1 H), 6.21-6.22 (m, 1 H), 4.57 (s, 2H), 3.06 (s, 3 H), 1.63 (q, 2 H, J=7.6 Hz ), 1.27(s, 6 H), 0.85 (t, 3 H, J=7.6 Hz) LC-MS (ESI) [M+H] ⁺calad forC₁₂H₁₉NO₂, 210.1; found 210.3.

### Compound 44: Preparation of N,2,2-trimethyl-N-((2-methylthiazol-5-yl)methyl)butanamide

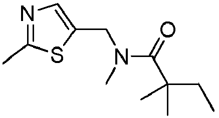

The titled compound 44 was prepared in 23% yield from 2-methylthiazole-5-carbaldehyde (60mg), methanamine hydrochloride(48mg) and2,2-dimethylbutanoylchloride(74mg) according to the procedure outlined for compound 13.¹HNMR(CDCl₃, 400MHz):δ7.50 (s, 1 H), 4.59 (s, 2 H), 3.07 (s, 3 H), 2.67 (s, 3 H), 1.64 (q, 2 H, J = 7.6 Hz), 1.25 (s, 6 H), 0.84 (t, 3 H, J = 7.6 Hz). LC-MS (ESI) [M+H] ⁺calad forC₁₂H₂₀N₂OS, 241.1; found 241.4.

### Compound 45: Preparation of N,2,2-trimethyl-N-((1-methyl-1H-pyrazol-3-yl)methyl)butanamide

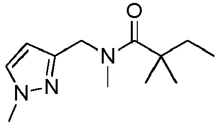

The titled compound 45 was prepared in 23% yield from N,1-dimethyl-1H-pyrazol-3-amine (30mg), and 2,2-dimethylbutanoylchloride(48mg) according to the procedure outlined for compound 13.¹HNMR (CDCl₃,400MHz):δ7.26 (d, 1 H, J= 4.0 Hz), 6.12 (d, 1 H, J = 4.0 Hz ), 4.57 (s, 2 H), 3.85 (s, 3 H), 3.01 (s, 3 H), 1.65 (q, 2 H, J = 7.6 Hz), 1.26 (s, 6 H), 0.85 (t, 3 H, J = 7.6 Hz). LC-MS (ESI) [M+H] ⁺calad for C₁₂H₂₁N₃O, 224.2; found 224.4.

### Compound 46: Preparation of N,2,2-trimethyl-N-(naphthalen-2-ylmethyl)butanamide

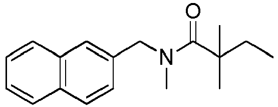

The titled compound 46 was prepared in 74% yield from 2-naphthaldehyde (312mg) methanamine hydrochloride(202mg) and 2,2-dimethylbutanoylchloride(275mg) according to the procedure outlined for compound 13

1.¹HNMR(CDCl₃,400MHz):δ7.80(m, 3H), 7.63(m, 1H), 7.46(m, 3H), 4.78(s, 2H), 3.00(s, 3H), 1.70(q, 2 H, J = 7.6 Hz)), 1.30(s, 6H), 0.93(t, 3H, J = 7.6 Hz).LC-MS (ESI) [M+H] ⁺calad for C₁₈H₂₃NO, 270.2; found 270.4.

### Compound 47: Preparation of N,2,2-trimethyl-N-(quinolin-3-ylmethyl)butanamide

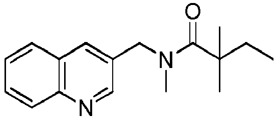

The titled compound 47 was prepared in60% yield from quinoline-3-carbaldehyde (157mg), methanamine hydrochloride101mg) and2,2-dimethylbutanoylchloride(175mg) according to the procedure outlined for compound 13.¹HNMR(CDCl₃,400MHz):δ8.85 (d, 1 H, J = 4.4 Hz), 8.13 (d, 1 H, J = 8.4 Hz ), 7.97 (d, 1 H, J = 8.4 Hz), 7.68-7.73 (m, 1 H), 7.53-7.57 (m, 1 H), 7.155 (d, 1 H, J = 4.4 Hz), 5.10 (s, 2 H), 3.07 (s, 3 H), 1.70 (q, 2 H, J = 7.6 Hz), 1.29 (s, 6 H), 0.91 (t, 3 H, J = 7.6 Hz). LC-MS (ESI) [M+H] ⁺calad for C₁₇H₂₂N₂O, 271.2; found 271.4.

### Compound 48: Preparation of N,2,2-trimethyl-N-(2,4,6-trifluorobenzyl)butanamide

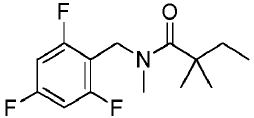

The titled compound 48 was prepared in 63% yield from 2,4,6-trifluorobenzaldehydhyde (320mg), methanamine hydrochloride (202mg)and2,2-dimethylbutanoylchloride (275mg) according to the procedure outlined for compound 13.¹HNMR(CDCl₃,400MHz):δ6.61-6.69 (m, 2 H), 4.64 (s, 2 H), 3.06 (s, 3 H), 1.64 (q, 2 H, J = 7.6 Hz), 1.24 (s, 6 H), 0.82 (t, 3 H, J = 7.6 Hz). LC-MS (ESI) [M+H] ⁺calad forC₁₄H₁₈F₃NO, 274.1; found 274.3.

### Compound 49: Preparation of N,2,2-trimethyl-N-(2,3,4-trifluorobenzyl)butanamide

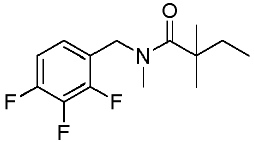

The titled compound 49 was prepared in 66% yield from 2,3,4-trifluorobenzaldehyde (160mg), methanamine hydrochloride(101mg) and 2,2-dimethylbutanoylchloride (175mg) according to the procedure outlined for compound 13.¹HNMR(CDCl₃,400MHz):δ7.03-7.05 (m, 1 H), 6.88-6.95 (m, 1 H), 4.61 (s, 2 H), 3.09 (s, 3 H), 1.66 (q, 2 H, J = 7.6 Hz), 1.26 (s, 6 H), 0.84 (t, 3 H, J = 7.6 Hz). LC-MS (ESI) [M+H] ⁺calad forC₁₄H₁₈F₃NO, 274.1; found 274.3.

### Compound 50: Preparation of N-(2,6-difluoro-3-methylbenzyl)-N,2,2-trimethylbutanamide

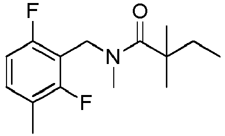

The titled compound 50 was prepared in 66% yield from 2,6-difluoro-3-methylbenzaldehyde (156mg), methanamine hydrochloride(101mg) and 2,2-dimethylbutanoylchloride(140mg) according to the procedure outlined for compound 13.¹HNMR(CDCl₃, 400MHz):δ7.06 (q,1 H, J = 8.4 Hz), 6.77 (t, 1 H, J = 8.8 Hz), 4.71 (s, 2 H), 3.02 (s, 3 H), 2.22 (s, 3 H), 1.66 (q, 2 H, J = 7.6 Hz), 1.26 (s, 6 H), 0.85 (t, 3 H, J = 7.6 Hz). LC-MS (ESI) [M+H] calad forC₁₅H₂₁F₂NO, 270.2; found 270.4.

### Compound 51: Preparation of N,2,2-trimethyl-N-(2,3,5,6-tetrafluorobenzyl)butanamide

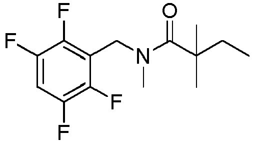

The titled compound 51 was prepared in 66% yield from 2,3,5,6-tetrafluorobenzaldehyde (178mg), methanamine hydrochloride (101mg) and 2,2-dimethylbutanoylchloride (140mg) according to the procedure outlined for compound 13.¹HNMR(CDCl₃, 400MHz):δ6.95-7.03 (m, 1 H) 4.70 (s, 2 H), 3.16 (s, 3 H), 1.65 (q, 2 H, J = 7.6 Hz), 1.24 (s, 6 H), 0.83 (t, 3 H, J = 7.6 Hz). LC-MS (ESI) [M+H] ⁺calad forC₁₄H₁₇F₄NO, 292.1; found 292.4.

### Compound 52 (for exemplary purposes only): Preparation of 2,2-dimethyl-N-(1-phenylethyl)butanamide

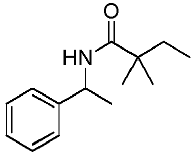

1-phenylethanamine (1 g, 8.26 mmoL) and Triethylamine (0.918 g, 9.09 mmoL) were dissolved in 20 mL of dry CH₂Cl₂. 2,2-dimethylbutanoyl chloride (1.223 g,9.09m moL) in 2 mL of CH₂Cl₂ was added slowly to the solution at 0°C under nitrogen. The mixture was stirred at room temperature for 2 h, diluted with CH₂Cl₂ and water. The organic layers were washed with saturated NaHCO₃, brine, dried with Na₂SO₄ and concentrated The residue was purified by chromatography to give compound 40(1.35g, 74.6%) as an white solid. ¹HNMR(CDCl₃, 400MHz):δ 7.26-7.34 (m, 5 H), 5.77 (brs, 1 H), 5.10-5.17 (m, 1H), 1.545 (q, 2 H, J = 8Hz), 1.485 (d,3H, J=4Hz), 1.15 (s, 6H), 0.82 (t, 3H, J= 8Hz). LC-MS (ESI) [M+H] ⁺calad for C₁₄H₂₁NO, 220.2; found 220.4.

### Compound 53 (for exemplary purposes only): Preparation of N,2,2-trimethyl-N-(1-phenylethyl)butanamide

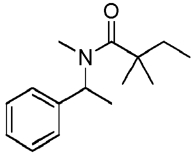

To a solution of compound 52 (50mg) in dry THF (1ml) was added sodium hydride (13.7mg) under nitrogen at 0°C. The mixture was stirred at 0°C for 30 minutes, then iodomethane (38.9mg) was added. The mixture was stirred at room temperature for 2 h and quenched with cold water and extracted with CH₂Cl₂. The combined organic layer were washed with H₂O, dried with Na₂SO₄ and concentrated The residue was purified by chromatography to give compound 53 ( 8mg, 15%) as a colorless oil. ¹HNMR(CDCl₃, 400MHz): δ7.32-7.35 (m, 2 H), 7.22-7.27 (m, 3 H), 5.62-6.30 (m, 1 H), 2.70 (s, 3 H), 1.68 (q, 2 H, J = 7.6 Hz), 1.51 (d, 3 H, J = 6.0 Hz), 1.30 (s, 3 H), 1.29 (s, 3 H), 0.91 (t, 3 H, J = 7.6 Hz). LC-MS (ESI) [M+H] ⁺calad for C₁₅H₂₃NO, 234.2; found 234.4.

### Compound 54 (for exemplary purposes only): Preparation of 2,2-dimethyl-N-(1-phenylcyclopropyl)butanamide

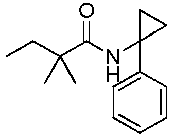

The titled compound 54 was prepared in 96% yield from1-phenylcyclopropanamine (106mg) and 2,2-dimethylbutanoyl chloride(160mg) according to the procedure outlined for compound 52. ¹HNMR(CDCl₃, 400MHz):δ7.22-7.29 (m, 4 H), 7.15-7.19 (m, 1 H), 6.25 (brs, 1 H), 1.54 (q, 2 H, J = 7.6 Hz), 1.24-1.29 (m, 2 H), 1.18-1.22 (m, 2 H), 1.16 (s, 6 H), 0.81 (t, 3 H, J = 7.6 Hz). LC-MS (ESI) [M+H] ⁺calad forC₁₅H₂₁NO, 232.1; found 232.4.

### Compound 55 (for exemplary purposes only): Preparation of N,2,2-trimethyl-N-(1-phenylcyclopropyl)butanamide

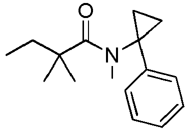

The titled compound 55 was prepared in 30% yield from compound 42(90mg), sodium hydride (32mg) and iodomethane (85.2mg) according to the procedure outlined for compound 53.¹HNMR(CDCl₃, 400MHz):7.28-7.30 (m, 2 H), 7.15-7.19 (m, 3 H), 3.12 (s, 3 H), 1.67 (q, 2 H, J = 7.6 Hz), 1.30-1.32(m, 2 H), 1.26(s, 6 H), 1.24-1.25(m, 1H) ,0.81 (t, 3 H, J = 7.6 Hz)LC-MS (ESI) [M+H] ⁺calad forC₁₆H₂₃NO, 246.1;found , 246.4.

### Compound 56: Preparation of N-(2-bromo-5-fluorobenzyl)-N,2,2-trimethylbutanamide

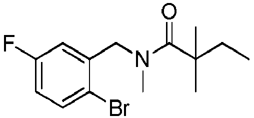

The titled compound 56 was prepared in 56% yield from 2-bromo-5-fluorobenzaldehyde (500mg), methanamine hydrochloride(249mg) and 2,2-dimethylbutanoyl chloride(317mg) according to the procedure outlined for compound 13.¹HNMR(CDCl₃, 400MHz):7.48-7.52 (m, 1 H),7.04-7.10(m, 2 H), 4.80 (s, 2 H), 3.15 (s, 3 H), 1.71 (q, 2 H, J = 7.6 Hz), 1.30(s, 6 H), 0.91 (t, 3 H, J = 7.6 Hz) LC-MS (ESI) [M+H] ⁺calad for C14H19BrFNO,316.1;found, 316.1, 318.2.

### Compound 57: Preparation of N-(2-cyano-5-fluorobenzyl)-N,2,2-trimethylbutanamide

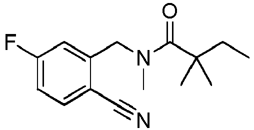

A mixture of compound 56(30mg), sodium iodide (1.4mg) and copper(I) cyanide (22mg) in dry DMF(1mL) was stirred at 180°C for 6h.The mixture was diluted with saturated aqueous NaHCO₃ solution (2mL) and the aqueous layer was extracted with dichloromethane(5mL x 3). The combined organic layers were washed with brine, dried with Na₂SO₄, filtered and concentrated. The residue was purified by Pre- TLC to give compound 57(18mg, 72%) . 1H-NMR (CDCl₃, 400MHz): δ 7.64-7.67 (m, 1 H), 6.83-6.88 (m, 2 H), 4.65 (s, 2 H), 3.09 (s, 3 H), 1.71 (q, 2 H, J = 7.6 Hz), 1.29 (s, 6 H), 0.93 (t, 3 H, J = 7.6 Hz)MS(ES) [M+H] ⁺calad forC15H19FN2O, 263.1; found ,263.3.

### Compound 58 (for exemplary purposes only): Preparation of N-benzyl-2,2-dimethylbutanamide

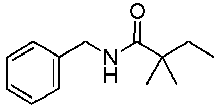

The titled compound 58 was prepared in 84% yield from phenylmethanamine(107mg) and 3,3-dimethylbutanoyl chloride(140mg) according to the procedure outlined for compound 52. ¹H NMR (CDCl₃): δ 7.25-7.36 (m, 5H), 5.88 (br, 1H), 4.45 (d, 2H, *J* = 8.0 Hz), 1.58 (q, 2H, *J* = 5.6 Hz), 1.19 (s, 6H), 0.81 (t, 3H, *J*=5.6 Hz).

### Compound 59 (for exemplary purposes only): Preparation of N-(2-fluorobenzyl)-N,3,3-trimethylbutanamide

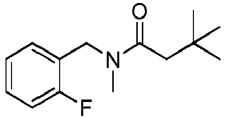

The titled compound 59 was prepared in 34% yield from 2-fluorobenzaldehyde (124mg), methanamine hydrochloride (101mg) and 3,3-dimethylbutanoyl chloride(140mg) according to the procedure outlined for compound 13. ¹HNMR(CDCl₃, 400MHz): δ 7.22-7.36 (m, 2 H), 7.01-7.15 (m, 2 H), 4.65 (s, 2 H), 2.97 (s, 3 H), 2.31(s, 2 H), 1.07 (s, 9 H).LC-MS (ESI) [M+H] ⁺calad for C₁₄H₂₀FNO, 238.2; found 238.4.

### Compound 60 (for exemplary purposes only): Preparation of N-(2-fluorobenzyl)-N-methylcyclohexanecarboxamide

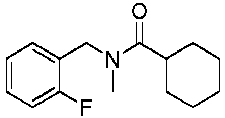

The titled compound 60 was prepared in 66% yield from 2-fluorobenzaldehyde (124mg), methanamine hydrochloride (101mg) and cyclohexane carbonyl chloride(153mg) according to the procedure outlined for compound 13.¹HNMR(CDCl₃, 400MHz):7.23-7.28 (m, 2 H), 7.01-7.11 (m, 2 H), 4.63 (s, 2 H), 2.97 (s, 3 H), 2.51-2.56 (m, 1 H), 1.52-1.79(m, 7 H), 1.24-1.31 (m, 3 H). LC-MS (ESI) [M+H] ⁺calad for C₁₅H₂₀FNO, 250.2; found, 250.4.

### Compound 61 (for exemplary purposes only): Preparation of N-(2-fluorobenzyl)-N-methylbenzamide

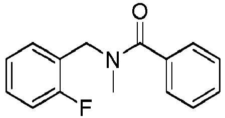

The titled compound 61 was prepared in 56% yield from 2-fluorobenzaldehyde (124mg), methanamine hydrochloride (101mg) and benzoyl chloride(147mg) according to the procedure outlined for compound 1.¹HNMR(DMSO, 400MHz): δ 7.20-7.42 (m, 9 H), 4.70 (s, 1 H), 4.50 (s, 1 H), 2.84 (s, 3 H). LC-MS (ESI) [M+H] ⁺calad for C₁₅H₁₄FNO, 244.1; found 244.3.

### Compound 62 (for exemplary purposes only): Preparation of N-(2-fluorobenzyl)-N-methylcyclopropanecarboxamide

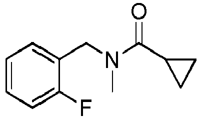

The titled compound 50 was prepared in 54% yield from 2-fluorobenzaldehyde (124mg), methanamine hydrochloride (101mg) and cyclopropanecarbonyl chloride(147mg) according to the procedure outlined for compound 13.¹HNMR(CDCl₃, 400MHz): δ 7.25-7.27 (m, 2 H), 7.03-7.14 (m,2 H), 4.76 (s, 2 H), 3.11 (s, 3 H), 1.69-1.83 (m, 1H), 1.01-1.05 (m, 2 H), 0.69-0.86 (m, 2 H). LC-MS (ESI) [M+H] ⁺calad forC₁₂H₁₄FNO, 208.1; found,208.3.

### Compound 63 (for exemplary purposes only): Preparation of N-(3-fluorobenzyl)-N-(3-methoxypropyl)-2,2-dimethylbutanamide

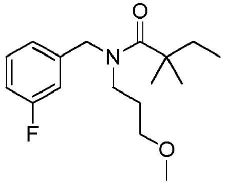

A mixture of (2-fluorophenyl)methanamine (125mg), potassium carbonate(414mg) and 1-chloro-3-methoxypropane(108mg) in DMF(5ml) was stirred at 100°C for 16h. The mixture was diluted with CH₂Cl₂, washed with H₂O, dried with Na₂SO₄. After removal of the solvent to give the crude N-(3-fluorobenzyl)-3-methoxypropan-1-amine (200mg). The resulting compound was dissolve in dry THF (10ml), and DIPEA (193mg) was added. The mixture was cooled to 0°C, 2,2-dimethylbutanoylchloride(201mg) was added and stirred for 4 h at room temperature. The mixture was quenched with water and extracted with EtOAc. The combined organic layer were washed with brine and dried over Na₂SO₄. After removal of the solvent, the residue was purified by silica gel chromatography to afford the compound 63(177mg, 60%). ¹HNMR(CDCl₃, 400MHz): δ7.27-7.31 (m, 1 H), 6.88-6.98 (m, 3 H), 4.66 (s, 2 H), 3.41 (m, 2 H), 3.36 (t, 2 H, J = 6.0 Hz), 3.29 (s, 3 H), 1.84-1.86 (m, 2 H), 1.67 (q, 2 H, J = 7.6 Hz), 1.28 (s, 6 H), 0.90 (t, 3 H, J = 7.6 Hz). LC-MS (ESI) [M+H] ⁺calad forC₁₇H₂₆FNO₂, 296.2; found 296.4.

### Compound 64 (for exemplary purposes only): Preparation of N-(cyclopropylmethyl)-N-(3-fluorobenzyl)-2,2-dimethylbutanamide

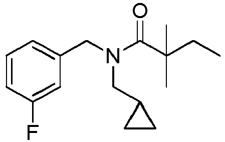

The titled compound 64 was prepared in 54% yield from (2-fluorophenyl)methanamine (125mg), (bromomethyl)cyclopropane (135mg) and 2,2-dimethylbutanoylchloride(201mg) according to the procedure outlined for compound 63. ¹HNMR(CDCl₃, 400MHz):δ 7.24-7.30 (m, 1 H), 6.88-6.98 (m, 3 H), 4.81 (s, 2 H), 3.24 (d, 2 H, J = 6.4 Hz), 1.70 (q, 2 H, J = 7.6 Hz), 1.29 (s, 6 H), 0.94 (t, 3 H, J = 7.6 Hz), 0.87-0.90 (m, 1 H), 0.51 (m, 2 H), 0.13 (m, 2 H). LC-MS (ESI) [M+H] ⁺calad forC₁₇H₂₄FNO, 278.2; found 278.3.

### Compound 65 (for exemplary purposes only): Preparation of N,1-dimethyl-N-(2,3,5-trifluorobenzyl)cyclohexanecarboxamide

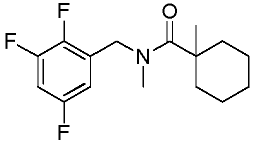

N-methyl-1-(2,3,5-trifluorophenyl)methanamine (37 mg, 0.211 mmol), which was prepared from 2,3,5-trifluorobenzaldehyde and methanamine hydrochloride according to the procedure outlined for compound 13, and 1-methylcyclohexanecarboxylic acid (30 mg, 0.211 mmoL) were dissolved in dry DMF (1ml), 2-(7-Aza-1H-benzotriazole-

1-yl)-1,1,3,3-tetramethyluronium hexafluorophosphate(119.7 mg, 0.315 mmoL) and N,N-Diisopropylethylamine (54.2 mg, 0.42 mmoL) were added to the solution. The mixture was stirred at room temperature for 16 h. The solvent was removed under reduced pressure and the residue was purified by column chromatography to give 12 mg of desired compound 65 as colorless oil ( yield =19.5% )¹H NMR:(CDCl₃, 400 M Hz)δ (ppm) 6.80-6.82 (m, 1 H), 6.72-6.76 (m, 1H), 4.65 (s, 2 H), 3.10 (s, 3 H), 2.06-2.11 (m, 2 H), 1.36-1.54 (m, 8 H), 1.27 (s, 3 H).LC-MS (ESI) [M+H]⁺ calad for C₁₆H₂₀F₃NO, 300.1; found 300.3.

### Compound 66 (for exemplary purposes only): Preparation of 3-hydroxy-N,2,2-trimethyl-N-(3,4,5-trifluorobenzyl)propanamide

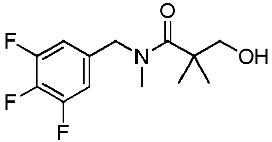

The titled compound 66 was prepared in 34% yield from N-methyl-1-(3,4,5-trifluorophenyl)methanamine (447mg), which was prepared from 2,3,5-trifluorobenzaldehyde and methanamine hydrochloride according to the procedure outlined for compound 13, and 3-hydroxy-2,2-dimethylpropanoic

acid(300mg) and according to the procedure outlined for compound 65.¹H NMR: (CDCl₃, 400 M Hz): δ 6.82 (m, 2 H), 4.52 (s, 2 H), 3.57 (s, 2 H), 3.06 (s, 3 H), 1.32 (s, 6 H). LC-MS (ESI) [M+H]⁺ calad for C₁₃H₁₆F₃NO₂,276.1; found 276.3.

### Compound 67 (for exemplary purposes only): Preparation of 2-methoxy-N,2-dimethyl-N-(2,3,5-trifluorobenzyl)propanamide

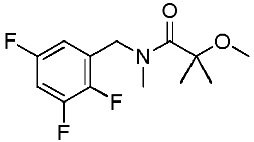

The titled compound 55 was prepared in 14% yield from 2-methoxy-2-methylpropanoicacid (300mg) and N-methyl-1-(2,3,5-trifluorophenyl)methanamine (447mg) according to the procedure outlined for compound 65.¹H NMR: (CDCl₃, 400 M Hz): δ7.01-7.04 (m, 2 H), 4.64 (s, 2 H), 3.42 (s, 3 H), 3.37 (s, 3 H), 1.62 (s, 6 H).LC-MS (ESI) [M+H]⁺ calad for C₁₃H₁₆F₃NO₂,276.1; found 276.3.

### Compound 68 (for exemplary purposes only): Preparation of N,2,2-trimethyl-3-(methylamino)-N-(3,4,5-trifluorobenzyl) propanamide

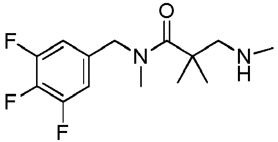

A mixture of 3-chloro-N,2,2-trimethyl-N-(3,4,5-trifluorobenzyl)propanamide(60mg) methanamine hydrochloride(27mg), potassium carbonate(138mg) and potassium iodide (33.2mg) in methyl cyanide(5mL) was refluxed for overnight. The mixture was diluted with water(2mL), The aqueous layer was extracted with dichloromethane(5mL x 3). The organic layers were combined and concentrated. The residue was purified by Pre-HPLC to give 2mg of compound 68 as TFA salt. ¹H NMR: (CDCl₃, 400 M Hz) δ 6.80-6.88 (m, 2 H), 4.51 (s, 2 H), 4.22 (brs, 1 H), 3.00-3.16 (m, 5 H), 2.82 (s, 3 H), 1.51 (s, 6 H) . LC-MS (ESI) [M+H]⁺ calad for C₁₄H₁₉F₃N₂O, 289.2; found 289.4.

### Compound 69 (for exemplary purposes only): Preparation ofN-methyl-N-(3,4,5-trifluorobenzyl)pivalamide

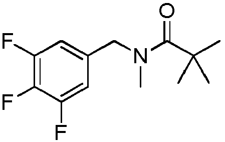

The titled compound 69 was prepared in 27% yield from pivalic acid(14.6mg) and N-methyl-1-(3,4,5-trifluorophenyl)methanamine (25mg) according to the procedure outlined for compound 65.¹H NMR: (CDCl₃, 400 M Hz): δ 6.79-6.86 (m, 2 H), 4.52 (s, 2 H), 3.05 (s, 3 H), 1.33 (s, 9 H). LC-MS (ESI) [M+H]⁺ calad forC₁₃H₁₆F₃NO, 260.1; found 260.3

### Compound 70 (for exemplary purposes only): Preparation of 2,2-dimethyl-N-(2,3,5-trifluorobenzyl)butanamide

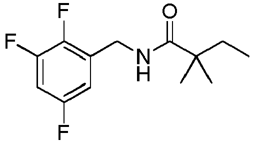

The titled compound 58 was prepared in 41.5% yield from (2,3,5-trifluorophenyl)methanamine (30mg) and 2,2-dimethylbutanoyl chloride(27.6mg) according to the procedure outlined for compound 52.¹H NMR: (CDCl₃, 400 M Hz): δ6.81-6.87 (m, 2 H), 6.02 (brs, 1 H), 4.50 (d, 1 H, J = 1.2 Hz), 4.48 (d, 1 H, J = 1.2 Hz), 1.56 (q, 2 H, J = 7.6 Hz), 1.18 (s, 6 H), 0.82 (t, 3 H, J = 7.6 Hz). LC-MS (ESI) [M+H]⁺ calad forC₁₃H₁₆F₃NO, 260.1; found 260.3.

### Compound 71 (for exemplary purposes only): Preparation of 3-methoxy-N,2,2-trimethyl-N-(3,4,5-trifluorobenzyl)propanamide

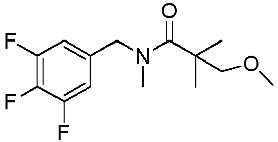

The titled compound 71 was prepared in 13% yield from 3-methoxy-2,2-dimethyl-propanoic acid (14mg) and N-methyl-1-(3,4,5-trifluorophenyl)methanamine (447mg) according to the procedure outlined for compound 65.¹H NMR: (CDCl₃, 400 M Hz): δ6.84-6.88 (m, 2 H), 4.54 (s, 2 H), 3.48 (s, 2 H), 3.36 (s, 3 H), 3.04 (s, 3 H), 1.33 (s, 6 H). LC-MS (ESI) [M+H]⁺ calad forC₁₄H₁₈F₃NO₂, 290.1; found , 290.4.

### Compound 72 (for exemplary purposes only): Preparation of 2-ethyl-N,2-dimethyl-N-(3,4,5-trifluorobenzyl)butanamide

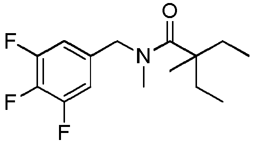

The titled compound 72 was prepared in 18% yield from N-methyl-1-(3,4,5-trifluorophenyl)methanamine (20mg) and 2-ethyl-2-methylbutanoic acid(15mg) according to the procedure outlined for compound 65. ¹H NMR: (CDCl₃, 400 M Hz): δ6.87-6.91 (m, 2 H), 4.50 (s, 2 H), 3.05 (s, 3 H), 1.77-1.84 (m, 2 H), 1.47-1.56 (m, 2 H), 1.23 (s, 3 H), 0.87 (t, 6 H, J = 7.6 Hz).LC-MS (ESI) [M+H]⁺ calad forC₁₅H₂₀F₃NO, 288.1;found 288.3.

### Compound 73 (for exemplary purposes only): Preparation of 2-ethyl-2-methyl-N-(2,3,5-trifluorobenzyl)butanamide

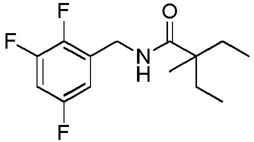

The titled compound 73 was prepared in 12% yield from (2,3,5-trifluorophenyl)methanamine (30mg) and 2-ethyl-2-methylbutanoic acid(24.4mg) according to the procedure outlined for compound 65. ¹H NMR: (CDCl₃, 400 M Hz): δ 6.82-6.88 (m, 2 H), 6.03 (brs, 1H), 4.49 (d, 2 H, J = 6.0 Hz), 1.61-1.69 (m, 2 H), 1.39-1.48 (m, 2 H), 1.12 (s, 3 H), 0.80 (t, 6 H, J = 7.6 Hz). LC-MS (ESI) [M+H]⁺ calad forC₁₄H₁₈F₃NO, 274.1; found 274.3.

### Compound 74 (for exemplary purposes only): Preparation of N-ethyl-N-(2,3,5-trifluorobenzyl)cyclohexanecarboxamide

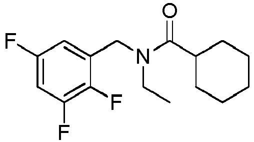

The titled compound 74 was prepared in 53% yield from 2,3,5-trifluorobenzaldehyde (320mg), ethylamine hydrochloride(244mg) and 2,2-dimethylbutanoylchloride (275mg) according to the procedure outlined for compound 13.¹HNMR(CDCl₃,400MHz):δ6.77-683 (m, 1 H), 6.59-6.75(m, 1H), 4.61(s, 2 H), 3.35(q,2 H, J= 7.2 Hz), 2.47-2.54 (m,1 H), 1.41-1.95 (m,10 H), 1.20 (t, 3 H, J = 7.2 Hz).LC-MS (ESI) [M+H]⁺ calad forC₁₆H₂₀F₃NO, 300.1; found, 300.3.

### Compound 75: Preparation of N,2,2-trimethyl-N-(2,3,5-trifluorobenzyl)butanamide

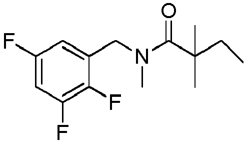

The titled compound 75 was prepared in 50% yield from 2,3,5-trifluorobenzaldehyde (320mg), methanamine hydrochloride( 202mg) and2,2-dimethylbutanoylchloride (275mg) according to the procedure outlined for compound 13.¹HNMR(CDCl₃,400MHz):δ 6.75-6.85 (m, 2 H), 4.64 (s, 2 H), 3.11 (s, 3 H), 1.68 (q, 2 H, J = 7.6 Hz), 1.28 (s, 6 H), 0.88 (t, 3 H, J = 7.6 Hz). LC-MS (ESI) [M+H]⁺ calad forC₁₄H₁₈F₃NO, 274.1; found 274.3.

### Compound 76 (for exemplary purposes only): Preparation ofN-methyl-N-(2,3,5-trifluorobenzyl)adamantane-1 -carboxamide

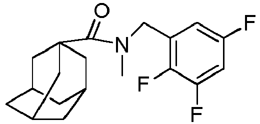

The titled compound 76 was prepared in 16% yield from 2,3,5-trifluorobenzaldehyde (50mg), methanamine hydrochloride(29mg) and adamantane-1-carbonyl chloride (40mg) according to the procedure outlined for compound 13..¹HNMR(CDCl₃,400MHz):δ7.30-7.51 (m, 1 H), 6.66-6.70 (m, 1 H), 4.58 (s, 2 H), 3.10 (s, 3 H), 1.93-1.96 (m,10 H), 1.64-1.67 (m,4 H). LC-MS (ESI) [M+H]⁺ calad forC₁₉H₂₂F₃NO, 338.2;found 338.4.

### Compound 77 (for exemplary purposes only): Preparation of N-(2-hydroxyethyl)-2,2-dimethyl-N-(2,3,5-trifluorobenzyl)butanamide

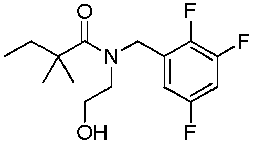

The titled compound 77 was prepared in 30% yield from 2-((2,3,5-trifluorobenzyl)amino)ethanol (50mg) and 2,2-dimethylbutanoylchloride (33mg) according to the procedure outlined for compound 52. ¹HNMR(CDCl₃,400MHz):δ 6.92-6.97 (m, 1 H), 6.80-6.87 (m, 1 H), 4.21 (t, 2 H, J = 5.2 Hz), 3.92 (s, 2 H), 2.89 (t, 2 H, J = 5.2 Hz), 1.57(q, 2 H, J = 7.2 Hz), 1.16 (s, 6 H), 0.83 (t, 3 H, J = 7.2 Hz). LC-MS (ESI) [M+H]⁺ calad for C₁₅H₂₀F₃NO₂, 304.1; found 304.3.

### Compound 78 (for exemplary purposes only): Preparation of N,2-dimethyl-N-(2,3,5-trifluorobenzyl)propane-2-sulfinamide

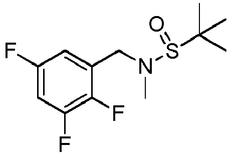

The titled compound 78 was prepared in 30% yield from N-methyl-1-(2,3,5-trifluorophenyl)methanamine (50mg) and 2,2-dimethylbutanoylchloride (57mg) according to the procedure outlined for compound 52. ¹HNMR(CDCl₃,400MHz):δ 6.83-6.94(m, 2H), 4.23-4.32(m, 2H), 2.64(s, 3H), 1.21(s, 9H). LC-MS (ESI) [M+H]⁺ calad for C₁₂H₁₆F₃NOS, 280.1; found, 280.2.

### Compound 79 (for exemplary purposes only): Preparation ofN-methyl-N-(2,3,5-trifluorobenzyl)cyclohexanesulfonamide

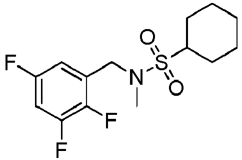

The titled compound 79 was prepared in 11% yield from N-methyl-1-(2,3,5-trifluorophenyl)methanamine (20mg) and cyclohexanesulfonyl chloride (30mg) according to the procedure outlined for compound 52. ¹HNMR(CDCl₃,400MHz):δ7.02-7.07 (m, 1 H), 6.84-6.91 (m, 1 H), 4.46 (s, 2 H), 2.99-3.05 (m, 1 H), 2.86 (s, 3 H), 1.95-2.15 (m, 2 H), 1.91-1.94 (m, 2 H), 1.57-1.74 (m, 5 H), 1.21-1.28 (m, 1 H). LC-MS (ESI) [M+H]⁺ calad forC₁₄H₁₈F₃NO₂S, 322.1; found 322.3.

### Compound 80 (for exemplary purposes only): Preparation of N,1-dimethyl-N-(2,3,5-trifluorobenzyl)cyclopropanecarboxamide

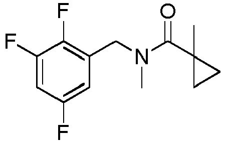

The titled compound 80 was prepared in 35% yield from 1-methylcyclopropanecarboxylicacid (20mg) and N-methyl-1-(2,3,5-trifluorophenyl)methanamine (35mg) according to the procedure outlined for compound 65. ¹H NMR: (CDCl₃, 400 M Hz): δ 6.80-6.83 (m, 2 H), 4.52 (s, 2 H), 3.05 (s, 3 H), 1.34 (s, 3 H), 0.98 (t, 2 H, J=4.8 Hz), 0.63(t, 2 H, J= 4.8 Hz). LC-MS (ESI) [M+H]⁺ calad for C₁₃H₁₄F₃NO,258.1; found 258.3

### Compound 81 (for exemplary purposes only): Preparation of N,2,2,3,3-pentamethyl-N-(3,4,5-trifluorobenzyl)-cyclopropanecarboxamide

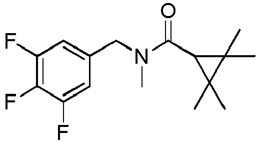

The titled compound 81 was prepared in 28.5% yield from 2,2,3,3-tetramethylcyclopropanecarboxylic acid (30mg) and N-methyl-1-(3,4,5-trifluorophenyl)methanamine (37mg) according to the procedure outlined for compound 65. ¹H NMR: (CDCl₃, 400 MHz): δ6.83-6.87 (m, 2 H), 4.50 (s, 2 H), 2.96 (s, 3 H), 1.21 (s, 6 H), 1.18 (s, 6 H). LC-MS (ESI) [M+H]⁺ calad for C₁₆H₂₀F₃NO,300.1; found 300.3.

### Compound 82 (for exemplary purposes only): Preparation of N-methyl-1-phenyl-N-(2,3,5-trifluorobenzyl)cyclopropanecarboxamide

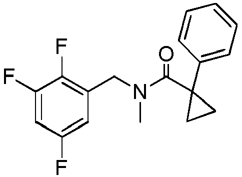

The titled compound 82 was prepared in 30% yield from phenylcyclopropanecarboxylic acid (50mg) and N-methyl-1-(2,3,5-trifluorophenyl)methanamine (55mg) according to the procedure outlined for compound 65. ¹H NMR: (CDCl₃, 400 M Hz): δ7.26-7.30 (m, 1 H), 7.16-7.26 (m, 3 H), 6.74-6.94 (m, 3 H), 4.65 (s, 2 H), 2.85 (s, 3 H), 1.43-1.46 (m, 2 H), 1.23 (m, 2 H). LC-MS (ESI) [M+H]⁺ calad for C₁₈H₁₆F₃NO,320.1; found 320.3.

### Compound 83 (for exemplary purposes only): Preparation ofN-methyl-N-(2,3,5-trifluorobenzyl)cyclobutanecarboxamide

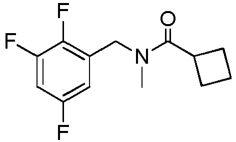

The titled compound 83 was prepared in 29.2% yield from cyclobutanecarboxylic acid (20mg) and N-methyl-1-(2,3,5-trifluorophenyl)methanamine (35mg) according to the procedure outlined for compound 65. ¹H NMR: (CDCl₃, 400 M Hz): δ6.76-6.86 (m, 2 H), 4.61(s, 2 H), 3.29-3.33 (m, 1 H), 2.89 (s, 3 H), 2.32-2.41 (m, 2 H), 2.17-2.22 (m, 2 H), 1.86-1.99 (m, 2 H). LC-MS (ESI) [M+H]⁺ calad for C₁₃H₁₄F₃NO,258.2; found 258.4.

### Compound 84 (for exemplary purposes only): Preparation of N-methyl-N-(2,3,5-trifluorobenzyl)-1-(trifluoromethyl)cyclobutanecarboxamide

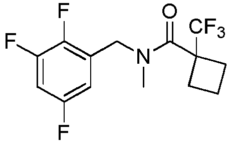

The titled compound 84 was prepared in 25.9% yield from 1-(trifluoromethyl)cyclobutanecarboxylic acid (30mg) and N-methyl-1-(2,3,5-trifluorophenyl)methanamine (31mg) according to the procedure outlined for compound 65. ¹H NMR: (CDCl₃, 400 M Hz): δ 6.80-6.89 (m, 1 H), 6.74-6.77 (m, 1 H), 4.66(s, 2 H), 2.92 (s, 3 H), 2.68-2.77(m, 2 H), 2.52-2.58(m, 2 H), 2.08-2.16(m, 1 H), 1.83-1.87(m, 1 H). LC-MS (ESI) [M+H]⁺ calad for C₁₄H₁₃F₆NO,326.1; found 326.4.

### Compound 85 (for exemplary purposes only): Preparation ofN-methyl-N-(3,4,5-trifluorobenzyl)cyclopentanecarboxamide

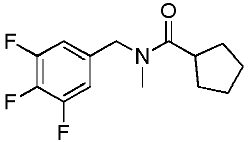

The titled compound 85 was prepared in 25.2% yield from cyclopentanecarboxylic acid (30mg) and N-methyl-1-(3,4,5-trifluorophenyl)methanamine (46mg) according to the procedure outlined for compound 65. ¹H NMR: (CDCl₃, 400 M Hz): δ 6.28-6.86 (m, 2 H), 4.50 (s, 2 H), 2.99 (s, 3 H), 2.93-2.97 (m, 1 H), 1.73-1.89 (m, 6 H), 1.57-1.62 (m, 2 H). LC-MS (ESI) [M+H]⁺ calad for C₁₄H₁₆F₃NO, 272.1; found 272.3.

### Compound 86 (for exemplary purposes only): Preparation ofN-methyl-N-(2,3,5-trifluorobenzyl)-1-(trifluoromethyl)cyclopentanecarboxamide

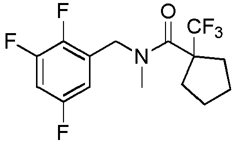

The titled compound 86 was prepared in 26.9% yield from 1-(trifluoromethyl)cyclopentanecarboxylic acid (30mg) and N-methyl-1-(2,3,5-trifluorophenyl)methanamine (29mg) according to the procedure outlined for compound 65. ¹H NMR: (CDCl₃, 400 M Hz): δ 6.79-6.85 (m, 1 H), 6.65-6.69 (m, 1 H), 4.65 (s, 2 H), 3.07(s, 3 H), 2.38-2.44 (m, 2 H), 2.15-2.21(m, 2 H), 1.59-1.74 (m, 4 H). LC-MS (ESI) [M+H]⁺ calad for C₁₅H₁₅F₆NO, 340.1; found, 340.3.

### Compound 87 (for exemplary purposes only): Preparation of N-methyl-1-phenyl-N-(2,3,5-trifluorobenzyl)cyclopentanecarboxamide

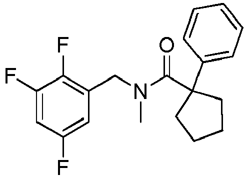

The titled compound 87 was prepared in 29.5% yield from 1-phenylcyclopentanecarboxylic acid (50mg) and N-methyl-1-(2,3,5-trifluorophenyl)methanamine (47mg) according to the procedure outlined for compound 65. ¹H NMR: (CDCl₃, 400 M Hz): δ7.19-7.31 (m, 5 H), 6.74-6.77 (m, 2 H), 4.60 (s, 2 H), 2.54 (s, 3 H), 2.37-2.43 (m, 2 H), 2.02-2.05 (m, 2 H), 1.66-1.77 (m, 4 H). LC-MS (ESI) [M+H]⁺ calad for C₂₀H₂₀F₃NO,348.1; found 348.3.

### Compound 88 (for exemplary purposes only): Preparation of 1-ethyl-N-methyl-N-(3,4, 5-trifluorobenzyl)cyclobutanecarboxamide

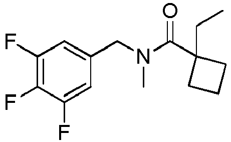

The titled compound 88 was prepared in 19.7% yield from 1-ethylcyclobutanecarboxylic acid (18mg) and N-methyl-1-(3,4,5-trifluorophenyl)methanamine (25mg) according to the procedure outlined for compound 65. ¹H NMR: (CDCl₃, 400 M Hz): δ6.74-6.94 (m, 2 H), 4.46 (s, 2 H), 2.82 (s, 3 H), 2.46-2.55 (m, 2 H), 1.74-1.98 (m, 6 H), 0.88 (t, 3 H, J = 7.6 Hz). LC-MS (ESI) [M+H]⁺ calad for C₁₅H₁₈F₃NO, 286.1; found 286.4.

### Compound 89 (for exemplary purposes only): Preparation of 1-ethyl-N-methyl-N-(3,4,5-trifluorobenzyl)cyclopentanecarboxamide

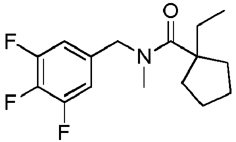

The titled compound 89 was prepared in 18.7% yield from 1-ethylcyclopentanecarboxylic acid (20mg) and N-methyl-1-(3,4,5-trifluorophenyl)methanamine (25mg) according to the procedure outlined for compound 65. ¹H NMR: (CDCl₃, 400 M Hz): δ 6.82-6.90 (m, 2 H), 4.50 (s, 2 H), 2.99 (s, 3 H), 2.18-2.27 (m, 2 H), 1.66 (q, 2 H, J = 7.6 Hz), 1.57-1.62 (m, 6 H), 0.84 (t, 3 H, J = 7.6 Hz). LC-MS (ESI) [M+H]⁺ calad for C₁₆H₂₀F₃NO, 300.2; found 300.4.

### Compound 90: Preparation of N-benzyl-N,2,2-trimethylbutanamide

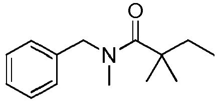

The titled compound 90 was prepared in 72% yield from N-(2-fluorobenzyl)-2,2-dimethylbutanamide (1.312g) and iodomethane ( 1g ) according to the procedure outlined for compound 10. ¹H NMR (CDCl₃): δ 7.21-7.33 (m, 5H), 4.64 (s, 2H), 2.99 (s, 3H), 1.68 (q, 2H, *J* = 7.6 Hz), 1.29 (s, 6H), 0.90 (t, 3H, J=7.6 Hz).

### Compound 91: Preparation of N-(3,4-difluorobenzyl)-N,2,2-trimethylbutanamide

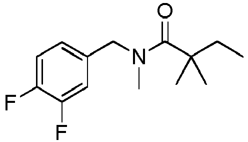

The titled compound 91 was prepared in 45% yield from compound 8 (71.7 mg) and iodomethane ( 84.5mg ) according to the procedure outlined for compound 10.

¹H NMR (CDCl₃): δ 7.03-7.14 (m, 2H), 6.94-6.98 (m, 1H), 4.55 (s, 2H), 3.02 (s, 3H), 1.69 (q, 2H, *J*= 7.6 Hz), 1.29 (s, 6H), 0.89 (t, 3H, *J*=7.6 Hz).

### Compound 92: Preparation of N-benzyl-N-hydroxy-2,2-dimethylbutanamide

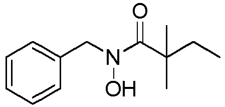

n-benzylhydroxylamine hydrochloride(100mg) was dissolved in 2ml of THF/ H₂O(1:1) and 0.45ml of saturated aqueous NaHCO₃. The solution was cooled to 0°C and 2,2-dimethylbutanoylchloride(81mg) was added and the mixture was stirred at room temperature for 16h. The mixture was extracted with EtOAc and the combined organic layer washed with brine, dried (Na₂SO4)and concentrated in vacuo. Purification by silica gel chromatography to give compound 80(60mg, 43.3%) as an white solid. ¹HNMR(CDCl₃,400MHz):δ7.34-37 (m, 2 H), 7.31-7.33 (m, 3 H), 4.89 (s, 2 H), 1.69 (q, 2 H, J = 7.6Hz), 1.26 (s, 6 H), 0.86 (t, 6 H, J = 7.6 Hz). LC-MS (ESI) [M+H]⁺ calad forC₁₃H₁₉NO₂, 222.1; found 222.4.

### Compound 93: Preparation of N-hydroxy-2,2-dimethyl-N-(2,3,5-trifluorobenzyl)butanamide

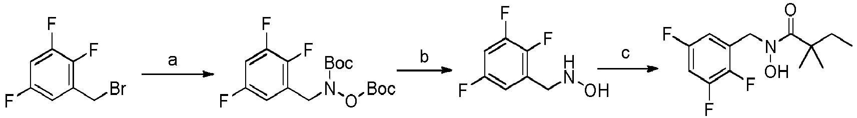

Reagent and conditions; (a) tert-butyl (tert-butoxycarbonyl)oxycarbamate, 1N NaOH, TBAB, DCM; (b) TFA, DCM; (c) 2,2-dimethylbutanoyl chloride, aq. NaHCO₃, THF, H₂O.

Tert-butyl (tert-butoxycarbonyl)oxycarbamate (104mg) and 1-(bromomethyl)-2,3,5-trifluorobenzene(100mg) were dissolved in CH₂Cl₂(10ml). The mixture was added 1M NaOH(4.5ml) and tetrabutylammonium bromide(7mg), and stirred at room temperature for overnight. The resulting mixture was washed with water and dried with Na₂SO₄, concentrated in vacuo and purification by silica gel chromatography to give tert-butyl (tert-butoxycarbonyl)oxy (2,3,5-trifluorobenzyl)-carbamate (150mg, 89%). ¹HNMR(CDCl₃,400MHz):δ 6.95-6.98(m, 1H), 6.81-6.89(m, 1H), 4.82(s, 2H), 1.50(s, 9H), 1.49(s, 9H).

The above intermediate was dissolved in CH₂Cl₂ (2.5ml), TFA(0.8ml)was added at 0 °C. The mixture was stirred at room temperature for 4h and concentrated to give N-(2,3,5-trifluorobenzyl)hydroxylamine (100mg) as a TFA salt, which was used without further purification.

The above intermediate was dissolved in THF (3ml) and water (3ml) and 1ml of saturated aqueous NaHCO₃ was added. The mixture was stirred at room temperature for 30 min, then cooled to 0°C, 2,2-dimethylbutanoylchloride(54mg) was added and stirred for overnight. The mixture was extracted with EtOAc, washed with brine, dried (Na₂SO4), and concentrated in vacuo. Purification by silica gel chromatography to give compound 93 (80mg, total yield 65%). ¹HNMR (CDCl₃, 400MHz): δ 9.80 (s, 1 H), 7.41-7.48 (m, 1 H), 6.91-6.96 (m, 1 H), 4.74 (s, 2 H), 1.64 (q, 2 H, J = 7.6 Hz), 1.13 (s, 6 H), 0.72 (t, 3 H, J = 7.6 Hz). LC-MS (ESI) [M+H]⁺ calad forC₁₃H₁₆F₃NO₂, 276.1; found, 276.2.

### Compound 94: Preparation of N-(4-fluorobenzyl)-N-hydroxy-2,2-dimethylbutanamide

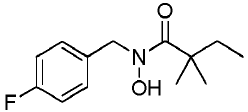

The titled compound 94 was prepared in 71% yield from tert-butyl (tert-butoxycarbonyl)oxycarbamate (247mg), 1-(bromomethyl)-4-fluorobenzene (200mg) and 2,2-dimethylbutanoylchloride (135mg) according to the procedure outlined for compound 93.¹HNMR(CDCl₃,400MHz):δ7.27-7.31 (m, 2 H), 7.02-7.06 (m, 2 H), 4.85 (s, 2 H), 1.68 (q, 2 H, J = 7.6 Hz), 1.26 (s, 6 H), 0.84 (t, 3 H, J = 7.6 Hz). LC-MS (ESI) [M+H]⁺ calad forC₁₃H₁₈FNO₂, 240.1; found 240.2.

### Compound 95: Preparation of N-(3,4-difluorobenzyl)-N-hydroxy-2,2-dimethylbutanamide

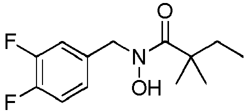

The titled compound 95 was prepared in 71% yield from tert-butyl (tert-butoxycarbonyl)oxycarbamate (225mg), 4-(bromomethyl)-1,2-difluorobenzene (200mg) and 2,2-dimethylbutanoylchloride(135mg) according to the procedure outlined for compound 93.¹HNMR(CDCl₃,400MHz):δ7.10-7.17 (m, 2H), 7.02-7.06 (m, 1 H), 4.81 (s, 2 H), 1.68 (q, 2 H, J = 7.6 Hz), 1.25 (s, 6 H), 0.84 (t, 3 H, J = 7.6 Hz). LC-MS (ESI) [M+H]⁺ calad for C₁₃H₁₇F₂NO₂, 258.1; found 258.2.

### Compound 96: Preparation of N-(2,4-difluorobenzyl)-N-hydroxy-2,2-dimethylbutanamide

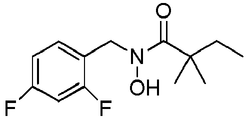

The titled compound 96 was prepared in 65% yield from tert-butyl (tert-butoxycarbonyl)oxycarbamate (225mg), 1-(bromomethyl)-2,4-difluorobenzene (200mg) and 2,2-dimethylbutanoylchloride(135mg) according to the procedure outlined for compound 93.¹HNMR(CDCl₃,400MHz):δ77.32-7.38 (m, 1 H), 6.80-6.90 (m, 2 H), 4.90 (s, 2 H), 1.68 (q, 2 H, J = 7.6 Hz), 1.25 (s, 6 H), 0.84 (t, 3 H, J = 7.6 Hz). LC-MS (ESI) [M+H]⁺ calad forC₁₃H₁₇F₂NO₂, 258.1; found 258.2.

### Compound 97: Preparation of N-hydroxy-2,2-dimethyl-N-(2,3,4-trifluorobenzyl)butanamide

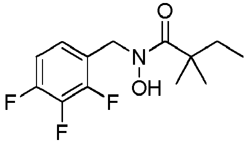

The titled compound 97 was prepared in 65% yield from tert-butyl (tert-butoxycarbonyl)oxycarbamate (104mg), 1-(bromomethyl)-2,3,4-trifluorobenzene (100mg) and 2,2-dimethylbutanoylchloride(54mg) according to the procedure outlined for compound 93.¹HNMR(CDCl₃,400MHz):δ7.08-7.14 (m, 1 H), 6.93-7.00 (m, 1 H), 4.92 (s, 2 H), 1.68 (q, 2 H, J = 7.6 Hz), 1.25 (s, 6 H), 0.84 (t, 3 H, J = 7.6 Hz). LC-MS (ESI) [M+H]⁺ calad forC₁₃H₁₆F₃NO₂, 276.1; found 276.2.

### Compound 98: Preparation of N-hydroxy-2,2-dimethyl-N-(2,4,5-trifluorobenzyl)butanamide

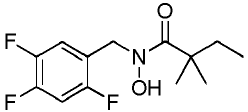

The titled compound 98 was prepared in 65% yield from tert-butyl (tert-butoxycarbonyl)oxycarbamate(104mg), 1-(bromomethyl)-2,4,5-trifluorobenzene(100mg) and 2,2-dimethylbutanoylchloride(54mg) according to the procedure outlined for compound 93.¹HNMR(CDCl₃,400MHz):δ7.19-7.24 (m, 1 H), 6.91-6.98 (m, 1 H), 4.88 (s, 2 H), 1.68 (q, 2 H, J = 7.6 Hz), 1.25 (s, 6 H), 0.84 (t, 3 H, J = 7.6 Hz). LC-MS (ESI) [M+H]⁺ calad forC₁₃H₁₆F₃NO₂, 276.1; found 276.2.

### Compound 99: Preparation of N-hydroxy-2,2-dimethyl-N-(3,4,5-trifluorobenzyl)butanamide

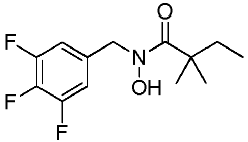

The titled compound 99 was prepared in 65% yield from tert-butyl (tert-butoxycarbonyl)oxycarbamate(104mg), 5-(bromomethyl)-1,2,3-trifluorobenzene(100mg) and 2,2-dimethylbutanoylchloride(54mg) according to the procedure outlined for compound 93.¹HNMR(CDCl₃,400MHz): δ6.92-7.00 (m, 2 H), 4.79 (s, 2 H), 1.68 (q, 2 H, J = 7.6 Hz), 1.26 (s, 6 H), 0.85 (t, 3 H, J = 7.6 Hz).LC-MS (ESI) [M+H]⁺ calad forC₁₃H₁₆F₃NO₂, 276.1; found, 276.2.

### Compound 100 : Preparation of (S)-methyl 3-(2,2-dimethylbutanamido)-3-phenylpropanoate

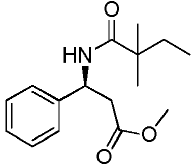

(S)-3-amino-3-phenylpropanoic acid (1g) was dissolved in methanol (10ml), 1ml of thionyl chloride was added at 0°C. The mixture was refluxed for 4h. The solvent was evaporated to dryness and the resulting solid was washed with petroleum ether. The crude product and triethylamine (0.7ml) were dissolved in 15ml of CH₂Cl₂, 2,2-dimethylbutanoylchloride(1g) was added slowly at 0°C under nitrogen. The mixture was stirred at room temperature for 4h. After removal of solvent and purified by silica gel column chromatography to give compound 100 (110mg, 32%). ¹HNMR(CDCl₃,400MHz):δ7.31-7.35 (m, 2H), 7.23-7.28 (m, 3 H), 5.40-5.45 (m, 1 H), 3.62 (s, 3 H), 2.87 (m, 2 H), 1.57 (q, 2 H, J = 7.6Hz), 1.19 (s, 3H ),1.18 (s, 3H ), 0.83 (t, 3 H, J = 7.6 Hz).LC-MS (ESI) [M+H]⁺ calad forC₁₆H₂₃NO₃, 278.2; found 278.4.

### Compound 101 : Preparation of (S)-2,2-dimethyl-N-(3-(methylamino)-3-oxo-1-phenylpropyl)butanamide

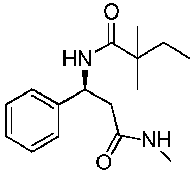

Compound 100(840mg) was dissolved in 30 ml of methanol, 1M NaOH (40ml) was added. The mixture was stirred at room temperature for 5h.The solvent was removed and acidified with 1N HCl. The aqueousphase was extracted with CH₂Cl₂. The combined organic layer was wash with water, dried with Na₂SO₄. Filtered and evaporated to dryness to give (S)-3-(2,2-dimethylbutanamido)-3-phenylpropanoic acid(780mg, 98%) as a brown oil. ¹HNMR(CDCl₃,400MHz):δ 7.31-7.34 (m, 2H), 7.24-7.28(m, 3H), 6.75(d, 1H, J=8.4Hz), 5.40-5.45(m, 1H), 2.83-2.93(m, 2H), 1.55(q, 2H, J= 7.6Hz), 1.16(s, 1H), 1.15(s, 1H), 0.81(t, 3 H, J = 7.6 Hz).

The titled compound 101 was prepared in 55% yield from (S)-3-(2,2-dimethylbutanamido)-3-phenylpropanoic acid (30mg) and methanamine hydrochloride (9.2mg) according to the procedure for compound 65. ¹HNMR(CDCl₃,400MHz):δ7.83 (brs, 1 H), 7.26-7.33 (m, 3 H), 7.21-7.25 (m, 2 H), 5.92 (brs, 1 H), 5.26-5.31 (m, 1 H), 3.67-3.72 (m, 1 H), 3.12-3.19 (m, 1 H), 2.71 (d, 3 H, J = 4.8Hz), 1.58 (m, 2 H), 1.20(s, 3 H), 1.19(s, 3 H), 0.82 (t, 3 H, J = 7.6 Hz). LC-MS (ESI) [M+H]⁺ calad forC₁₅H₂₁NO₃, 277.2; found 277.4.

### Compound 102:

### Preparation of (S)-N-(3-((2-(2-methoxyethoxy)ethyl)amino)-3-oxo-1-phenylpropyl)-2,2-dimethylbutanamide

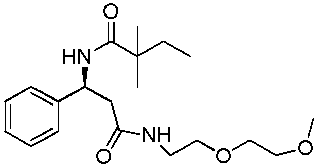

The titled compound 102 was prepared in 51% yield from (S)-3-(2,2-dimethylbutanamido)-3-phenylpropanoic acid (30mg) and 2-(2-methoxyethoxy)ethanamine (16.3mg) according to the procedure outlined for compound 101. ¹HNMR(CDCl₃,400MHz): δ7.98-8.01 (brs, 1 H), 7.27-7.33 (m, 4 H), 7.21-7.25 (m, 1 H), 6.41-6.45 (brs, 1 H), 5.27-5.32 (m, 1 H), 3.67-3.74 (m, 1 H), 3.40-3.56 (m, 5 H), 3.36 (s, 3 H), 3.13-3.19 (m, 2 H), 2.78-2.83 (m, 1 H), 2.68-2.73 (m, 1 H), 1.54-1.62 (m, 2 H), 1.22 (s, 3 H), 1.21 (s, 3 H), 0.83 (t, 3 H, J = 7.2 Hz). LC-MS (ESI) [M+H]⁺ calad forC₂₀H₃₂N₂O₄, 365.2;found 365.4.

### Compound 103:

### Preparation of (S)-N-(3-(ethylamino)-3-oxo-1-phenylpropyl)-2,2-dimethylbutanamide

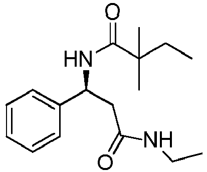

The titled compound 103 was prepared in 39% yield from (S)-3-(2,2-dimethylbutanamido)-3-phenylpropanoic acid (30mg) and ethylamine hydrochloride(11mg) according to the procedure outlined for compound 101. ¹HNMR(CDCl₃,400MHz):δ¹H-NMR (CDCl3) δ 7.87-7.89 (brs, 1 H), 7.27-7.32 (m, 4 H), 7.23-7.25 (m, 1 H), 5.74 (brs, 1 H), 5.28-5.32 (m, 1 H), 3.12-3.24 (m, 2 H), 2.757 (dd, 1 H, J = 4.8, 14.4 Hz), 2.582 (dd, 1 H, J = 5.6, 14.4 Hz), 1.56-1.62 (qd, 2 H, J=7.2, 1.6 Hz), 1.21 (s, 3 H), 1.21 (s, 3 H), 1.006 (t, 3 H, J = 7.2 Hz), 0.823 (t, 3 H, J = 7.2 Hz) LC-MS (ESI) [M+H]⁺ calad forC₁₇H₂₆N₂O₂, 291; found 291.2.

### Compound 104:

### Preparation of (S)-N-(3-(cyclohexylamino)-3-oxo-1-phenylpropyl)-2,2-dimethylbutanamide

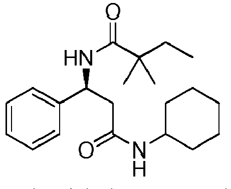

The titled compound 104 was prepared in 21% yield from (S)-3-(2,2-dimethylbutanamido)-3-phenylpropanoic acid(30mg) and cyclohexanamine (14mg) according to the procedure outlined for compound 101. ¹HNMR(CDCl₃,400MHz):δ7.95 (brs, 1 H), 7.27-7.31 (m, 3 H), 7.20-7.24 (m, 2 H), 5.41 (brs, 1 H), 5.28-5.32 (m, 1 H), 3.62-3.71 (m, 1 H), 2.73 (dd, 1 H, J = 4.8, 14.4 Hz), 2.50 (dd, 1 H, J = 4.8, 14.4 Hz), 1.79-1.82 (m, 2 H), 1.56-1.67 (m, 6 H), 1.26-1.36 (m, 2 H), 1.21 (s, 3 H), 1.20 (s, 3 H), 1.01-1.13 (m, 2 H), 0.82 (t, 3 H, J = 7.6 Hz). LC-MS (ESI) [M+H]⁺ calad for C₂₁H₃₂N₂O₂, 345.2; found 345.4.

### Compound 105:

### Preparation of (S)-2,2-dimethyl-N-(3-oxo-1-phenyl-3-(piperidin-1-yl)propyl)butanamide

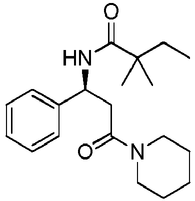

The titled compound 105 was prepared in 29% yield from (S)-3-(2,2-dimethylbutanamido)-3-phenylpropanoic acid (30mg) and piperidine (14mg) according to the procedure outlined for compound 101. ¹HNMR(CDCl₃,400MHz): δ7.99 (brs, 1 H), 7.27-7.32 (m, 4 H), 7.19-7.23 (m, 1 H), 5.32 (m, 1 H), 3.56-3.60 (m, 1 H), 3.32-3.39 (m, 1 H), 3.17-3.24 (m, 2 H), 3.033 (dd, 1 H, J = 5.6, 14.4 Hz), 2.679 (dd, 1 H, J = 4.8, 14.4 Hz), 1.55-1.61 (m, 2 H), 1.50-1.54 (m, 2 H), 1.36-1.48 (m, 3 H), 1.20 (s, 3 H), 1.19 (s, 3 H), 1.04-1.10 (m, 1 H), 0.83 (t, 3 H, J = 7.6 Hz). LC-MS (ESI) [M+H]⁺ calad forC₂₀H₃₀N₂O₂, 331.2, found 331.4.

### Compound 106:

### Preparation of (S)-2,2-dimethyl-N-(3-oxo-1-phenyl-3-(phenylamino)propyl)butanamide

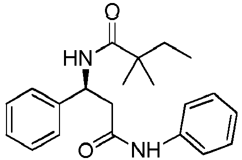

The titled compound 106 was prepared in 28% yield from (S)-3-(2,2-dimethylbutanamido)-3-phenylpropanoic acid(30mg) and aniline(13mg) according to the procedure outlined for compound 101. ¹HNMR (CDCl₃, 400MHz):δ7.65 (brs, 1 H), 7.27-7.40 (m, 9 H), 7.106 (t, 1 H, J = 7.2 Hz), 5.41-5.45 (m, 1 H), 2.83-2.99 (m, 2 H), 1.578 (q, 2 H, J = 7.6 Hz), 1.19 (s, 6 H), 0.81 (t, 3 H, J = 7.6 Hz). LC-MS (ESI) [M+H]⁺ calad forC₂₁H₂₆N₂O₂, 339.2; found, 339.4.

### Compound 107:

### Preparation of (S)-N-(3-(benzylamino)-3-oxo-1-phenylpropyl)-2,2-dimethylbutanamide

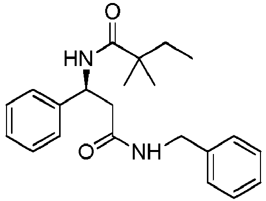

The titled compound 107 was prepared in 28% yield from (S)-3-(2,2-dimethylbutanamido)-3-phenylpropanoic acid(30mg) and phenylmethanamine (15mg) according to the procedure outlined for compound 101. ¹HNMR(CDCl₃,400MHz):δ7.91 (brs, 1 H), 7.28-7.39 (m, 8 H), 7.02-7.04 (m, 2 H), 6.29 (brs, 1 H), 5.33-5.36 (m, 1 H), 4.39 (d, 2 H, J = 5.2 Hz), 2.84-2.91 (m, 1 H), 2.71-2.76 (m, 1 H), 1.59 (qd, 2 H, J=1.2, 7.6 Hz), 1.21 (s, 3 H), 1.20 (s, 3 H), 0.83 (t, 3 H, J = 7.6 Hz). LC-MS (ESI) [M+H]⁺ calad forC₂₂H₂₈N₂O₂, 353.2;found, 353.4.

### Compound 108:

### Preparation of (S)-2,2-dimethyl-N-(3-oxo-3-(phenethylamino)-1-phenylpropyl)butanamide

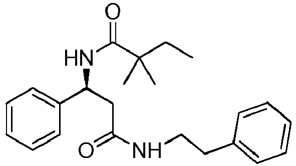

The titled compound 108 was prepared in 31% yield from (S)-3-(2,2-dimethylbutanamido)-3-phenylpropanoic acid (30mg) and phenylmethanamine (16mg) according to the procedure outlined for compound 101. ¹HNMR(CDCl₃,400MHz):δ7.96-7.98 (brs, 1H), 7.27-7.34 (m, 4 H), 7.17-7.25 (m, 4 H), 6.94-6.97 (m, 2 H), 5.59 (brs, 1 H), 5.28-5.32 (m, 1 H), 3.46-3.55 (m, 1 H), 3.25-3.33 (m, 1 H), 2.67-2.75 (m, 2 H), 2.49-2.61 (m, 2 H), 1.58 (qd, 2 H, J=2.0, 7.6 Hz), 1.22 (s, 3 H), 1.21 (s, 3 H), 0.84 (t, 3 H, J = 7.6 Hz). LC-MS (ESI) [M+H]⁺calad forC₂₃H₃₀N₂O₂, 367.2; found, 367.4.

### Compound 109:

### Preparation of (R)-N-(2-hydroxy-1-phenylethyl)-2,2-dimethylbutanamide

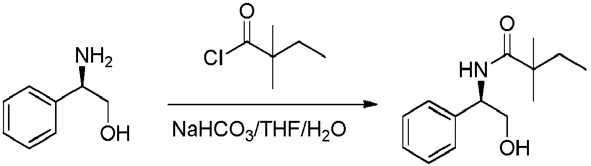

(R)-2-amino-2-phenylethanol (50 mg, 0.365 mmoL) and NaHCO₃ (91.9 mg, 1.094
mmol) were dissolved in 2 mL of THF/H₂O (v/v =1/1).2,2-dimethylbutanoylchloride
(43 mg, 0.398 mmoL) was added slowly to the solution at 0°C under nitrogen. The mixture was stirred at room temperature for 16 h, and extracted with EtOAc (15 mL x 3). The combined organic layers were washed with brine, dried with Na₂SO₄. Filtered and evaporated to dryness. The residue was purified by column chromatography to give compound 109 as white solid (45mg, 59.2%). ¹H NMR: (CDCl₃, 400 M Hz): δ (ppm) 7.35-7.39 (m, 2 H), 7.28-7.32 (m, 3 H), 6.26 (brs, 1 H), 5.05-5.09 (m, 1 H), 3.87-3.94 (m, 2 H), 2.70 (brs, 1 H), 1.59 (q, 2 H, J = 7.6 Hz), 1.20 (s, 3 H), 1.19 (s, 3 H), 0.87 (t, 3 H, J = 7.6 Hz).LC-MS (ESI) [M+H]⁺ calad for C₁₄H₂₁NO₂, 236.2;found 236.3.

### Compound 110:

### Preparation of N-(2-hydroxy-1-phenylethyl)-2,2-dimethylbutanamide

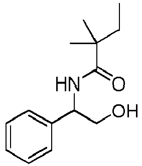

The titled compound 110 was prepared in 78.3% yield from2-amino-2-phenylethanol (50 mg) and 2,2-dimethylbutanoyl chloride (54 mg) according to the procedure outlined for compound109.¹H NMR: (CDCl₃, 400 M Hz) δ7.36-7.40 (m, 2 H), 7.28-7.33 (m , 3 H), 6.27 (brs, 1 H), 5.07 (dd, 1 H, J = 5.6, 10.8 Hz), 3.88-3.92 (m, 2 H), 2.72 (brs, 1 H), 1.58 (q, 2 H, J = 7.6 Hz), 1.20 (s, 3 H), 1.19 (s, 3 H), 0.87 (t, 3 H, J = 7.6 Hz). LC-MS (ESI) [M+H]⁺ calad forC₁₅H₂₃NO₂, 236.2;found 236.3.

### Compound 111:

### Preparation ofN-(2-hydroxy-1-phenylethyl)-N,2,2-trimethylbutanamide

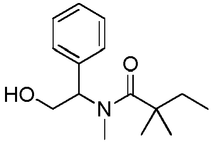

The titled compound 111 was prepared in 9.8% yield from 2-(methylamino)-2-phenylethanol (50 mg) and 2,2-dimethylbutanoyl chloride (48 mg) according to the procedure outlined for compound 109.¹H NMR: (MeOD, 400 M Hz) δ7.43-7.44 (m, 3 H), 7.28-7.38 (m, 2 H), 4.29-4.38 (m, 2 H), 5.88 (m, 1 H), 2.43 (s, 3 H), 1.528 (q, 2 H, J = 7.6 Hz), 1.11 (s, 6 H), 0.75 (t, 3 H, J = 7.6 Hz). LC-MS (ESI) [M+H]⁺ calad forC₁₅H₂₃NO₂,250.2; found 250.3.

### Compound 112:

### Preparation of N-(2-methoxy-1-phenylethyl)-N,2,2-trimethylbutanamide

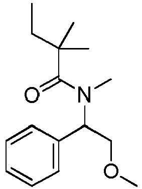

N-(2-hydroxy-1-phenylethyl)-2,2-dimethylbutanamide (40 mg, 0.17 mmoL) was dissolved in 2 mL of dry THF. NaH (20.4 mg, 0.51 mmoL, 60% in material oil) was added in portions to the solution at 0°C under nitrogen. After stirring at 0°C for 0.5h, iodomethane (72.5 mg, 0.51 mmoL) was added. The mixture was stirred at room temperature for 16 h, quenched with water (1ml) and extracted with EtOAc. The combined organic layers were evaporated to dryness and the residue was purified by column chromatography to give compound112(20mg, 44.7%) as colorless oil .¹H NMR: (CDCl₃, 400 MHz) δ7.31-7.35 (m, 2 H), 7.22-7.28 (m, 3 H), 5.70-6.20 (m, 1 H), 3.81-3.90 (m, 2 H), 3.41 (s, 3 H), 2.84 (s, 3 H), 1.63-1.73 (m, 2 H), 1.29 (s, 3 H), 1.28 (s, 3 H), 0.91 (t, 3 H, J = 7.6 Hz). LC-MS (ESI) [M+H]⁺ calad forC₁₆H₂₅NO₂, 264.2;found, 264.4.

### Compound 113:

### Preparation of 3-cyclohexyl-1-(2-fluorobenzyl)-1 -methylurea

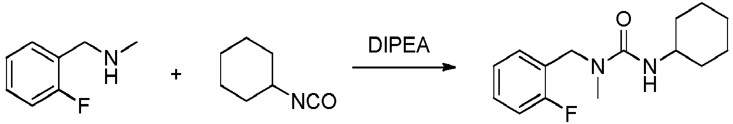

To a solution of 1-(2-fluorophenyl)-N-methylmethanamine (97mg) in THF(10ml) was added N,N-Diisopropylethylamine (135mg), then isocyanatocyclohexane (131mg) in THF(1ml) was added. The mixture was stirred for overnight and diluted with water. The aqueous layer was extracted with CH₂Cl₂ and the combined organic layers were washed with water and brine, dried (Na₂SO₄) and concentrated in vacuo. The residue was purified by column chromatography to give compound 113 (65mg, 35%).¹HNMR: (CDCl₃, 400 MHz): δ7.22-7.34 (m, 2 H), 7.02-7.14 (m, 2 H), 4.53 (s, 2 H), 3.59-3.71 (m, 1 H), 2.90 (s, 3 H), 1.91-1.97 (m, 2 H), 1.57-1.71 (m, 3 H), 1.31-1.42 (m, 2 H), 1.03-1.18 (m, 3 H). LC-MS (ESI) [M+H]⁺ calad for C₁₅H₂₁FN₂O, 265.2;found 265.4.

### Compound 114:

### Preparation of 1-(2-fluorobenzyl)-3-isopropyl-1-methylurea

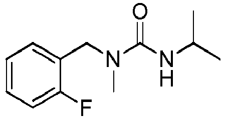

The titled compound 117 was prepared in 30% yield from 2-isocyanatopropane (92mg) and 1-(2-fluorophenyl)-N-methylmethanamine (97mg) according to the procedure outlined for compound 113. ¹H NMR: (CDCl₃, 400 MHz): δ7.22-7.32 (m, 2 H), 7.02-7.14 (m, 2 H), 4.53(s, 2 H), 3.96-4.03 (m, 1 H), 2.89 (s, 3 H), 1.15 (d, 6 H, J = 6.4 Hz), LC-MS (ESI) [M+H]⁺ calad for C₁₂H₁₇FN₂O, 225.1; found 225.2.

### Compound 115:

### Preparation of 1-ethyl-3-isopropyl-3-methyl-1-(2,3,5-trifluorobenzyl)urea

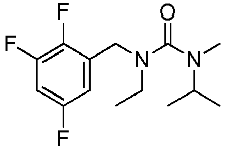

To a solution of triphosgene (154mg) in dichloromethane(4ml) was added N-methylpropan-2-amine(40mg) at 0 °C under nitrogen The mixture was stirred at 0 °C for 4h. Then the solvent was removed , and a solution of N-(2,3,5-trifluorobenzyl)-ethanamine (60mg) in dichloromethane (2ml) was added. The mixture was stirred at 35°C for overnight, diluted with water. The aqueous layer was extracted with EtOAc (5mL x 3). The combined organic layers were washed with saturated NaHCO₃ and brine, dried with Na₂SO₄, filtered and evaporated to dryness. The residue was purified by column chromatography to give compound 115 (4mg, 2.5%) as a yellow oil. ¹H NMR: (CDCl₃, 400 MHz): δ6.86-6.89 (m, 1 H), 6.76-6.81 (m, 1 H), 4.38 (s, 2 H), 4.07 (m, 1 H), 3.09-3.14(q, 2 H, J = 7.2 Hz), 2.70 (s, 3 H), 1.13-1.17 (m, 9 H).LC-MS (ESI) [M+H]⁺ calad forC₁₄H₁₉F₃N₂O, 289.1; found 289.2.

### Compound 116:

### Preparation of 2-ethyl-N-methyl-N-(2,3, 5-trifluorobenzyl)piperidine-1-carboxamide

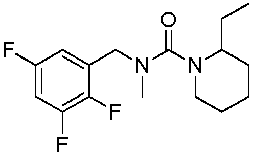

The titled compound 119 was prepared in 14.9% yield from triphosgene (77. 1mg), 2-ethylpiperidine(29.4mg) and N-methyl-1-(2,3,5-trifluorophenyl)methanamine (30mg) according to the procedure outlined for compound 115. ¹H NMR: (CDCl₃, 400 M Hz):δ6.79-6.88 (m, 2 H), 4.45 (d, 1 H, J = 15.6 Hz), 4.29 (d, 1 H, J = 15.6 Hz), 3.72-3.74 (m, 1 H), 3.43-3.48 (m, 1 H), 2.94-3.01 (m, 1 H), 2.77 (s, 3 H), 1.57-1.70 (m, 8 H), 0.98 (t, 3 H, J = 7.6 Hz). LC-MS (ESI) [M+H]⁺ calad for C₁₆H₂₁F₃N₂O, 315.2; found 315.3.

### Compound 117 (for exemplary purposes only): Preparation of N,2-dimethyl-N-(2,3,5-trifluorobenzyl)piperidine-1-carboxamide

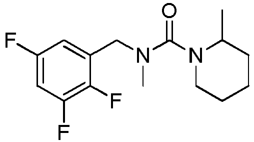

The titled compound 120 was prepared in 15.6% yield from triphosgene (77. 1mg), 2-methylpiperidine (25.71mg) andN-methyl-1-(2,3,5-trifluorophenyl)methanamine (30mg) according to the procedure outlined for compound 115. ¹H NMR: (CDCl₃, 400 M Hz): δ6.78-6.84 (m, 2 H), 4.43 (d, 1 H, J = 16 Hz), 4.34 (d, 1 H, J = 16 Hz), 3.90-3.93 (m, 1 H), 3.32-3.36 (m, 1 H), 2.95-3.02 (m, 1 H), 2.76 (s, 3 H), 1.44-1.68 (m, 6 H), 1.18 (d, 3 H, J = 4 Hz). LC-MS (ESI) [M+H]⁺ calad for C₁₅H₁₉F₃N₂O ,301.1; found 301.3.

### Compound 118:

### Preparation of N,3-dimethyl-N-(2,3,5-trifluorobenzyl)piperidine-1-carboxamide

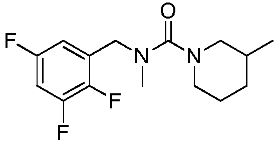

The titled compound 121 was prepared in 9.7% yield from triphosgene (84.8mg), 2-methylpiperidine (28.3mg) and N-methyl-1-(2,3,5-trifluorophenyl)methanamine (30mg) according to the procedure outlined for compound 115 . ¹H NMR: (CDCl₃, 400 MHz): δ7.05-7.07 (m, 1 H), 6.89-6.91 (m, 1 H), 4.42-4.84 (m, 2 H), 2.80 (s, 3 H), 3.49-3.53 (m, 2 H), 2.75-2.77 (m, 2 H), 1.77-1.79 (m, 1 H), 1.57-1.67 (m, 4 H), 0.98 (d, 3 H, J = 6.4 Hz). LC-MS (ESI) [M+H]⁺ calad for C₁₅H₁₉F₃N₂O ,301.1; found 301.3.

### Compound 119:

### Preparation of 1,1-diisopropyl-3-methyl-3-(2,3,5-trifluorobenzyl)urea

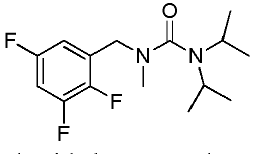

The titled compound 119 was prepared in 19.3% yield from triphosgene (84.8mg), diisopropylamine (26.86mg) and N-methyl-1-(2,3,5-trifluorophenyl)methanamine (30mg) according to the procedure outlined for compound 115. ¹H NMR: (CDCl₃, 400 M Hz): δ6.79-6.82 (m, 2 H), 4.30 (s, 2 H), 3.58-3.62 (m, 2 H), 2.68 (s, 3 H), 1.26 (d, 12 H, J = 6.4 Hz).LC-MS (ESI) [M+H]⁺ calad for C₁₅H₂₁F₃N₂O, 303.2; found 303.4.

### Compound 120:

### Preparation of isopropyl-1,3-dimethyl-3-(2,3,5-trifluorobenzyl)urea

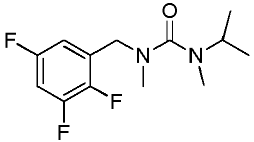

The titled compound 120 was prepared in 20.8% yield from triphosgene(84.8mg), N-methylpropan-2-amine (20.86mg) andN-methyl-1-(2,3,5-trifluorophenyl)methanamine (30mg) according to the procedure outlined for compound 115. ¹H NMR: (CDCl₃, 400 M Hz): δ6.80-6.87 (m, 2 H), 4.38 (s, 2 H), 4.00-4.11 (m, 1 H), 2.75 (s, 3 H), 2.67 (s, 3 H), 1.10 (d, 6 H, J = 6.4 Hz). LC-MS (ESI) [M+H]⁺ calad for C₁₃H₁₇F₃N₂O, 275.1; found 275.2.

### Compound 121:

### Preparation of N,2,6-trimethyl-N-(2,3,5-trifluorobenzyl)piperidine-1-carboxamide

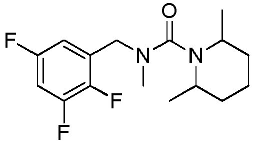

The titled compound 121 was prepared in 14.5% yield from triphosgene (84.8mg), 2,6-dimethylpiperidine (32.28mg) and N-methyl-1-(2,3,5-trifluorophenyl)methanamine (30mg) according to the procedure outlined for compound 115. ¹H NMR: (CDCl₃, 400 M Hz):δ6.80-6.90 (m, 2 H), 4.61 (s, 2 H,), 3.18-3.23 (m, 2 H), 2.97 (s, 3 H), 1.70-1.75 (m, 1 H), 1.59-1.65 (m,2 H), 1.32-1.45 (m, 1 H), 1.22-1.39 (m, 2 H), 1.12 (d, 6 H, J = 6.4 Hz).LC-MS (ESI) [M+H]⁺ calad forC₁₆H₂₁F₃N₂O, 315.2;found 315.4.

### Compound 122:

### Preparation of (R)-2,2-dimethyl-N-(2-(methylamino)-2-oxo-1-phenylethyl)butanamide

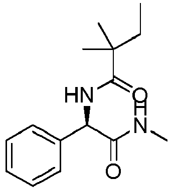

The titled compound 122 was prepared in 35% yield from (R)-2-(2,2-dimethylbutanamido)-2-phenylacetic acid (30mg), which was prepared from (R)-2-amino-2-phenylacetic acid according to the procedure outlined for compound 101, and methanamine hydrochloride(10mg) according to the procedure outlined for compound 65. ¹HNMR(CDCl₃,400MHz): δ7.28-7.38 (m, 5 H), 7.14 (brs, 1 H), 6.38 (brs, 1 H), 5.52 (d, J=6.8 Hz, 1H), 2.77 (d, J=4.8 Hz, 3H), 1.51-1.60 (m, 2 H), 1.18 (s, 3H), 1.17 (s, 3H), 0.78 (t, 3 H, J = 7.6 Hz). LC-MS (ESI) [M+H]⁺calad for C₁₅H₂₂N₂O₂, 263.2; found, 263.3.

### Compound 123:

### Preparation of (R)-N-(2-(dimethylamino)-2-oxo-1-phenylethyl)-2,2-dimethylbutanamide

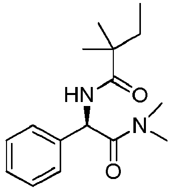

The titled compound 186 was prepared in 35% yield from (R)-2-(2,2-dimethylbutanamido)-2-phenylacetic acid (30mg) and dimethylamine (6.48mg) according to the procedure outlined for compound 65. ¹HNMR (CDCl₃,400MHz): δ7.28-7.41 (m, 5 H), 5.80(d, 1 H, J = 6.8 Hz), 2.99 (s, 3 H), 2.89 (s, 3 H), 1.45-1.55 (m, 2 H), 1.13 (s, 3 H), 1.12 (s, 3 H), 0.70 (t, 3 H, J = 7.6 Hz). LC-MS (ESI) [M+H]⁺calad forC₁₆H₂₄N₂O₂, 277.2; found, 277.4.

### Compound 124:

### Preparation of (R)-N-(2-(benzylamino)-2-oxo-1 -phenylethyl)-2,2-dimethylbutanamide

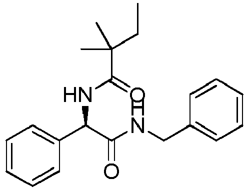

The titled compound 124 was prepared in 37% yield from (R)-2-(2,2-dimethylbutanamido)-2-phenylacetic acid (30mg) and phenylmethanamine (15.4mg) according to the procedure outlined for compound 65. ¹HNMR(CDCl₃,400MHz): δ 7.28-7.38 (m, 5 H), 7.23-7.26 (m, 2 H), 7.07-7.12 (m, 3 H), 6.14(brs, 1 H), 5.49 (d, 1 H, J = 6.4 Hz), 4.42 (d, 2 H, J = 5.2 Hz), 1.49-1.55 (m, 2 H), 1.16 (s, 3 H), 1.15 (s, 3 H), 0.76 (t, 3 H, J=7.6 Hz).LC-MS (ESI) [M+H]⁺calad forC₂₁H₂₆N₂O₂, 339.2;found,339.4.

### Compound 125:

### Preparation of (R)-2,2-dimethyl-N-(2-oxo-2-(phenethylamino)-1-phenylethyl)butanamide

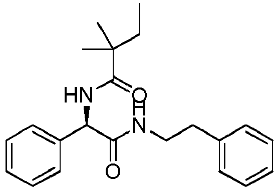

The titled compound 188 was prepared in 38% yield from(R)-2-(2,2-dimethylbutanamido)-2-phenylacetic acid (30mg) and 2-phenylethanamine (17.4mg) according to the procedure outlined for compound 65. ¹HNMR(CDCl₃,400MHz): δ7.28-7.35 (m, 5 H), 7.17-7.23 (m, 3 H), 7.11-7.12 (brs, 1 H), 6.92-6.94 (m, 2H), 5.57 (brs, 1 H), 5.25-5.27 (d, 1 H, J=6.0 Hz), 3.57-3.65 (m, 1 H), 3.32-3.40 (m, 1 H), 2.63-2.77 (m, 2 H), 1.50-1.56 (qd, 2 H, J=7.6, 2.0 Hz), 1.16 (s, 3 H), 1.15 (s, 3 H), 0.76 (t, 3 H, J = 7.6 Hz). LC-MS (ESI) [M+H]⁺ calad for C₂₂H₂₈N₂O₂, 353.2; found, 353.4.

### Compound 126:

### Preparation of (S)-2,2-dimethyl-N-(3-oxo-3-((2-phenoxyethyl)amino)-1-phenylpropyl)butanamide

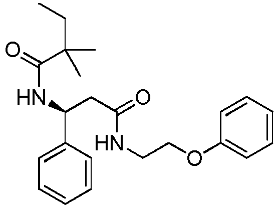

The titled compound 126 was prepared in 42% yield from (S)-3-(2,2-dimethylbutanamido)-3-phenylpropanoic acid (30mg) and 2-phenoxyethanamine (19mg) according to the procedure outlined for compound 65.¹HNMR(CDCl₃,400MHz): δ 7.79-7.81 (brs, 1 H), 7.27-7.31 (m, 2 H), 7.19-7.26 (m, 4 H), 7.09-7.13 (m, 1 H), 6.95-7.00 (m, 1 H), 6.78-6.81 (m, 2 H), 5.94 (brs, 1 H), 5.31-5.35 (m, 1 H), 3.90-3.94 (m, 1 H), 3.79-3.84 (m, 1 H), 3.50-3.61 (m, 2 H), 2.77 (dd, 1 H, J = 4.8, 14.4 Hz), 2.62 (dd, 1 H, J = 4.8, 14.4 Hz), 1.56-1.63 (m, 2 H), 1.21 (s, 3 H), 1.20 (s, 3 H), 0.83 (t, 3 H, J = 7.6 Hz). LC-MS
(ESI)[M+H]⁺caladforC₂₃H₃₀N₂O₃, 383.2, found, 383.4.

### Compound 127:

### Preparation of (R)-2,2-dimethyl-N-(2-oxo-2-((2-phenoxyethyl)amino)-1-phenylethyl)butanamide

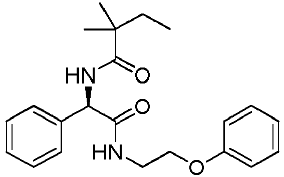

The titled compound 127 was prepared in 40% yield from (R)-2-(2,2-dimethylbutanamido)-2-phenylacetic acid (30mg) and 2-phenoxyethanamine (20mg) according to the procedure outlined for compound 65.¹HNMR(CDCl₃,400MHz): δ 7.27-7.36 (m, 5 H), 7.23-7.25 (m, 1 H), 6.92-7.00 (m, 2 H), 6.77-6.80 (m, 2 H), 6.10 (brs, 1 H), 5.41 (d, 1 H, J = 6.4 Hz), 3.94-4.03 (m, 2 H), 3.57-3.71 (m, 2 H), 1.51-1.57 (m, 2 H), 1.17 (s, 3 H), 1.16 (s, 3 H), 0.77 (t, 3 H, J = 7.6 Hz). LC-MS (ESI) [M+H]⁺caladfor C₂₂H₂₈N₂O₃, 369.2; found, 369.4.

### Compound 128: Preparation of N-((4,5-dimethylthiophen-2-yl)methyl)-N,2,2-trimethylbutanamide

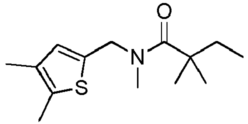

The titled compound 128 was prepared in 36% yield from 1-(4,5-dimethylthiophen-2-yl)-N-methylmethanamine (60mg), which was prepared from 4,5-dimethylthiophene-2-carbaldehyde and methanamine hydrochloride according to the procedure outlined for compound 13, and 2,2-dimethylbutanoyl chloride (57mg) according to the procedure outlined for compound 52. ¹H NMR: (CDCl₃, 400 M Hz):δ6.61 (s, 1 H), 4.58 (s, 2 H), 3.03 (s, 3 H), 2.28 (s, 3 H), 2.07 (s, 3 H), 1.66 (q, 2 H, J = 7.6 Hz), 1.27 (s, 6 H), 0.88 (t, 3 H, J = 7.6 Hz).LC-MS (ESI) [M+H]⁺calcd for C₁₄H₂₃NOS254.2;found 254.3

### Compound 129:

### Preparation of 2,6-dichloro-N-methyl-N-(3,4, 5-trifluorobenzyl)benzamide

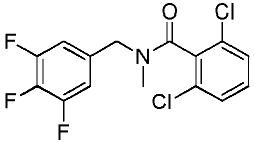

The titled compound 129 was prepared in 77% yield from N-methyl-1-(3,4,5-trifluorophenyl)methanamine (30mg) and 2,6-dichlorobenzoyl chloride (39.5mg) according to the procedure outlined for compound 52. ¹H NMR: (CDCl₃, 400 M Hz):δ7.33-7.36 (m, 2 H), 7.26-7.29 (m, 1 H), 7.04-7.07 (m, 2 H), 4.72 (s, 2 H), 2.77 (s, 3 H). LC-MS (ESI) [M+H]⁺calad forC₁₅H₁₀C₁₂F₃NO, 348.0; found, 348.2

### Compound 130-135 and 151:

### Compound 130-135 are prepared according to the method of scheme 1

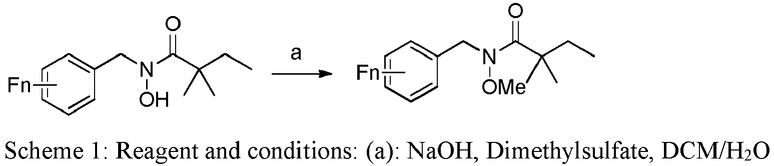

### Compound 136-147:

Compounds 136-147 are prepared according to the method of scheme 2

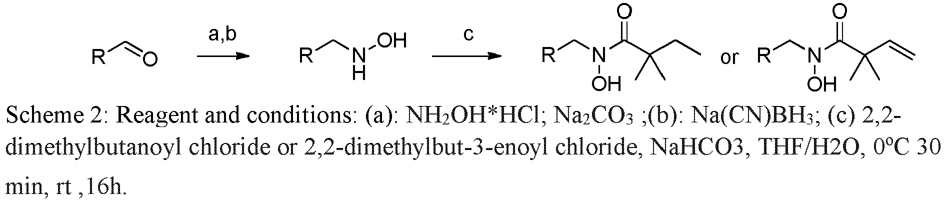

### Compound 148 and 149:

Compound 148 and 149 are prepared according to the method of scheme 3

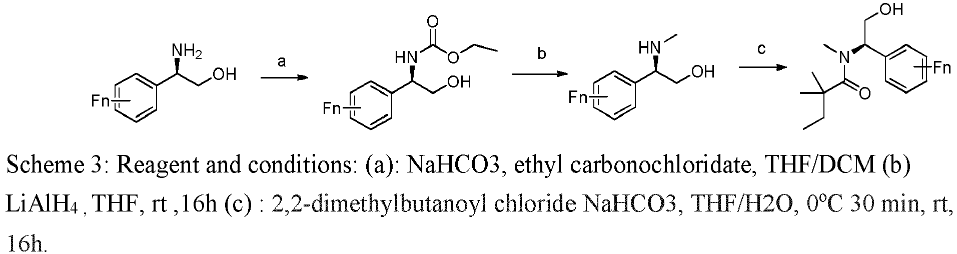

### Compound 150:

Compound 150 is prepared according to the method of scheme 4

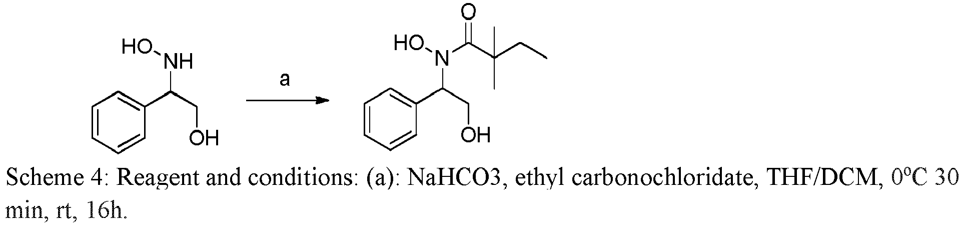

### Compound S1:

### Preparation of N-(2,3,5-trifluorobenzyl)pivalamide

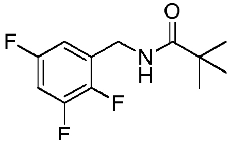

(2,3,5-trifluorophenyl)methanamine (42 mg, 0.263 mmol) and triethylamine (53.2 mg, 0.526mmol) were dissolved in 2 mL of dry CH₂Cl₂. Pivaloyl chloride (38mg, 0.316 mmol) was added slowly to the solution at 0°C under nitrogen. The mixture was stirred at room temperature for 2 h, diluted with CH₂Cl₂ and water. The organic layer were washed with saturated NaHCO₃ solution , brine, dried with Na₂SO₄ and concentrated The residue was purified by chromatography to give compound **S1** (49 mg, 74%) as an light yellow oil. ¹H NMR (400 MHz, CDCl₃) δ 6.86 - 6.77 (m, 2H), 4.47 (d, *J=* 4.9 Hz, 2H), 1.21 (s, 9H). LC-MS (ESI) [M+H]⁺calad for C₁₂H₁₅F₃NO, 246.11; found, 246.17.

### Compound S2:

### Preparation ofN-methyl-N-(2,3,5-trifluorobenzyl)pivalamide

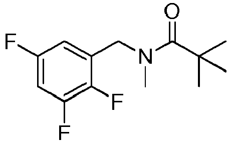

Compound **S1** ( 70mg) was dissolved in 2 mL of dry THF, 17mg of NaH(60%) was added at 0oC under N2 and stirred for 2h. Iodomethane (0.026 mL ) was added and the mixture was allowed to warm to room temperature and stirred for 12h. The mixture was quenched with cold water and extracted with DCM, the combined organic layers was washed with water, brine, dried over Na₂SO₄, concentrated and the residue was purified by pre-TLC to give the product S2 ( 35 mg, 47%). ¹H NMR (400 MHz, CDCl₃) δ 6.82 -6.71 (m, 2H), 4.65 (s, 2H), 3.11 (s, 3H), 1.33 (s, 9H). LC-MS (ESI) [M+H]⁺calad for C₁₃H₁₇F₃NO, 260.13; found, 260.19.

### Compound S3 (for exemplary purposes only): Preparation of N-acetoxy-N-benzyl-2, 2-dimethylbutanamide

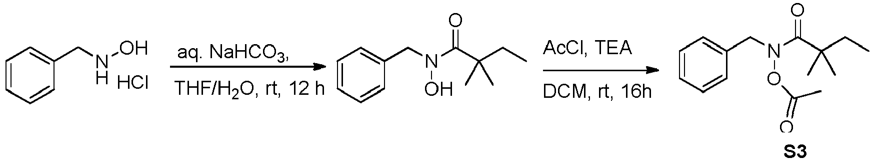

n-benzylhydroxylamine hydrochloride(100 mg) was dissolved in 2 mL of THF/ H₂O(1:1) and 0.45mL of saturated aqueous NaHCO₃. The solution was cooled to 0°C and 2,2-dimethylbutanoylchloride(81mg) was added and the mixture was stirred at rt for 16h. The mixture was extracted with EtOAc and the combined organic layer washed with brine, dried (Na₂SO4) and concentrated in vacuo. Purification by silica gel chromatography to give N-benzyl-N-hydroxy-2,2-dimethylbutanamide (60 mg, 43.3%) as an white solid. ¹H NMR (400 MHz, CDCl₃) δ 7.37-7.34 (m, 2H), 7.33-7.31 (m, 3H), 4.89 (s, 2H), 1.69 (q, *J* = 7.6Hz, 2 H), 1.26 (s, 6 H), 0.86 (t, *J* = 7.6 Hz, 6 H).

N-benzyl-N-hydroxy-2,2-dimethylbutanamide(800 mg) and TEA(2.5 mL) were dissolved in 20 mL of DCM. Acetyl chloride(0.283 mL) was added slowly to the mixture at 0°C and the mixture was stirred at room temperature for 16h, concentrated and the residue was purified by chromatography to give product **S3** (260 mg, 27.3%).¹H NMR (400 MHz, CDCl₃) δ 7.33 - 7.23 (m, 5H), 4.89 (s, 2H), 2.09 (s, 3H), 1.53 (q, *J*= 7.5 Hz, 2H), 1.16 (s, 6H), 0.80 (t, *J*= 7.5 Hz, 3H).

### Compound S4:

### Preparation of N-benzyl-N-methoxy-2,2-dimethylbutanamide

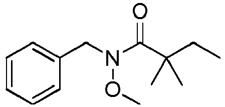

N-benzyl-N-hydroxy-2,2-dimethylbutanamide (800 mg), iodomethane(565.2 mg) and KOH(179.4 mg) were added in 30 mL of ethanol. The mixture was stirred at 50°C for 5h and evaporated to dryness. The residue was diluted with CH₂Cl₂ and water. The organic layer were washed with brine, dried with Na₂SO₄ and concentrated The residue was purified by chromatography to give compound **S4** (210 mg, 28.2%). ¹H NMR (400 MHz, CDCl₃) δ 7.34 - 7.22 (m, 5H), 4.79 (s, 2H), 3.64 (s, 3H), 1.63 (q, *J* = 7.5 Hz, 2H), 1.20 (s, 6H), 0.79 (t, *J*= 7.5 Hz, 3H).

### Compound S5: Preparation of

### 3,3-difluoro-N,2,2-trimethyl-N-(2,3,5-trifluorobenzyl)butanamide

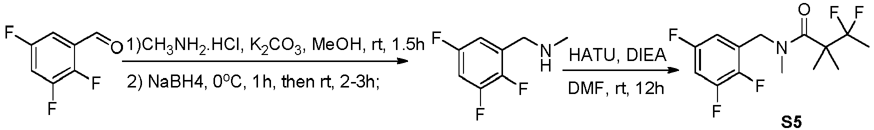

A mixture of K₂CO₃ (324 mg, 2.35 mmol) and methanamine hydrochloride (316 mg, 4.69 mmol) in 10 mL of MeOH was stirred at rt for 30 min. Then 2,3,5-trifluorobenzaldehyde (500 mg, 3.125 mmol) was added to the mixture and stirred at rt for 2 h. The mixture was cooled to 0°C, and NaBH₄ (178.2 mg, 4.69 mmol) was added in portions. The mixture was stirred at 0°C for 1h and warmed to room temperature and stirred for 12h . The solid was filtered and washed with EtOAc. The filtrate was evaporated to dryness and the residue was dissolved in EtOAc and the organic layer was washed with water, brine, dried over Na₂SO₄, concentrated to give N-methyl-1-(2,3,5-trifluorophenyl)methanamine (260 mg ), which used for next step without further purification. ¹H NMR (400 MHz, CDCl₃) δ 6.94 - 6.87 (m, 1H), 6.86 - 6.76 (m, 1H), 3.81 (d, *J* = 1.4 Hz, 2H), 2.44 (s, 3H).

To a solution ofN-methyl-1-(2,3,5-trifluorophenyl)methanamine(44 mg) and 3,3-difluoro-2,2-dimethylbutanoic acid (38 mg) in dry DMF (1 mL)was added 2-(7-Aza-1H-benzotriazole-1-yl)-1,1,3,3-tetramethyluronium hexafluorophosphate( 142 mg) and DIEA (0.08 mL). The mixture was stirred at room temperature for 12h and concentrated in vacuo. The residue was diluted with CH₂Cl₂ and water. The aqueous layer was extracted with CH₂Cl₂. The combined organic layer was washed with saturated brine, dried with Na₂SO₄ and concentrated. The residue was purified by pre-TLC to give compound **S5** (36 mg, 46%). ¹H NMR (400 MHz, CDCl₃) δ 6.88 - 6.78 (m, 1H), 6.71 (m, 1H), 4.67 (s, 2H), 3.15 (s, 3H), 1.66 (t, *J* = 19.4 Hz, 3H), 1.46 (s, 6H). LC-MS (ESI) [M+H]⁺calad for C₁₄H₁₇F₅NO, 310.12; found, 310.21.

### Compound S6: Preparation of

### N-benzyl-3,3-difluoro-N-hydroxy-2,2-dimethylbutanamide

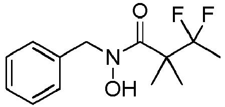

To a solution of n-benzylhydroxylamine hydrochloride (36.8 mg) and 3,3-difluoro- 2,2-dimethylbutanoic acid(35 mg) in dry DMF (1 mL) was added 1-Ethyl-3-(3-dimethylaminopropyl)carbodiimide hydrochloride (66 mg) and DIEA (0.16 mL). The mixture was stirred at room temperature for 12h and concentrated in vacuo. The residue was diluted with CH₂Cl₂ and water. The aqueous layer was extracted with CH₂Cl₂. The organic layer were washed with saturated brine, dried with Na₂SO₄ and concentrated. The residue was purified by pre-TLC to give compound **S6** (10 mg, 17%). ¹H NMR (400 MHz, CDCl₃) δ 7.34 - 7.25 (m, 5H), 4.64 (s, 2H), 1.73 (t, *J* = 19.8 Hz, 3H), 1.38 (s, 6H).

### Compound S7: Preparation of N-(4-fluorobenzyl)-N,2,2-trimethylbutanamide

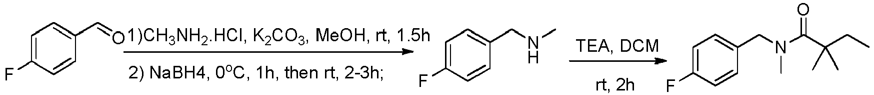

A mixture of K₂CO₃ (207 mg, 1.5 mmol) and methanamine hydrochloride (202 mg, 3.0 mmol) in 5 mL of MeOH was stirred at rt for 30 min. Then 4-fluorobenzaldehyde (248 mg, 2.0 mmol) was added to the mixture and stirred at rt for 1 h. The mixture was cooled to 0°C, and NaBH₄ (113.5 mg, 3.0 mmol) was added in portions. The mixture was stirred at 0°C for 1h and warmed to room temperature and stirred for 2h . The solid was filtered and washed with EtOAc. The filtrate was evaporated to dryness and the residue was dissolved in EtOAc and the organic layer was washed with water, brine, dried over Na₂SO₄. The residue was dissolved in 10 mL of dry THF. DIEA (264 mg, 2.05 mmol ) was added, 2,2-dimethylbutanoyl chloride (275 mg, 2.05 mmol) was added slowly to the solution at 0°C under nitrogen, then stirred at room temperature for 2 h. 15 mL of water was added to the solution and extracted with EtOAc (10 mL x 3). The combined organic was washed with 1M HCl, brine, dried with Na₂SO₄ and concentrated in vacuo. The residue was purified by silica gel column chromatography (PE/EA= 1/2) to give the 189 mg of S7 as a brown solid (total yield = 40%). ¹H NMR (400 MHz, CDCl₃) δ 7.21 - 7.12 (m, 2H), 6.98-6.93 (m, 2H), 4.54 (s, 2H), 2.95 (s, 3H), 1.64 (q, *J* = 7.5 Hz, 2H), 1.24 (s, 6H), 0.84 (t, *J* = 7.5 Hz, 3H).LC-MS (ESI) [M+H] ⁺calad for: C₁₄H₂₁F₂NO, 256.16; found, 256.18.

### Compound S8: Preparation of N-(2,3-difluorobenzyl)-N,2,2-trimethylbutanamide

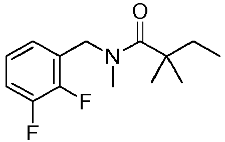

Compound **S8** was prepared in 56% yield from 2,3-difluorobenzaldehyde (284 mg), methanamine hydrochloride(202 mg) and 2,2-dimethylbutanoyl chloride(275 mg) according to the procedure outlined for compound 7. ¹H NMR(400 MHz, CDCl₃) δ 7.11 - 6.94 (m, 3H), 4.66 (s, 2H), 3.06 (s, 3H), 1.66 (q, *J* = 7.5 Hz, 2H), 1.25 (s, 6H), 0.85 (t, *J* = 7.5 Hz, 3H). LC-MS (ESI) [M+H] ⁺calad for: C₁₄H₂₀F₂NO, 256.15; found, 256.18.

### Compound S9-S20:

### Compound S9-S20 are prepared according to the procedure outlined in scheme1

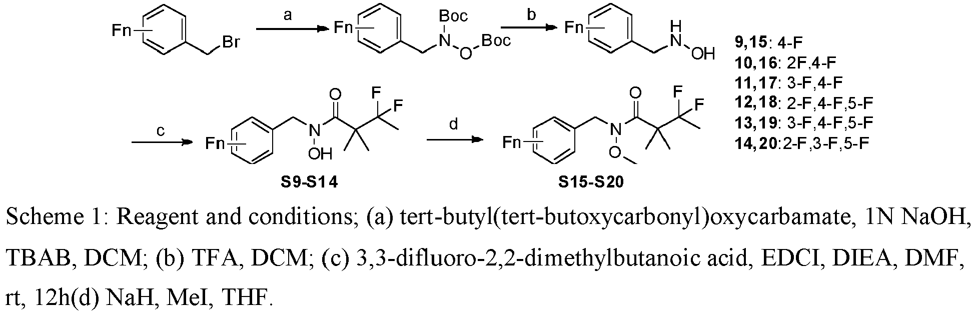

### 3. Kinase assay of RIPK1

Materials: Recombinant full-length RIPK1 protein with N-terminal GST-tag (Cat#R07-34G) was purchased from SignalChem. The ADP-GloTM kinase assay kit (Cat#V9102) was from Promega. MBP (cat# M2295) protein and all the other chemicals were from Sigma. The 384-well assay plates (Cat# 3674, white, opaque) were purchased from Corning.

Kinase activity assay and data analysis: The RIPK1 kinase assay was performed in white 384-well plate. The assay buffer contained 25mM HEPES (pH7.2), 20 mM MgCl2, 12.5 mM MnCl2, 5 mM EGTA, 2 mM EDTA, 12.5 mM β-glycerol phosphate and 2 mM DTT. RIPK1 was first incubated with compounds or DMSO control for 15 min, then ATP/MBP substrate mixture was added to initiate the reaction. The final concentration of RIPK1 was 161 nM, while the final concentration of ATP was 50 uM , and MBP 20uM. After 90 min reaction at room temperature, the ADP-Glo reagent and detection solution were added following the technical manual of ADP-GloTM kinase assay kit (Promega). The luminescence was measured on PerkinElmer Enspire. The data was analyzed using Graphpad Prism (GraphPad Software; www.graphpad.com). The curves were fitted using a non-linear regression model with a sigmoidal dose response.

Results: pIC50 of hRIP1 kinase assay correlated with our pIC50 of cell necrosis assay. Exemplary data are shown below:

| # | RIP 1 CMPD ID | Cell viability assay, EC50 (nM) | hRIP1 kinase assay, IC50(nM) or % inhibition at 2uM |
|---|---|---|---|
| 14 | TC001004 | 3277 | 66% inhibition at 2uM |
| 16 | TC001014 | 150.9/70.59 | IC50=52nM |
| 75 | TC001035 | 0.247/10.21 | IC50=33nM |
| 92 | TC001165 | 28.36 | IC50=13.2nM |
| 99 | TC001186 | 45.7 | IC50=29.6nM |

### 4. Necrosis Assay

### Methods:

HT-29 cells were cultured in McCoy's 5A culture medium (Invitrogen). On day one, HT-29 cells were plated in 96-well assay plates at density of 2,500-3,500 cells per well. On day two, necrosis were induced by adding 20ng/ml TNF-α (T), 100nM Smac mimetic (S), and 20mM z-VAD (Z). At the same time, 10mM compound from a chemical library of ∼200,000 compounds was delivered into each well. After 24 hrs treatment, cell viability was determined by measuring ATP level using the CellTiter-Glo Luminescent Cell Viability Assay kit. A CellTiter-Glo Assay (Promega) was performed according to the manufacturer's instructions Luminescence was recorded with a PerkinElmer EnSpire Multimode Plate Reader. Survived cells were normalized to those cells treated with DMSO. Nec-1 was used as a positive control for screening necrosis inhibitors. Data are represented as mean ± standard deviation of duplicates

Dose-dependent inhibition of necrosis by the compounds in HT-29 cells were determined by measuring ATP levels as described above. Compound necrosis activity data are reported below:

| # | EC50 | # | EC50 | # | EC50 |
|---|---|---|---|---|---|
| 1 | 1-10uM | 52 | 1-100uM | 103 | 1-100uM |
| 2 | 1-10uM | 53 | 1-100uM | 104 | 1-100uM |
| 3 | 1-10uM | 54 | 1-100uM | 105 | 1-100uM |
| 4 | 1-10uM | 55 | 1-100uM | 106 | 1-100uM |
| 5 | 1-10uM | 56 | 1-10uM | 107 | 1-100uM |
| 6 | 1-10uM | 57 | 1-10uM | 108 | 1-100uM |
| 7 | 1-10uM | 58 | 1-1000nM | 109 | 1-100uM |
| 8 | 1-10uM | 59 | 1-10uM | 110 | 1-100uM |
| 9 | 1-10uM | 60 | 1-10uM | 111 | 1-100uM |
| 10 | 1-10uM | 61 | 1-100uM | 112 | 1-100uM |
| 11 | 1-10uM | 62 | 1-100uM | 113 | 1-100uM |
| 12 | 1-10uM | 63 | 1-100uM | 114 | 1-100uM |
| 13 | 1-1000nM | 64 | 1-100uM | 115 | 1-100uM |
| 14 | 1-10uM | 65 | 1-1000nM | 116 | 1-100uM |
| 15 | 1-100uM | 66 | 1-100uM | 117 | 1-10uM |
| 16 | 1-1000nM | 67 | 1-10uM | 118 | 1-100uM |
| 17 | 1-1000nM | 68 | 1-100uM | 119 | 1-100uM |
| 18 | 1-1000nM | 69 | 1-10uM | 120 | 1-10uM |
| 19 | 1-10uM | 70 | 1-1000nM | 121 | 1-1000nM |
| 20 | 1-100uM | 71 | 1-100uM | 122 | 1-100uM |
| 21 | 1-100uM | 72 | 1-10uM | 123 | 1-100uM |
| 22 | 1-100uM | 73 | 1-10uM | 124 | 1-100uM |
| 23 | 1-100uM | 74 | 1-100uM | 125 | 1-100uM |
| 24 | 1-1000nM | 75 | 1-1000nM | 126 | 1-100uM |
| 25 | 1-1000nM | 76 | 1-100uM | 127 | 1-100uM |
| 26 | 1-1000nM | 77 | 1-1000nM | 128 | 1-1000nM |
| 27 | 1-1000nM | 78 | 1-100uM | 129 | 1-100uM |
| 28 | 1-1000nM | 79 | 1-100uM | 130 | 1-100uM |
| 29 | 1-10uM | 80 | 1-100uM | 131 | 1-100uM |
| 30 | 1-100uM | 81 | 1-100uM | 132 | 1-100uM |
| 31 | 1-100uM | 82 | 1-100uM | 133 | 1-100uM |
| 32 | 1-100uM | 83 | 1-10uM | 134 | 1-100uM |
| 33 | 1-100uM | 84 | 1-1000nM | 135 | 1-100uM |
| 34 | 1-100uM | 85 | 1-100uM | 136 | 1-100uM |
| 35 | 1-10uM | 86 | 1-100uM | 137 | 1-100uM |
| 36 | 1-100uM | 87 | 1-100uM | 138 | 1-100uM |
| 37 | 1-100uM | 88 | 1-100uM | 139 | 1-100uM |
| 38 | 1-100uM | 89 | 1-10uM | 140 | 1-100uM |
| 39 | 1-1000nM | 90 | 1-1000nM | 141 | 1-100uM |
| 40 | 1-1000nM | 91 | 1-1000nM | 142 | 1-100uM |
| 41 | 1-10uM | 92 | 1-1000nM | 143 | 1-100uM |
| 42 | 1-1000nM | 93 | 1-1000nM | 144 | 1-100uM |
| 43 | 1-1000nM | 94 | 1-1000nM | 145 | 1-100uM |
| 44 | 1-10uM | 95 | 1-1000nM | 146 | 1-100uM |
| 45 | 1-100uM | 96 | 1-1000nM | 147 | 1-100uM |
| 46 | 1-100uM | 97 | 1-1000nM | 148 | 1-100uM |
| 47 | 1-100uM | 98 | 1-1000nM | 149 | 1-100uM |
| 48 | 1-10uM | 99 | 1-1000nM | 150 | 1-100uM |
| 49 | 1-1000nM | 100 | 1-100uM | 151 | 1-100uM |
| 50 | 1-10uM | 101 | 1-100uM | S1 | 1-10uM |
| 51 | 1-10uM | 102 | 1-100uM | S2 | 1-1000nM |
| S3 | 1-1000nM | S4 | 1-1000nM | S5 | 1-1000nM |
| S6 | 1-1000nM | S7 | 1-10uM | S8 | 1-1000nM |
| S9 | 1-100uM | S10 | 1-100uM | S11 | 1-100uM |
| S12 | 1-100uM | S13 | 1-100uM | S14 | 1-100uM |
| S15 | 1-100uM | S16 | 1-100uM | S17 | 1-100uM |
| S18 | 1-100uM | S19 | 1-100uM | S20 | 1-100uM |

## Claims

1. An amide compound that is an inhibitor of human receptor interacting protein 1 kinase (RIP1), of formula: 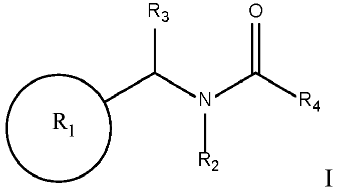 wherein:
R₁ is substituted or unsubstituted phenyl;
R₂ is hydroxyl;
R₃ is H; and
R₄ is 1,1-dimethylpropyl; or
a pharmaceutically acceptable salt, hydrate or stereoisomer of the compound for use in the treatment of a necrosis-associated disease or condition selected from the group consisting of: neurodegenerative disease of the central or peripheral nervous system, endotoxic/septic shock, terminal ileitis, myocarditis, arthritis, atherosclerosis, acute enteritis, ischemic necrosis, pathology resulting from renal failure or cell death, including retinal neuronal, cardiac muscle or immune cell death, such as chemo-or radiation-induced necrosis; liver disease, including drug-induced liver damage or toxicity, acute hepatitis, pancreatic disease, including necrotizing pancreatitis, heart, mesenteric, retinal, hepatic or brain/cerebral ischemic injury, nephritis, ischemic injury during reperfusion or organ storage, head trauma, including traumatic brain injury, stroke, septic shock, coronary heart disease, cardiomyopathy, myocardial infarction, bone avascular necrosis, sickle cell disease, muscle wasting, gastrointestinal disease, tuberculosis, diabetes, pathogenic alteration of blood vessels, muscular dystrophy, graft-versus-host disease, viral, bacterial and fungal infection, Crohn's disease, ulcerative colitis or asthma, comprising administering an effective amount of a compound or a pharmaceutical composition to a patient in need thereof.

2. The compound for use of claim 1 having a formula selected from Table 1: 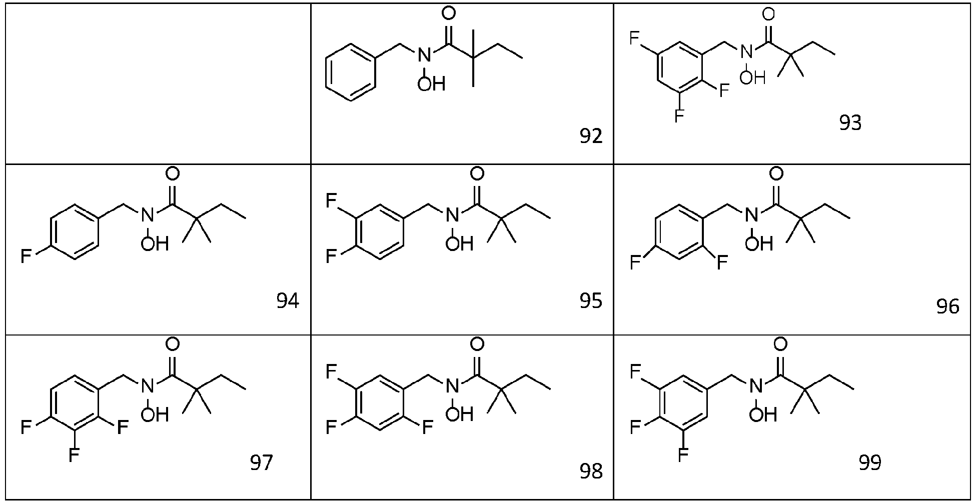 , or a pharmaceutically acceptable salt, hydrate or stereoisomer of the compound.

3. A pharmaceutical composition comprising a compound of claim 1 and a pharmaceutically-acceptable excipient, in unit dosage.

## Patentansprüche

1. Amidverbindung, die ein Inhibitor der humanen Rezeptor-interagierenden Protein-1-Kinase (RIP1) ist, mit der Formel: 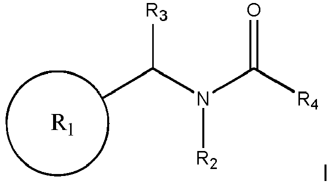 wobei:
R₁ substituiertes oder unsubstituiertes Phenyl ist;
R₂ Hydroxyl ist;
R₃ H ist; und
R₄ 1,1-Dimethylpropyl ist; oder
ein pharmazeutisch annehmbares Salz, Hydrat oder Stereoisomer der Verbindung zur Verwendung bei der Behandlung einer Nekrose-assoziierten Krankheit oder eines Nekrose-assoziierten Zustandes, ausgewählt aus der Gruppe bestehend aus:
neurodegenerative Erkrankung des zentralen oder peripheren Nervensystems, endotoxischer/septischer Schock, terminale Ileitis, Myokarditis, Arthritis, Atherosklerose, akute Enteritis, ischämische Nekrose, Pathologie infolge von Nierenversagen oder Zelltod, einschließlich retinalem Neuronen-, Herzmuskel- oder Immunzelltod, wie chemo- oder strahleninduzierte Nekrose; Lebererkrankungen, einschließlich arzneimittelinduzierter Leberschäden oder -toxizität, akute Hepatitis, Bauchspeicheldrüsenerkrankungen, einschließlich nekrotisierender Pankreatitis, ischämische Schädigungen des Herzens, des Mesenteriums, der Netzhaut, der Leber oder des Gehirns/Zerebrums, Nephritis, ischämische Schädigungen während der Reperfusion oder der Organlagerung, Schädeltrauma, einschließlich traumatischer Hirnverletzungen, Schlaganfall, septischer Schock, koronare Herzkrankheit, Kardiomyopathie, Myokardinfarkt, Knochengefäßnekrose, Sichelzellenkrankheit, Muskelschwund, Magen-Darm-Krankheit, Tuberkulose, Diabetes, pathogene Veränderung von Blutgefäßen, Muskeldystrophie, Transplantat-gegen-Wirt-Krankheit, virale, bakterielle und Pilzinfektion, Morbus Crohn, Colitis ulcerosa oder Asthma, umfassend die Verabreichung einer wirksamen Menge einer Verbindung oder einer pharmazeutischen Zusammensetzung an einen Patienten, der diese benötigt.

2. Die Verbindung zur Verwendung gemäß Anspruch 1 mit einer Formel ausgewählt aus Tabelle 1: 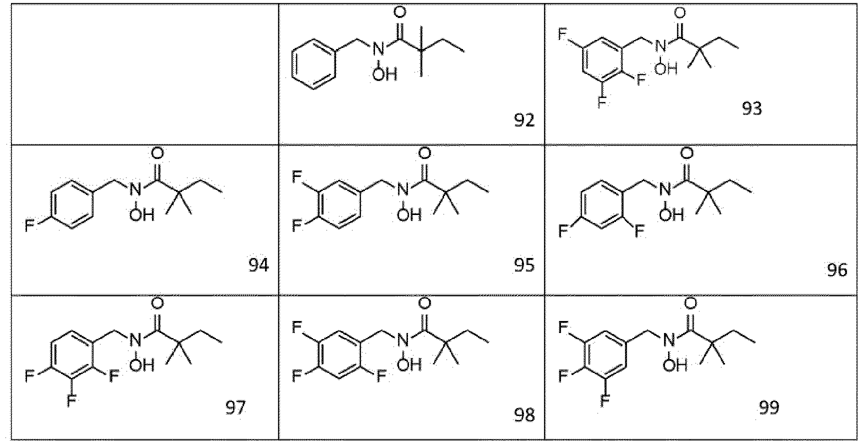 , oder ein pharmazeutisch annehmbares Salz, Hydrat oder Stereoisomer der Verbindung.

3. Eine pharmazeutische Zusammensetzung umfassend eine Verbindung gemäß Anspruch 1 und einen pharmazeutisch annehmbaren Hilfsstoff, in Einheitsdosis.

## Revendications

1. Composé d'amide qui est un inhibiteur de protéine 1 kinase interagissant avec des récepteurs humains (RIP1), de formule : 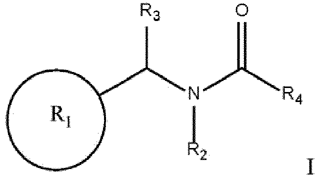 sachant que :
R₁ est du phényle substitué ou non substitué ;
R₂ est de l'hydroxyle ;
R₃ est H ; et
R₄ est du 1,1-diméthylpropyle ; ou
sel, hydrate ou stéréoisomère pharmaceutiquement acceptable du composé pour utilisation dans le traitement d'une maladie ou d'un état associé à une nécrose, sélectionné dans le groupe constitué par : la maladie neurodégénérative du système nerveux central ou périphérique, le choc endotoxique/septique, l'iléite terminale, la myocardite, l'arthrite, l'athérosclérose, l'entérite aiguë, la nécrose ischémique, la pathologie résultant d'une défaillance rénale ou d'une mort cellulaire, incluant la mort de cellules neuronales rétiniennes, de cellules du muscle cardiaque ou de cellules immunitaires, comme la nécrose induite par chimiothérapie ou rayonnement ; la maladie du foie, incluant la lésion ou la toxicité du foie induite par des médicaments, l'hépatite aiguë, la maladie du pancréas, incluant la pancréatite nécrosante, la lésion ischémique du cœur, mésentérique, rétinienne, hépatique ou du cerveau/cérébrale, la néphrite, la lésion ischémique pendant la reperfusion ou le stockage d'organe, le traumatisme crânien, incluant la lésion traumatique du cerveau, l'AVC, le choc septique, la maladie cardiaque coronarienne, la cardiomyopathie, l'infarctus du myocarde, la nécrose avasculaire des os, la drépanocytose, la fonte musculaire, la maladie gastrointestinale, la tuberculose, le diabète, l'altération pathogène des vaisseaux sanguins, la dystrophie musculaire, la maladie du greffon contre l'hôte, l'infection virale, bactérienne et fongique, la maladie de Crohn, la colite ulcéreuse ou l'asthme, comprenant l'administration d'une quantité efficace d'un composé ou d'une composition pharmaceutique à un patient qui en a besoin.

2. Le composé pour utilisation selon la revendication 1 ayant une formule sélectionnée dans le Tableau 1 : 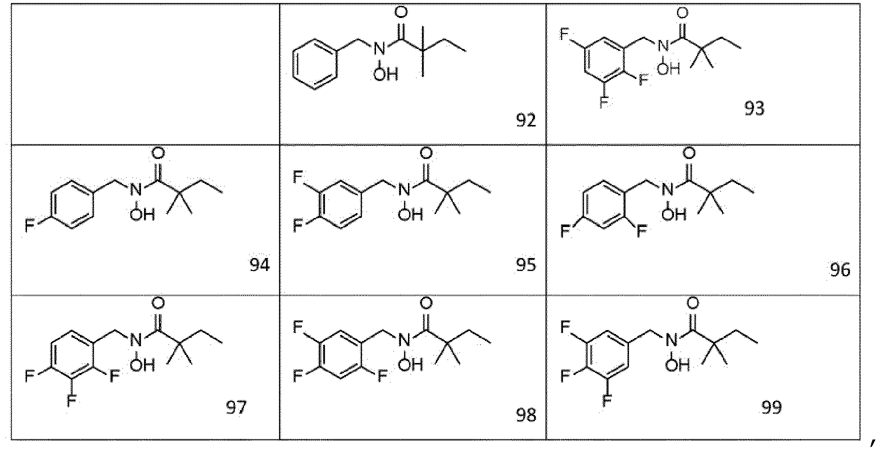 ou sel, hydrate ou stéréoisomère pharmaceutiquement acceptable du composé.

3. Composition pharmaceutique comprenant un composé de la revendication 1 et un excipient pharmaceutiquement acceptable, en dosage unitaire.
